# EUROPEAN PATENT APPLICATION

(11) **EP 4 036 086 A1**
(43) Date of publication of application: **03.08.2022**
(21) Application number: 20868130.4
(22) Date of filing: 23.09.2020
(51) Int. Cl.: C07D 401/14, C07D 405/14, A61K 31/416, A61K 31/4412, A61K 31/501, A61K 31/506, A61K 31/444, A61P 35/00, A61P 17/06, A61P 13/12, A61P 19/02, A61P 19/10, A61P 29/00

(54) **HETEROCYCLIC DERIVATIVE AND USE THEREOF**

(30) Priority: 24.09.2019 CN 201910909530; 17.01.2020 CN 202010052064; 21.02.2020 CN 202010106648
(71) Applicant: Transthera Sciences (Nanjing), Inc., Jiangbei New Area Nanjing Jiangsu 210032 (CN)
(72) Inventor: WU, Frank, Nanjing, Jiangsu 210032 (CN); WAN, Zhonghui, Nanjing, Jiangsu 210032 (CN)
(74) Representative: Grünecker Patent- und Rechtsanwälte PartG mbB
(86) International application number: PCT/CN2020/117074
(87) International publication number: WO 2021/057782

(57) **Abstract**

Disclosed are a TAM family kinase /and Ron kinase inhibitor compound as shown in general formula (I), a pharmaceutically acceptable salt, an ester, a stereoisomer and a tautomer thereof, a pharmaceutical composition and a pharmaceutical preparation containing same, and the use thereof. The compound can selectively inhibit TAM family tyrosine kinase /and Ron kinase, and can be used for the treatment and/or prevention of diseases mediated by the abnormal expression of a receptor of TAM family kinase /and Ron kinase and/or a ligand thereof. By means of targeted inhibition of the TAM family kinase /and Ron kinase, the compound can reverse the immunosuppressive environment in a tumor microenvironment, inhibit the growth, migration and/or drug resistance performance of tumors, and exert anti-tumor immunological effects and anti-tumor efficacy.

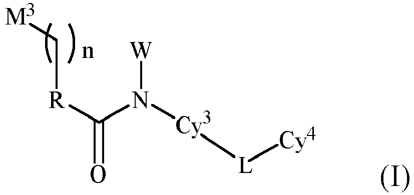

The definition of each group in the formula is described in the specification.

## Description

### TECHNICAL FIELD

The present invention belongs to the field of medicines, and particularly relates to a TAM family kinase and/or Ron kinase inhibitor compound of general formula (I), and a pharmaceutically acceptable salt, an ester, a stereoisomer and a tautomer thereof, a pharmaceutical composition and a pharmaceutical formulation comprising the same and use thereof. The compound of the present invention can selectively inhibit TAM family tyrosine kinase and/or Ron kinase, and can be used for the treatment and/or prevention of diseases mediated by abnormal expression of TAM family kinase and/or Ron kinase receptors and/or ligands thereof.

### BACKGROUND

The TAM family includes three members of Axl, Mer and Tyro-3, and contains an extracellular domain, a transmembrane domain, and a conserved intracellular kinase domain. The extracellular domain consists of two immunoglobulin-like domains linked to two type III fibronectin repeating units. The conserved amino acid sequence KW(I/L)A(I/L)ES of the intracellular kinase domain is a unique structural feature of the TAM family. The family has a common ligand, i.e., growth arrest-specific 6 protein (Gas6), which can bind to all TAM receptors, but with different binding strengths. In addition, the TAM family also includes related receptors, such as vitamin K dependent protein S (ProS), Tubby-like protein, recombinant human Tubby-like protein 1 (Tulp1) and galectin-3 (Wu Yanjun, Chinese Journal of New Drugs, 2016; Lu Ping, Chinese Journal of Practical Diagnosis and Therapy, 2016).

Axl (also referred to as UFO, Ark, Tyro-7 or JTK1), Mer (also referred to as c-Mer, Mertk, Eyk, Nyk or Tyro-12), Tyro-3 (also referred to as Sky, Byk, Rse or Dtk), galectin-3, Gas6 and ProS are abnormally expressed in various solid tumors, such as lung cancer, gastric cancer and liver cancer, and in various hematological tumors, such as AML, ALL and CML, and are closely associated with poor prognosis of a disease, disease progression, tumor metastasis, tumor drug resistance, etc. (Douglas K, Nature Reviews, 2014). In particular, Axl, a tyrosine kinase, has been proved to be one of the causes of resistance to EGFR inhibitors in NSCLC, and is closely related to the metastasis of various solid tumors. The medicaments developed by targeting Axl also confirmed that inhibiting Axl could delay the resistance to EGFR inhibitors and the metastasis of tumors (T. Jimbo, Annals of Oncology, 2017; Sacha J. Holland, American Association for Cancer Research, 2010). Moreover, Axl, Mer, Tryo-3 and TAM ligands also play a major role in tumor immunology. Inhibiting the TAM family and ligands thereof can reverse the immunosuppression in the tumor microenvironment and enhance the tumor cell killing effect of the immune system by promoting polarization of macrophages to M1-type macrophages, increasing the activation and function of effector T cells, enhancing the anti-tumor activity of NK cells, etc. (Yemsratch T. Akalu, Immunological Reviews, 2017; Greg Lemke, Nature Reviews Immunology, 2008). Therefore, such inhibitors can be developed to strongly inhibit and treat various solid and hematological tumors induced by the family, such as lung cancer, liver cancer, breast cancer, glioma, melanoma, AML, ALL and CML.

In addition to use in the field of tumor diseases, TAM family receptors and ligands thereof can regulate vascular smooth muscle homeostasis, platelet aggregation, thrombus stabilization, erythropoiesis, oligodendrocyte survival, osteoclast function, phagocytosis of apoptotic cells, inflammation, innate immunity and many other physiological functions. Therefore, TAM family inhibitors can also be used for the treatment of endometriosis, vascular disease/trauma, psoriasis, visual defect/lesion (caused by macular degeneration, diabetes, premature delivery, etc.), kidney disease, rheumatoid arthritis, osteoporosis and other related diseases caused by the disturbance of signaling pathways of the TAM family.

Human cell transmembrane receptor protein Ron (macrophage stimulating 1 receptor, MST1R) is also known as macrophage stimulating protein receptor (MSP-receptor) or hepatocyte growth factor like (HGFL) receptor. The Ron gene is expressed in human epithelial tissues, granulocytes, monocytes, megakaryocytes, tonsil germinal layer, small intestine, colon, kidney, lung, bone marrow, and osteoclasts.

The role of Ron in the development of many common epithelial tumors in humans has been widely documented. Ron is detectable in 33%-96% of clinical specimens of pancreatic cancer, and expression of Ron is positively correlated with the pathological progress of the tumor (Thomas RM, et al. CancerRes, 2007, 67(13): 6075-6082). About 50% of breast cancer patients overexpress Ron, and the expression level of Ron in postmenopausal breast cancer significantly increases compared to normal breast tissue and premenopausal breast cancer. Studies in transgenic mice have shown that overexpression of Ron is sufficient to induce mammary gland transformation and has high metastatic potential, with greater than 86% of transgenic mice having metastatic foci in the liver and lungs (Glendon M. Zinser, American Association for Cancer Research, 2006). In addition, Ron is also abnormally expressed in various malignant tumors such as non-small cell lung cancer and head and neck squamous cell carcinoma and is associated with tumor infiltration progress and prognosis.

### SUMMARY

The present invention provides a novel heterocyclic derivative inhibitor compound, and a pharmaceutically acceptable salt, an ester, a stereoisomer and a tautomer thereof (hereinafter, also referred to as the compound of the present invention). The compound of the present invention has an inhibitory effect on the TAM family kinase and can be used for the treatment and/or prevention of diseases mediated by abnormal expression of TAM family kinase receptors and/or ligands thereof. In addition, the compound of the present invention can also target Ron kinase and exhibits an inhibitory effect on Ron kinase. In addition, the compound of the present invention can be used for the treatment and/or prevention of diseases mediated by abnormal expression of TAM family kinase and/or Ron kinase receptors and/or ligands thereof. By means of inhibition of the TAM family kinase and/or Ron kinase, the compound of the present invention can reverse the immunosuppressive environment in a tumor microenvironment, inhibit the growth, migration and/or drug resistance performance of tumors, and exert anti-tumor immunological effects and anti-tumor efficacy.

Specifically, the technical solution adopted in the present invention is as follows:
A compound of general formula (I), or a pharmaceutically acceptable salt, an ester, a stereoisomer or a tautomer thereof: 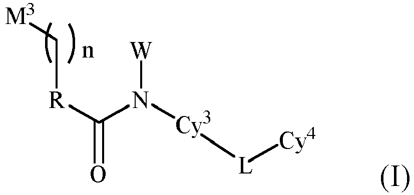 wherein W is selected from hydrogen and optionally substituted C₁₋₆ alkyl;
R represents a group shown as general formula (b): 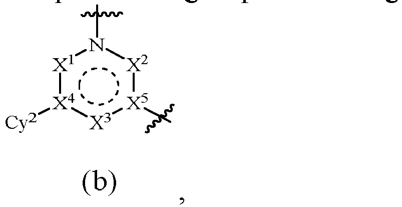 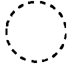 represents an optional double bond moiety in the ring structure, and the 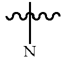 moiety is linked to the M³ group via a linking group;
X¹, X² and X³ are each independently selected from CR^{a}, C=O, NR^{b} and O, and at least one of the above is C=O;
X⁴ and X⁵ are each independently selected from C;
M³ is selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl;
Cy² is selected from 3-12 membered cycloalkyl, 3-12 membered cycloalkenyl, 3-14 membered heterocyclyl, 6-14 membered aryl and 5-10 membered heteroaryl optionally substituted with one or more R², and R² is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl;
Cy³ is selected from 5-10 membered heteroaryl optionally substituted with one or more R³, and R³ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl;
Cy⁴ is selected from 5-9 membered heterocyclyl and 5-9 membered heteroaryl optionally substituted with one or more R⁴, and R⁴ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl, or, two R⁴, together with the atoms attached thereto, can form a 5-6 membered cyclic group;
L is selected from -NR^{b}-, -O-, -S-, and -(CR^{a}R^{a})ₘ-, and m is selected from integers between 1 and 3;
R^{a} is present or absent, and when present, is each independently selected from hydrogen, cyano, hydroxyl, halogen, carboxyl, nitro, -NR^{e}R^{f}, -C(O)R^{g}, -C(O)NR^{e}R^{f}, -OC(O)NR^{e}R^{f}, -NR^{e}C(O)OR^{g}, -NR^{e}C(O)R^{g}, -SO₂-NR^{e}R^{f}, -SO₂R^{g}, and -NR^{e}SO₂R^{g}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{e}C(O)-R', 3-12 membered cycloalkyl, 3-12 membered cycloalkenyl, 3-14 membered heterocyclyl, 6-14 membered aryl and 5-10 membered heteroaryl;
R^{b} and R^{c} are present or absent, and when present, are each independently selected from hydrogen, hydroxyl, -C(O)R^{g}, -C(O)NR^{e}R^{f}, -SO₂-NR^{e}R^{f}, and -SO₂R^{g}; and optionally substituted C₁₋₆ alkyl, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R^{d} is present or absent, and when present, is each independently selected from hydrogen, -NR^{e}R^{f}, -NR^{e}C(O)OR^{g}, -NR^{e}C(O)R^{g}, and -NR^{e}SO₂R^{g}; and optionally substituted C₁₋₆ alkyl, C₁₋₆ alkoxy, -C₁₋₆ alkyl-R', -C₁₋₆ alkoxy-R', -O-R', -NR^{e}C(O)-R', 3-12 membered cycloalkyl, 3-12 membered cycloalkenyl, 3-14 membered heterocyclyl, 6-14 membered aryl and 5-10 membered heteroaryl;
R^{e} and R^{f} are present or absent, and when present, are each independently selected from hydrogen, hydroxyl, carboxyl, cyano, nitro and halogen; and optionally substituted C₁₋₆ alkyl, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R^{g} is present or absent, and when present, is each independently selected from hydrogen; and optionally substituted C₁₋₆ alkyl, C₁₋₆ alkoxy, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R' is 3-12 membered cycloalkyl, 3-12 membered cycloalkenyl, 3-14 membered heterocyclyl, 6-14 membered aryl, or 5-10 membered heteroaryl;
n is an integer between 0 and 4;
the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo.

In one embodiment of the present invention, provided is a compound of general formula (I), or a pharmaceutically acceptable salt, an ester, a stereoisomer or a tautomer thereof:
wherein W is selected from hydrogen and optionally substituted C₁₋₆ alkyl;
R represents a group shown as general formula (b): 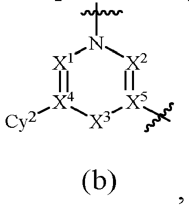
the 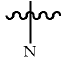 moiety is linked to the M³ group through a linking group;
X¹, X² and X³ are each independently selected from CR^{a}, C=O and NR^{b}, and at least one of the above is C=O;
X⁴ and X⁵ are each selected from C;
M³ is selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, 3-8 membered cycloalkyl and 3-14 membered heterocyclyl;
Cy² is selected from 6-14 membered aryl and 5-10 membered heteroaryl optionally substituted with one or more R², and R² is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-8 membered cycloalkyl, 3-8 membered cycloalkenyl, 3-14 membered heterocyclyl, 6-14 membered aryl and 5-10 membered heteroaryl;
Cy³ is selected from 5-10 membered heteroaryl optionally substituted with one or more R³, and R³ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-8 membered cycloalkyl, 3-8 membered cycloalkenyl, 3-14 membered heterocyclyl, 6-14 membered aryl and 5-10 membered heteroaryl;
Cy⁴ is selected from 5-9 membered heteroaryl optionally substituted with one or more R⁴, and R⁴ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-8 membered cycloalkyl, 3-8 membered cycloalkenyl, 3-14 membered heterocyclyl, 6-14 membered aryl and 5-10 membered heteroaryl, or, two R⁴, together with the atoms attached thereto, can form a 5-6 membered cyclic group;
L is selected from -NR^{b}-, -O- and -S-;
R^{a} is present or absent, and when present, is each independently selected from hydrogen, cyano, hydroxyl, halogen, carboxyl, nitro, -NR^{e}R^{f}, -C(O)R^{g}, -C(O)NR^{e}R^{f}, -OC(O)NR^{e}R^{f}, -NR^{e}C(O)OR^{g}, -NR^{e}C(O)R^{g}, -SO₂-NR^{e}R^{f}, -SO₂R^{g}, and -NR^{e}SO₂R^{g}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{e}C(O)-R', 3-8 membered cycloalkyl, 3-8 membered cycloalkenyl, 3-14 membered heterocyclyl, 6-14 membered aryl and 5-10 membered heteroaryl;
R^{b} and R^{c} are present or absent, and when present, are each independently selected from hydrogen, hydroxyl, -C(O)R^{g}, -C(O)NR^{e}R^{f}, -SO₂-NR^{e}R^{f}, and -SO₂R^{g}; and optionally substituted C₁₋₆ alkyl, -C₁₋₆ alkyl-R', -C(O)-R', -SO₂-R', 3-8 membered cycloalkyl, 3-8 membered cycloalkenyl, 3-14 membered heterocyclyl, 6-14 membered aryl and 5-10 membered heteroaryl;
R^{d} is present or absent, and when present, is each independently selected from hydrogen, -NR^{e}R^{f}, -NR^{e}C(O)OR^{g}, -NR^{e}C(O)R^{g}, and -NR^{e}SO₂R^{g}; and optionally substituted C₁₋₆ alkyl, C₁₋₆ alkoxy, -C₁₋₆ alkyl-R', -C₁₋₆ alkoxy-R', -O-R', -NR^{e}C(O)-R', 3-8 membered cycloalkyl, 3-8 membered cycloalkenyl, 3-14 membered heterocyclyl, 6-14 membered aryl and 5-10 membered heteroaryl;
R^{e} and R^{f} are present or absent, and when present, are independently selected from hydrogen, hydroxyl, carboxyl, cyano, nitro and halogen; and optionally substituted C₁₋₆ alkyl, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R^{g} is present or absent, and when present, is independently selected from hydrogen; and optionally substituted C₁₋₆ alkyl, C₁₋₆ alkoxy, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R' is 3-8 membered cycloalkyl, 3-8 membered cycloalkenyl, 3-14 membered heterocyclyl, 6-14 membered aryl, or 5-10 membered heteroaryl;
n is an integer between 0 and 3;
the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-8 membered cycloalkyl, 6-14 membered aryl, 3-8 membered heterocyclyl, 5-10 membered heteroaryl and oxo;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O.

In one embodiment of the present invention, provided is a compound of general formula (I), or a pharmaceutically acceptable salt, an ester, a stereoisomer or a tautomer thereof:
wherein, X¹, X² and X³ are each independently selected from CR^{a}, C=O and NR^{b}, and at least one of the above is C=O;
X⁴ is selected from C, and X⁵ is selected from C;
M³ is selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl, C₂₋₈ alkenyl, C₂₋₈ alkynyl, 3-8 membered cycloalkyl and 3-8 membered heterocyclyl;
Cy² is selected from phenyl and 5-6 membered heteroaryl optionally substituted with one or more R², and R² is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
Cy³ is selected from 5-6 membered heteroaryl optionally substituted with one or more R³, and R³ is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
Cy⁴ is selected from 5-6 membered heteroaryl and 9-membered ortho-fused heteroaryl optionally substituted with one or more R⁴, and R⁴ is each independently selected from hydrogen, hydroxyl, halogen, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy, or, two R⁴, together with the atoms attached thereto, can form a 5-6 membered oxygen-containing cyclic group;
L is selected from -O-;
R^{a} is present or absent, and when present, is each independently selected from hydrogen and optionally substituted C₁₋₆ alkyl;
R^{b} and R^{c} are present or absent, and when present, are each independently selected from hydrogen and optionally substituted C₁₋₆ alkyl;
R^{d} is present or absent, and when present, is each independently selected from hydrogen and optionally substituted C₁₋₆ alkyl;
n is an integer between 0 and 2;
the substituents involved in the phrase "optionally substituted" are each independently selected from hydroxyl, amino, carboxyl, cyano, nitro, halogen, C₁₋₆ alkyl and C₁₋₆ alkoxy;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O;
preferably, Cy³ is 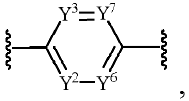 Y², Y³, Y⁶ and Y⁷ are each independently selected from CH and N, and at least one of the above is N.

In one embodiment of the present invention, provided is a compound of general formula (I), or a pharmaceutically acceptable salt, an ester, a stereoisomer or a tautomer thereof:
wherein, X¹, X² and X³ are each independently selected from CR^{a}, C=O and NR^{b}, and at least one of the above is C=O;
X⁴ is selected from C, and X⁵ is selected from C;
M³ is selected from hydrogen; and optionally substituted C₁₋₆ alkyl, C₂₋₈ alkenyl, C₂₋₈ alkynyl, 3-8 membered cycloalkyl and 5-6 membered heterocyclyl;
Cy² is selected from phenyl and 5-6 membered heteroaryl optionally substituted with one or more R²;
Cy³ represents 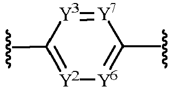 optionally substituted with one or more R³;
Y², Y³, Y⁶ and Y⁷ are each independently selected from CH and N, and at least one of the above is N;
Cy⁴ is selected from 6-membered heteroaryl and 9-membered ortho-fused heteroaryl optionally substituted with one or more R⁴;
R² is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R³ is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R⁴ is each independently selected from hydrogen, hydroxyl, halogen, nitro, -NR^{b}R^{c}, -C(O)R^{d}, -C(O)NR^{b}R^{c}, -NR^{b}C(O)R^{d} and 5-10 membered heteroaryl; and optionally substituted C₁₋₆ alkyl and C₁₋₆ alkoxy;
L is selected from -O-;
R^{a} is present or absent, and when present, is each independently selected from hydrogen, C₁₋₆ alkyl and halogenated C₁₋₆ alkyl;
R^{b} and R^{c} are present or absent, and when present, are each independently selected from hydrogen and C₁₋₆ alkyl;
R^{d} is present or absent, and when present, is each independently selected from hydrogen and C₁₋₆ alkyl;
n is an integer between 0 and 2;
the substituents involved in the phrase "optionally substituted" are each independently selected from hydroxyl, sulfydryl, amino, carboxyl, cyano, nitro, halogen, C₁₋₆ alkyl and C₁₋₆ alkoxy;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O;
preferably, Cy⁴ is selected from 6-membered N-containing heteroaryl and 9-membered N-containing ortho-fused heteroaryl optionally substituted with one or more R⁴.

In one embodiment of the present invention, provided is a compound of general formula (I), or a pharmaceutically acceptable salt, an ester, a stereoisomer or a tautomer thereof:
wherein X¹ and X² are each independently selected from CR^{a} and NR^{b}, and X³ is C=O;
M³ is selected from hydrogen, C₁₋₆ alkyl, C₂₋₈ alkenyl, C₂₋₈ alkynyl, 3-6 membered cycloalkyl and 5-6 membered heterocyclyl;
Cy² is selected from phenyl optionally substituted with one or more R²;
Cy³ represents 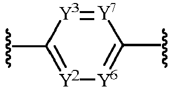 optionally substituted with one or more R³;
Y², Y³, Y⁶ and Y⁷ are independently selected from CH and N, and at least one of the above is N;
Cy⁴ is selected from 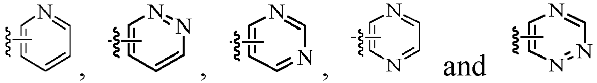 optionally substituted with one or more R⁴;
R² is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R³ is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R⁴ is each independently selected from hydrogen, hydroxyl, halogen, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
L is selected from -O-;
R^{a} is present or absent, and when present, is each independently selected from hydrogen, C₁₋₆ alkyl and halogenated C₁₋₆ alkyl;
R^{b} and R^{c} are present or absent, and when present, are each independently selected from hydrogen and C₁₋₆ alkyl;
R^{d} is present or absent, and when present, is each independently selected from hydrogen and C₁₋₆ alkyl;
n is an integer between 0 and 1;
the substituents involved in the phrase "optionally substituted" are each independently selected from hydroxyl, sulfydryl, amino, carboxyl, cyano, nitro and halogen.

In one embodiment of the present invention, provided is a compound of general formula (I), or a pharmaceutically acceptable salt, an ester, a stereoisomer or a tautomer thereof:
wherein W is selected from hydrogen;
R represents a group shown as general formula (b): 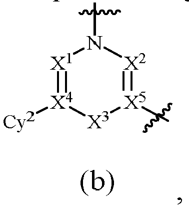
the 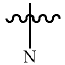 moiety is linked to the M³ group through a linking group;
X¹ and X² are each independently selected from CR^{a} and NR^{b}, and X³ is C=O;
X⁴ is selected from C, and X⁵ is selected from C;
M³ is selected from hydrogen, hydroxyl, halogen, nitro, C₁₋₄ alkyl, C₂₋₆ alkenyl, C₂₋₆ alkynyl, 3-6 membered cycloalkyl and 5-6 membered heterocyclyl;
Cy² is selected from 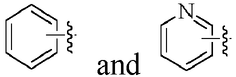 optionally substituted with one or more R², and R² is each independently selected from hydrogen, hydroxyl, fluoro, chloro, bromo and C₁₋₄ alkyl;
Cy³ is selected from 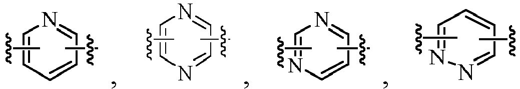 and 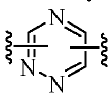 optionally substituted with one or more R³, and R³ is each independently selected from hydrogen, hydroxyl, fluoro, chloro, bromo and C₁₋₄ alkyl;
Cy⁴ is selected from 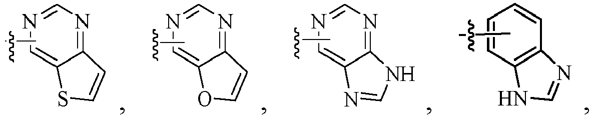 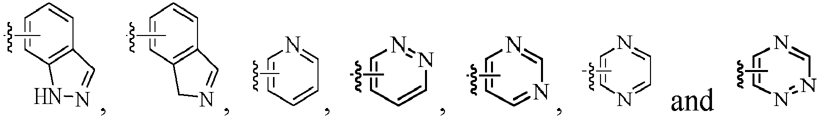 optionally substituted with one or more R⁴, and
R⁴ is each independently selected from hydrogen, hydroxyl, halogen, amino, (C₁₋₄ alkyl)₁₋₂ amino-, C₁₋₄ alkyl, halogenated C₁₋₄ alkyl, C₁₋₄ alkoxy, halogenated C₁₋₄ alkoxy, and 3-14 membered heterocyclyl;
L is -O-;
R^{a} is present or absent, and when present, is each independently selected from hydrogen, C₁₋₆ alkyl and halogenated C₁₋₆ alkyl;
R^{b} is present or absent, and when present, is each independently selected from hydrogen and C₁₋₆ alkyl;
n is an integer between 0 and 1;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O.

More specifically, the present invention provides the following technical solutions.

Solution 1: Provided is a compound of general formula (I), or a pharmaceutically acceptable salt, an ester, a stereoisomer or a tautomer thereof: 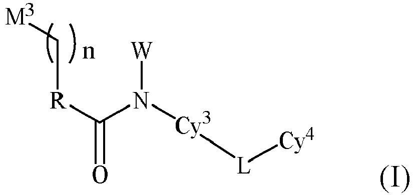 wherein W is selected from hydrogen and optionally substituted C₁₋₆ alkyl;
R represents a group shown as general formula (b'): 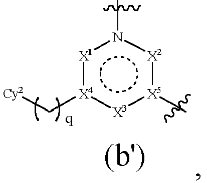
the 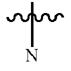 moiety is linked to the M³ group through a linking group;
X¹, X² and X³ are each independently selected from CR^{a}R^{a}, C=O and NR^{b}, and at least one of the above is C=O;
X⁴ and X⁵ are each independently selected from CR^{a} and N;
M³ is selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl; when N of the 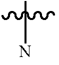 moiety is linked to a double bond in formula (b'), M³ is absent;
Cy² is selected from 3-12 membered cycloalkyl optionally substituted with one or more R², 3-12 membered cycloalkenyl optionally substituted with one or more R², 3-14 membered heterocyclyl optionally substituted with one or more R², 6-14 membered aryl optionally substituted with one or more R², and 5-10 membered heteroaryl optionally substituted with one or more R², and R² is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{c}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
Cy³ is selected from 5-10 membered heteroaryl optionally substituted with one or more R³, and R³ is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{c}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
Cy⁴ is selected from 3-12 membered cycloalkyl optionally substituted with one or more R⁴, 3-14 membered heterocyclyl optionally substituted with one or more R⁴, 5-14 membered heteroaryl optionally substituted with one or more R⁴ and 6-14 membered aryl optionally substituted with one or more R⁴, and R⁴ is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{c}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl and optionally substituted 5-10 membered heteroaryl, or, two R⁴, together with the atoms attached thereto, can form a 5-14 membered cyclic group;
L is selected from -NR^{b}-, -O-, -S-, and -(CR^{a}R^{a})ₘ-, and m is selected from integers between 1 and 3;
R^{a} is present or absent, and when present, is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{c}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
R^{b} and R^{c} are present or absent, and when present, are each independently selected from hydrogen, hydroxyl, optionally substituted C₁₋₆ alkyl, optionally substituted -C₁₋₆ alkyl-R', -C(O)-R ', -SO₂-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
R^{d} is present or absent, and when present, is each independently selected from hydroxyl, halogen, amino, -amino (optionally substituted C₁₋₆ alkyl)₁₋₂, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
R' is each independently selected from optionally substituted 3-12 membered cycloalkyl, optionally substituted 3-12 membered cycloalkenyl, optionally substituted 3-14 membered heterocyclyl, optionally substituted 6-14 membered aryl and optionally substituted 5-10 membered heteroaryl;
q is an integer between 0 and 4;
n is an integer between 0 and 4;
the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo.

Solution 2: Provided is the compound, or the pharmaceutically acceptable salt, the ester, the stereoisomer or the tautomer thereof according to Solution 1, wherein,
when M³ represents a group carrying aryl, Cy² does not represent heterocyclyl;
when M³ represents optionally substituted C₁₋₆ alkyl, X⁴ represents CR^{a};
when M³ represents a group carrying aryl, X⁴ represents CR^{a};
when M³ represents a group carrying cycloalkyl, X⁴ represents CR^{a};
when M³ represents optionally substituted C₁₋₆ alkyl and X¹ represents CR^{a}R^{a}, the R^{a} group on X¹ does not have carbonyl;
when M³ represents a group carrying aryl, X² does not represent C=O.

Solution 3: Provided is the compound, or the pharmaceutically acceptable salt, the ester, the stereoisomer or the tautomer thereof according to any one of the preceding solutions, wherein
M³ is selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)R^{d}, -NR^{b}C(O)OR^{d}, -SO₂-NR^{b}R^{c}, -SO₂R^{d}, and -NR^{b}SO₂R^{d}; and optionally substituted C₁₋₆ alkyl, optionally substituted C₁₋₆ alkoxy, optionally substituted C₂₋₈ alkenyl, optionally substituted C₂₋₈ alkynyl, optionally substituted 3-8 membered cycloalkyl, and optionally substituted 3-14 membered heterocyclyl;
Cy² is selected from 6-14 membered aryl optionally substituted with one or more R² and 5-10 membered heteroaryl optionally substituted with one or more R², and R² is each independently selected from hydrogen, hydroxyl, halogen, carboxyl, nitro, -NR^{b}R^{c}, -C(O)R^{d}, -C(O)NR^{b}R^{c}, -NR^{b}C(O)OR^{d}, optionally substituted C₁₋₆ alkyl, and optionally substituted C₁₋₆ alkoxy; preferably, R² is each independently selected from hydrogen, hydroxyl, halogen, C₁₋₆ alkyl, hydroxyl C₁₋₆ alkyl, amino C₁₋₆ alkyl, halogenated C₁₋₆ alkyl, C₁₋₆ alkoxy, and halogenated C₁₋₆ alkoxy; preferably, R² is each independently selected from hydrogen, hydroxyl, fluoro, chloro, bromo and C₁₋₄ alkyl;
Cy³ is selected from 5-10 membered heteroaryl optionally substituted with one or more R³, and R³ is each independently selected from hydrogen, hydroxyl, halogen, carboxyl, nitro, -NR^{b}R^{c}, -C(O)R^{d}, -C(O)NR^{b}R^{c}, -NR^{b}C(O)R^{d}, optionally substituted C₁₋₆ alkyl, and optionally substituted C₁₋₆ alkoxy; preferably, R³ is each independently selected from hydrogen, hydroxyl, halogen, C₁₋₆ alkyl, hydroxyl C₁₋₆ alkyl, amino C₁₋₆ alkyl, halogenated C₁₋₆ alkyl, C₁₋₆ alkoxy, and halogenated C₁₋₆ alkoxy; preferably, R³ is each independently selected from hydrogen, hydroxyl, fluoro, chloro, bromo and C₁₋₄ alkyl;
Cy⁴ is selected from 5-10 membered heterocyclyl optionally substituted with one or more R⁴, 5-10 membered heteroaryl optionally substituted with one or more R⁴, and 6-10 membered aryl optionally substituted with one or more R⁴; R⁴ is each independently selected from hydrogen, hydroxyl, halogen, carboxyl, nitro, -NR^{b}R^{c}, -C(O)R^{d}, -C(O)NR^{b}R^{c}, -NR^{b}C(O)R^{d}, optionally substituted C₁₋₆ alkyl, optionally substituted C₁₋₆ alkoxy, and optionally substituted 5-10 membered heteroaryl; preferably, R⁴ is each independently selected from hydrogen, hydroxyl, halogen, amino, (C₁₋₆ alkyl)₁₋₂ amino-, C₁₋₆ alkyl, hydroxyl C₁₋₆ alkyl, amino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkyl C₁₋₆ alkoxy, C₁₋₆ alkoxy C₁₋₆ alkoxy, and 5-6 membered heteroaryl; preferably, R⁴ is selected from hydrogen, hydroxyl, sulfydryl, halogen, amino, (C₁₋₄ alkyl)₁₋₂ amino-, C₁₋₄ alkyl, halogenated C₁₋₄ alkyl, C₁₋₄ alkoxy, C₁₋₄ alkoxy C₁₋₄ alkyl, C₁₋₄ alkyl C₁₋₄ alkoxy, C₁₋₄ alkoxy C₁₋₄ alkoxy, halogenated C₁₋₄ alkoxy, pyrrolyl, furanyl, thienyl, oxazolyl, isoxazolyl, pyrazolyl, imidazolyl, pyridyl, pyrimidinyl and pyridazinyl;
L is selected from -NR^{b}-, -O- and -S-;
R^{a} is present or absent, and when present, is each independently selected from hydrogen, halogen, hydroxyl, carboxyl, nitro, -NR^{b}R^{c}, -C(O)R^{d}, -C(O)NR^{b}R^{c}, -NR^{b}C(O)R^{d}, optionally substituted C₁₋₆ alkyl, and optionally substituted C₁₋₆ alkoxy; preferably, R^{a} is present or absent, and when present, is each independently selected from hydrogen, hydroxyl, halogen, carboxyl, nitro, C₁₋₆ alkyl, C₁₋₆ alkoxy, and halogenated C₁₋₆ alkyl, and preferably, R^{a} is present or absent, and when present, is each independently selected from hydrogen, C₁₋₆ alkyl and C₁₋₆ alkoxy;
R^{b} and R^{c} are present or absent, and when present, are each independently selected from hydrogen and optionally substituted C₁₋₆ alkyl; preferably, R^{b} and R^{c} are present or absent, and when present, are each independently selected from hydrogen, C₁₋₆ alkyl, and halogenated C₁₋₆ alkyl; preferably, R^{b} and R^{c} are present or absent, and when present, are each independently selected from hydrogen and C₁₋₆ alkyl;
R^{d} is present or absent, and when present, is each independently selected from hydroxyl, halogen, amino, -amino (optionally substituted C₁₋₆ alkyl)₁₋₂, optionally substituted C₁₋₆ alkyl, and optionally substituted C₁₋₆ alkoxy; preferably, R^{d} is present or absent, and when present, is each independently selected from hydroxyl, amino, -amino (C₁₋₆ alkyl)₁₋₂, C₁₋₆ alkyl, halogenated C₁₋₆ alkyl, C₁₋₆ alkoxy, and halogenated C₁₋₆ alkoxy; preferably, R^{d} is present or absent, and when present, is each independently selected from hydroxyl, amino, C₁₋₄ alkyl, halogenated C₁₋₄ alkyl, C₁₋₄ alkoxy, and halogenated C₁₋₄ alkoxy;
R' is selected from optionally substituted 3-8 membered cycloalkyl, optionally substituted 3-8 membered cycloalkenyl, optionally substituted 3-10 membered heterocyclyl, optionally substituted 6-10 membered aryl, and optionally substituted 5-6 membered heteroaryl; preferably, R' is selected from optionally substituted 3-6 membered cycloalkyl, optionally substituted 4-7 membered heterocyclyl, optionally substituted 6-10 membered aryl, and optionally substituted 5-6 membered heteroaryl; preferably, R' is selected from cyclopropyl, cyclobutyl, cyclopentyl, cyclohexyl, phenyl, naphthyl, oxacyclopropyl, oxacyclobutyl, oxacyclopentyl, oxacyclohexyl, oxacycloheptyl, pyrrolyl, furanyl, thienyl, oxazolyl, isoxazolyl, pyrazolyl, thiazolyl, pyridyl, pyrimidinyl and pyridazinyl;
the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-8 membered cycloalkyl, 6-10 membered aryl, 3-8 membered heterocyclyl, 5-6 membered heteroaryl and oxo; preferably, the substituents involved in "optionally substituted" are each independently selected from hydroxyl, sulfydryl, amino, carboxyl, cyano, nitro, halogen, C₁₋₄ alkyl, C₁₋₄ alkoxy C₁₋₄ alkyl, C₁₋₄ alkoxy, C₁₋₄ alkyl C₁₋₄ alkoxy, C₁₋₄ alkoxy C₁₋₄ alkoxy, C₁₋₄ alkylamino, (C₁₋₄ alkyl)₂ amino, C₁₋₄ alkylaminocarbonyl, (C₁₋₄ alkyl)₂ aminocarbonyl, C₁₋₄ alkylcarbonyl, C₁₋₄ alkylcarbonyloxy, C₁₋₄ alkylcarbonylamino, C₁₋₄ alkylsulfonylamino, halogenated C₁₋₄ alkyl, halogenated C₁₋₄ alkoxy, C₁₋₄ alkylsulfonyl, C₁₋₄ alkylthio, cyclopropyl, cyclobutyl, cyclopentyl, cyclohexyl, cycloheptyl, phenyl, naphthyl, oxacyclopropyl, oxacyclobutyl, oxacyclopentyl, oxacyclohexyl, oxacycloheptyl, pyrrolyl, furanyl, thienyl, oxazolyl, isoxazolyl, pyrazolyl, thiazolyl, pyridyl, pyrimidinyl, pyridazinyl and oxo;
n is 0, 1, 2, 3 or 4;
q is 0, 1, 2, 3 or 4.

Solution 4: Provided is the compound, or the pharmaceutically acceptable salt, the ester, the stereoisomer or the tautomer thereof according to any one of the preceding solutions, wherein
X¹ is NR^{b}, X² is CR^{a}R^{a}, and X³ is C=O; or
X¹ is CR^{a}R^{a}, X² is NR^{b}, and X³ is C=O; or
X¹ is C=O, X² is CR^{a}R^{a}, and X³ is C=O; or
X¹ is CR^{a}R^{a}, X² is C=O, and X³ is C=O; or
X¹ is CR^{a}R^{a}, X² is CR^{a}R^{a}, and X³ is C=O; or
X¹ is C=O, X² is CR^{a}R^{a}, and X³ is CR^{a}R^{a}; or
X¹ is CR^{a}R^{a}, X² is C=O, and X³ is CR^{a}R^{a}.

Solution 5: Provided is the compound, or the pharmaceutically acceptable salt, the ester, the stereoisomer or the tautomer thereof according to any one of the preceding solutions, wherein
X⁴ is selected from N, and X⁵ is selected from CR^{a}; or
X⁴ is selected from CR^{a}, and X⁵ is selected from CR^{a}; or
X⁴ is selected from N, and X⁵ is selected from N; or
X⁴ is selected from CR^{a}, and X⁵ is selected from N.

Solution 6: Provided is the compound, or the pharmaceutically acceptable salt, the ester, the stereoisomer or the tautomer thereof according to any one of the preceding solutions, wherein
M³ is selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl, optionally substituted C₂₋₈ alkenyl, optionally substituted C₂₋₈ alkynyl, optionally substituted 3-8 membered cycloalkyl and optionally substituted 3-8 membered heterocyclyl;
Cy² is selected from phenyl optionally substituted with one or more R², and 5-6 membered heteroaryl optionally substituted with one or more R², and R² is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and optionally substituted C₁₋₆ alkoxy;
Cy³ is selected from 5-6 membered heteroaryl optionally substituted with one or more R³, and R³ is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and optionally substituted C₁₋₆ alkoxy;
Cy⁴ is selected from 5-9 membered heteroaryl optionally substituted with one or more R⁴, and R⁴ is each independently selected from hydrogen, hydroxyl, halogen, nitro, -NR^{b}R^{c}, -C(O)R^{d}, -C(O)NR^{b}R^{c}, and -NR^{b}C(O)R^{d}; and optionally substituted C₁₋₆ alkyl, optionally substituted C₁₋₆ alkoxy and optionally substituted 5-6 membered heteroaryl;
L is selected from -O-;
R^{a} is present or absent, and when present, is each independently selected from hydrogen and optionally substituted C₁₋₆ alkyl;
R^{b} and R^{c} are present or absent, and when present, are each independently selected from hydrogen and optionally substituted C₁₋₆ alkyl;
R^{d} is present or absent, and when present, is each independently selected from hydrogen and optionally substituted C₁₋₆ alkyl;
n is an integer between 0 and 2;
the substituents involved in the phrase "optionally substituted" are each independently selected from hydroxyl, amino, carboxyl, cyano, nitro, halogen, C₁₋₆ alkyl and C₁₋₆ alkoxy.

Solution 7: Provided is the compound, or the pharmaceutically acceptable salt, the ester, the stereoisomer or the tautomer thereof according to any one of the preceding solutions, wherein
Cy³ represents a group shown in 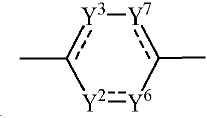 optionally substituted with one or more R³, wherein 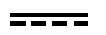 represents a single bond or a double bond, Y², Y³, Y⁶ and Y⁷ are each independently selected from CR^{a}R^{a} and NR^{b}, and at least one of the above is NR^{b}, and preferably, Cy³ represents the group shown in 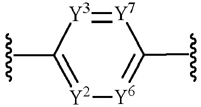 optionally substituted with one or more R³, wherein Y², Y³, Y⁶ and Y⁷ are each independently selected from CH and N, and at least one of the above is N;
R³ is each independently selected from hydrogen, hydroxyl, halogen, carboxyl, nitro, -NR^{b}R^{c}, -C(O)R^{d}, -C(O)NR^{b}R^{c}, -NR^{b}C(O)R^{d}, optionally substituted C₁₋₆ alkyl, and optionally substituted C₁₋₆ alkoxy; preferably, R³ is each independently selected from hydrogen, hydroxyl, halogen, C₁₋₆ alkyl, hydroxyl C₁₋₆ alkyl, amino C₁₋₆ alkyl, halogenated C₁₋₆ alkyl, C₁₋₆ alkoxy, and halogenated C₁₋₆ alkoxy; preferably, R³ is each independently selected from hydrogen, hydroxyl, fluoro, chloro, bromo and C₁₋₄ alkyl.

Solution 8: Provided is the compound, or the pharmaceutically acceptable salt, the ester, the stereoisomer or the tautomer thereof according to any one of the preceding solutions, wherein
Cy⁴ represents a group shown in 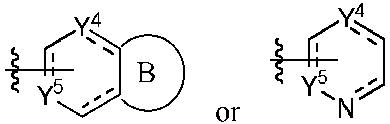 optionally substituted with one or more R⁴, wherein 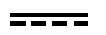 represents a single bond or a double bond, Y⁴ and Y⁵ are each independently selected from CR^{a}R^{a} and NR^{b}, ring B forms a five membered ring, and when Y⁴ and Y⁵ are CR^{a}R^{a}, ring B forms a five membered heteroaryl ring containing NR^{b}, and preferably, Cy⁴ represents 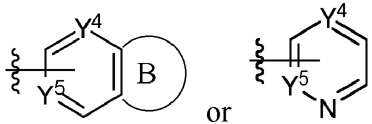 optionally substituted with one or more R⁴, wherein Y⁴ and Y⁵ are each independently CH, and ring B is a five membered heteroaryl ring containing 1 to 2 NR^{b};
R⁴ is each independently selected from hydrogen, hydroxyl, halogen, carboxyl, nitro, -NR^{b}R^{c}, -C(O)R^{d}, -C(O)NR^{b}R^{c}, -NR^{b}C(O)R^{d}, optionally substituted C₁₋₆ alkyl, optionally substituted C₁₋₆ alkoxy, and 5-6 membered heteroaryl; preferably, R⁴ is each independently selected from hydrogen, hydroxyl, halogen, amino, (C₁₋₆ alkyl)₁₋₂ amino-, C₁₋₆ alkyl, hydroxyl C₁₋₆ alkyl, amino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkyl C₁₋₆ alkoxy, C₁₋₆ alkoxy C₁₋₆ alkoxy, pyrrolyl, furanyl, thienyl, oxazolyl, isoxazolyl, pyrazolyl, imidazolyl, thiazolyl, pyridyl, pyrimidinyl and pyridazinyl; preferably, R⁴ is selected from hydrogen, hydroxyl, sulfydryl, halogen, amino, (C₁₋₄ alkyl)₁₋₂ amino-, C₁₋₄ alkyl, halogenated C₁₋₄ alkyl, C₁₋₄ alkoxy, C₁₋₄ alkoxy C₁₋₄ alkyl, C₁₋₄ alkyl C₁₋₄ alkoxy, C₁₋₄ alkoxy C₁₋₄ alkoxy, halogenated C₁₋₄ alkoxy, pyrrolyl, furanyl, thienyl, oxazolyl, isoxazolyl, pyrazolyl and imidazolyl.

Solution 9: Provided is the compound, or the pharmaceutically acceptable salt, the ester, the stereoisomer or the tautomer thereof according to any one of the preceding solutions, wherein
M³ is selected from hydrogen, hydroxyl, halogen, nitro, C₁₋₆ alkyl, C₂₋₆ alkenyl, C₂₋₆ alkynyl, 3-6 membered cycloalkyl and 5-6 membered heterocyclyl;
Cy² is selected from 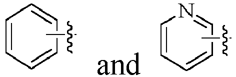 optionally substituted with one or more R², and R² is each independently selected from hydroxyl, fluoro, chloro, bromo and C₁₋₄ alkyl;
Cy³ is selected from 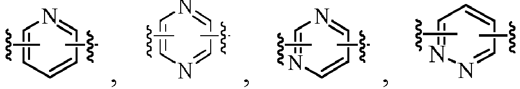 and 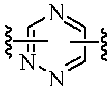 optionally substituted with one or more R³, and preferably from the following groups optionally substituted with one or more R³: 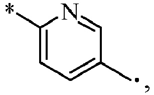 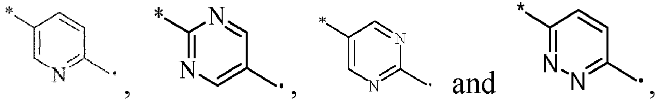 wherein the ^{∗} end is linked to N, the · end is linked to L, and R³ is each independently selected from hydroxyl, fluoro, chloro, bromo and C₁₋₄ alkyl;
Cy⁴ is selected from 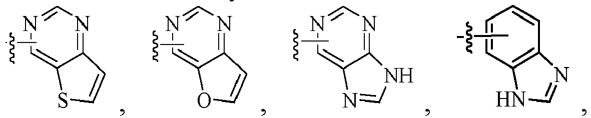 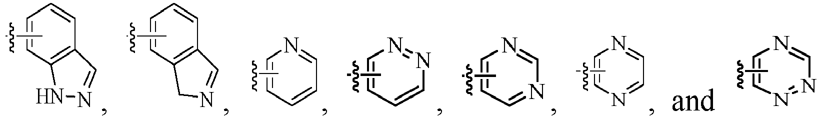 optionally substituted with one or more R⁴, and R⁴ is each independently selected from hydroxyl, halogen, amino, (C₁₋₄ alkyl)₁₋₂ amino-, C₁₋₄ alkyl, halogenated C₁₋₄ alkyl, C₁₋₄ alkoxy, halogenated C₁₋₄ alkoxy, pyrrolyl, furanyl, thienyl, oxazolyl, isoxazolyl, pyrazolyl and imidazolyl.

Solution 10: Provided is the compound, or the pharmaceutically acceptable salt, the ester, the stereoisomer or the tautomer thereof according to any one of the preceding solutions, wherein Cy⁴ is selected from 5-9 membered heterocyclyl optionally substituted with one or more R⁴, and 5-9 membered heteroaryl optionally substituted with one or more R⁴.

Solution 11: Provided is the compound, or the pharmaceutically acceptable salt, the ester, the stereoisomer or the tautomer thereof according to any one of the preceding solutions, wherein M³ represents methyl, ethyl, propyl, isopropyl, butyl, isobutyl, *sec*-butyl, *tert-butyl,* cyclopropyl, cyclobutyl, cyclopentyl, vinyl, allyl or propenyl.

In one embodiment of the present invention, provided is a compound of formula (I), or a pharmaceutically acceptable salt, an ester, a stereoisomer or a tautomer thereof, wherein the compound is selected from:

| No. | Structure | No. | Structure |
|---|---|---|---|
| 1 | 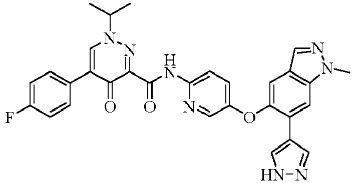 | 2 | 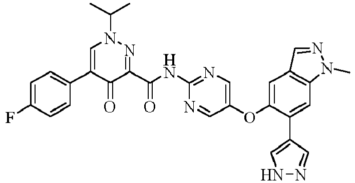 |
| 3 | 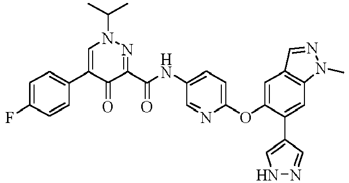 | 4 | 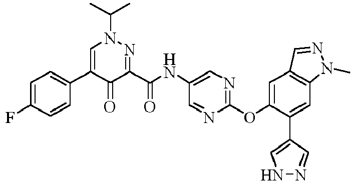 |
| 5 | 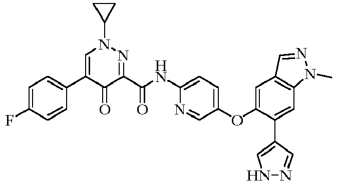 | 6 | 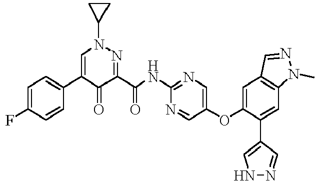 |
| 7 | 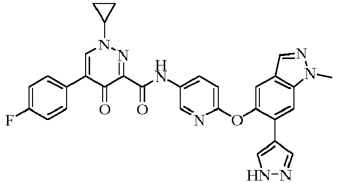 | 8 | 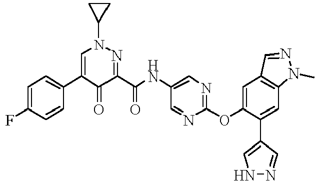 |
| 9 | 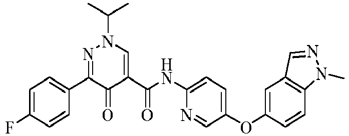 | | |
| 11 | 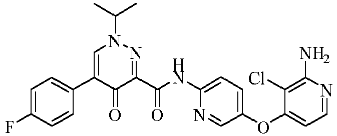 | 12 | 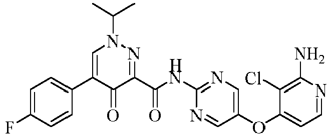 |
| 13 | 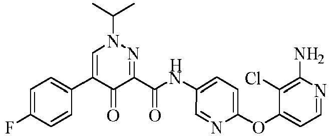 | 16 | 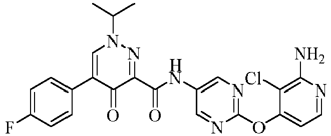 |
| 17 | 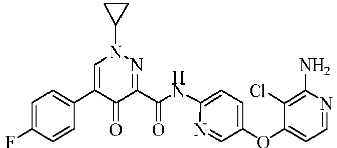 | 18 | 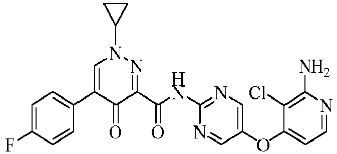 |
| 19 | 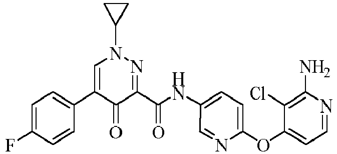 | 20 | 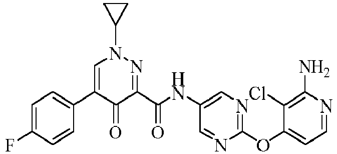 |
| 21 | 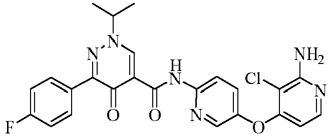 | 22 | 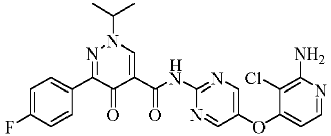 |
| 23 | 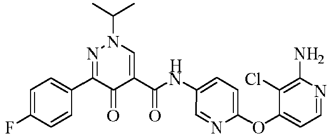 | 24 | 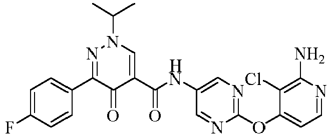 |
| 25 | 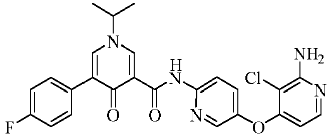 | 26 | 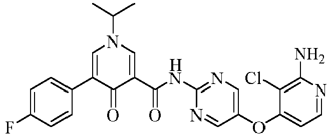 |
| 27 | 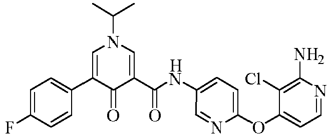 | 28 | 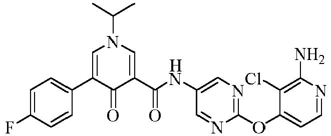 |
| 29 | 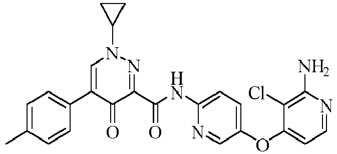 | 30 | 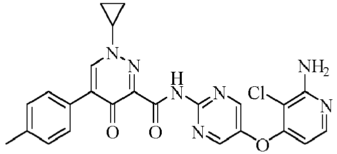 |
| 31 | 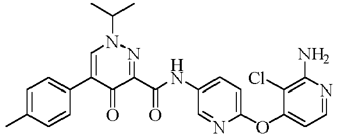 | 32 | 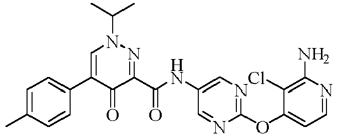 |
| 33 | 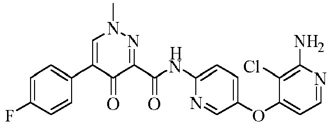 | 34 | 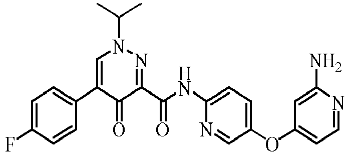 |
| 35 | 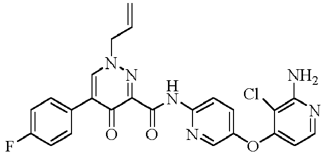 | 36 | 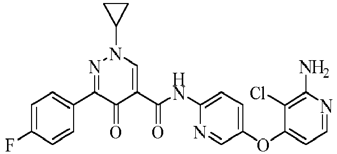 |
| 37 | 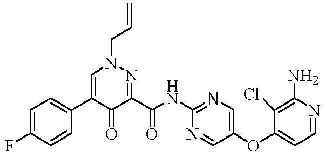 | 38 | 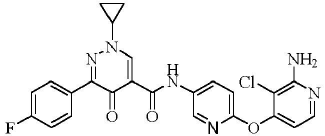 |
| 39 | 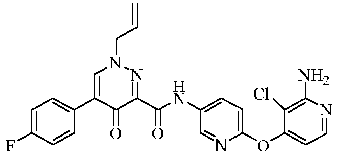 | 40 | 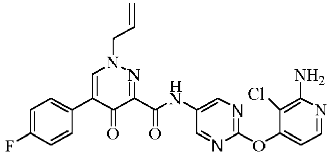 |
| 41 | 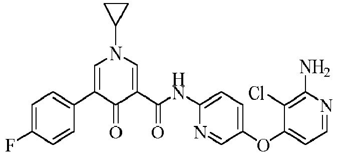 | 42 | 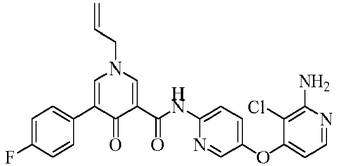 |
| 43 | 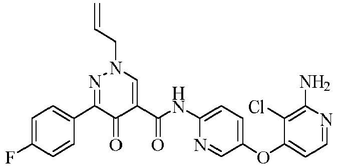 | 44 | 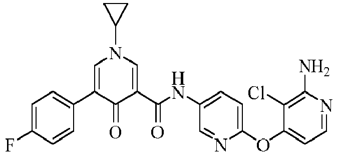 |
| 45 | 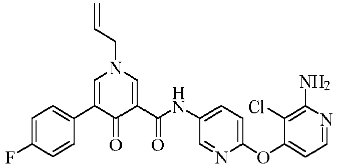 | 46 | 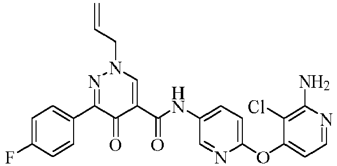 |
| 47 | 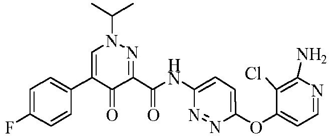 | 48 | 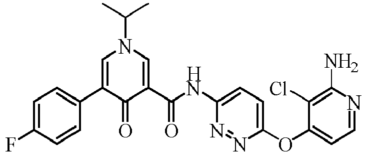 |
| 49 | 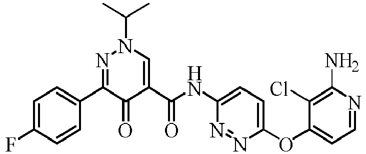 | 50 | 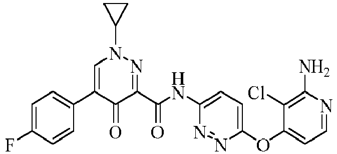 |
| 51 | 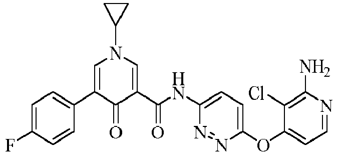 | 52 | 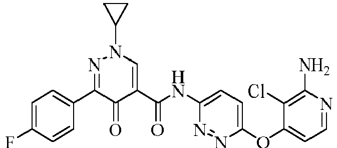 |
| 53 | 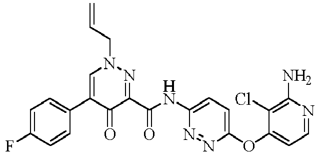 | 54 | 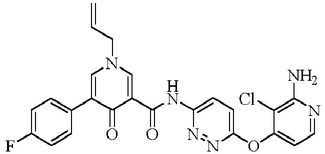 |
| 55 | 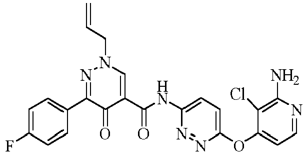 | 56 | 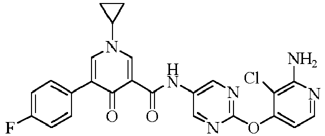 |
| 57 | 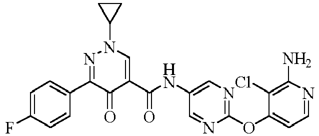 | 58 | 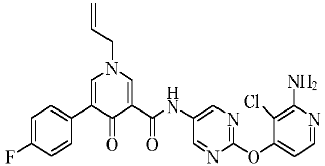 |
| 59 | 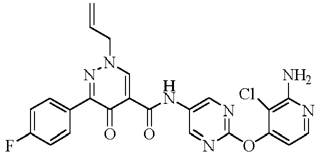 | 60 | 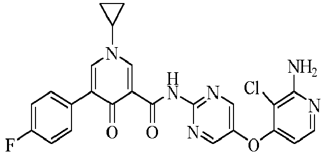 |
| 61 | 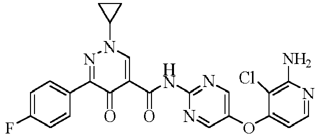 | 62 | 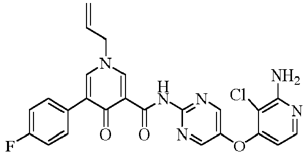 |
| 63 | 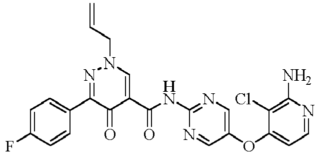 | | |

The present invention also provides a pharmaceutical composition, which comprises at least one of the compounds of any of the above formulas (I), and the pharmaceutically acceptable salt, the ester, the stereoisomer and the tautomer thereof.

The present invention also provides a pharmaceutical composition comprising the compound of any of the above formulas (I), or the pharmaceutically acceptable salt, the ester, the stereoisomer or the tautomer thereof, which may optionally contain one or more pharmaceutically acceptable carriers.

The present invention also provides a pharmaceutically acceptable formulation comprising the compound of any of the above formulas (I), or the pharmaceutically acceptable salt, the ester, the stereoisomer or the tautomer thereof, which optionally contains one or more pharmaceutically acceptable carriers.

In one embodiment of the present invention, provided is a pharmaceutical composition or a formulation comprising the compound of any of the above formulas (I), or the pharmaceutically acceptable salt, the ester, the stereoisomer or the tautomer thereof, which may optionally contain at least one second therapeutically active agent.

In one embodiment of the present invention, the second therapeutically active agent refers to at least one selected from antimetabolites, growth factor inhibitors, mitosis inhibitors, anti-tumor hormones, alkylating agents, platinum metal, topoisomerase inhibitors, hormone drugs, immunomodulators, tumor suppressor genes, cancer vaccines, immune checkpoint inhibitors and antibodies associated with tumor immunotherapy, small molecule drugs and cytotherapeutic agents.

In one embodiment of the present invention, the pharmaceutical composition or the formulation may be administered to a patient or subject in need of treatment and/or prevention by any suitable administration means known in the art, for example, by oral, parenteral (including subcutaneous, intramuscular, intravenous, intraarterial, intradermal, intrathecal and epidural), transdermal, rectal, nasal, transpulmonary, topical (including buccal and sublingual), vaginal, intraperitoneal, intrapulmonary and intranasal administration, and the like.

In one embodiment of the present invention, the pharmaceutical composition or the formulation may be prepared into a conventional solid formulation, such as tablet, capsule, pill and granule, and may also be prepared into an oral liquid formulation, such as oral solution, oral suspension and syrup. In the preparation of an oral formulation, an appropriate filler, binder, disintegrant, lubricant and the like may be added. For parenteral administration, the pharmaceutical composition can be prepared as an injection, a sterile powder for injection and a concentrated solution for injection. The injection can be produced by a conventional method existing in the pharmaceutical field, and during the preparation process, no additive may be added, or an appropriate additive may be added according to the property of the medicament. For rectal administration, the pharmaceutical composition can be prepared into a suppository and the like. For transpulmonary administration, the pharmaceutical composition can be prepared into a inhalant, spray or the like.

In one embodiment of the present invention, provided is use of the compound of any of the above formulas (I), or the pharmaceutically acceptable salt, the ester, the stereoisomer or the tautomer thereof, the pharmaceutical composition and the formulation described above in the manufacture of a medicament for the treatment and/or prevention of diseases mediated by abnormal expression of TAM family kinase receptors and/or ligands thereof, wherein the diseases mediated by abnormal expression of TAM family kinase receptors and/or ligands thereof include at least one of the following diseases: tumor, immuno-oncology, endometriosis, vascular disease/trauma, psoriasis, visual defect/lesion (caused by macular degeneration, diabetes, premature delivery, etc.), kidney disease, rheumatoid arthritis, osteoporosis and other related diseases.

In one embodiment of the present invention, provided is a method of the compound of any of the above formulas (I), or the pharmaceutically acceptable salt, the ester, the stereoisomer or the tautomer thereof, the pharmaceutical composition and the formulation described above for use in the treatment and/or prevention of diseases mediated by abnormal expression of TAM family kinase receptors and/or ligands thereof, wherein the diseases mediated by abnormal expression of TAM family kinase receptors and/or ligands thereof include at least one of the following diseases: tumor, immuno-oncology, endometriosis, vascular disease/trauma, psoriasis, visual defect/lesion (caused by macular degeneration, diabetes, premature delivery, etc.), kidney disease, rheumatoid arthritis, osteoporosis and other related diseases.

In one embodiment of the present invention, provided is use of the compound of any of the above formulas (I), or the pharmaceutically acceptable salt, the ester, the stereoisomer or the tautomer thereof, the pharmaceutical composition and the formulation described above in the manufacture of a medicament for the treatment and/or prevention of diseases mediated by abnormal expression of TAM family kinases and/or Ron kinase receptors and/or ligands thereof, wherein the diseases mediated by abnormal expression of TAM family kinases and/or Ron kinase receptors and/or ligands thereof include at least one of the following diseases: tumor, immuno-oncology, endometriosis, vascular disease/trauma, psoriasis, visual defect/lesion (caused by macular degeneration, diabetes, premature delivery, etc.), kidney disease, rheumatoid arthritis, osteoporosis and other related diseases.

In one embodiment of the present invention, provided is a method of the compound of any of the above formulas (I), or the pharmaceutically acceptable salt, the ester, the stereoisomer or the tautomer thereof, the pharmaceutical composition and the formulation described above for use in the treatment and/or prevention of diseases mediated by abnormal expression of TAM family kinases and/or Ron kinase receptors and/or ligands thereof, wherein the diseases mediated by abnormal expression of TAM family kinases and/or Ron kinase receptors and/or ligands thereof include at least one of the following diseases: tumor, immuno-oncology, endometriosis, vascular disease/trauma, psoriasis, visual defect/lesion (caused by macular degeneration, diabetes, premature delivery, etc.), kidney disease, rheumatoid arthritis, osteoporosis and other related diseases.

In one embodiment of the present invention, the tumor includes sarcoma, lymphoma and cancer, and specifically may be respiratory system cancer, mesothelioma, nervous system tumor, skin malignancy, bone cancer, squamous cell carcinoma, breast cancer, head and neck cancer, urinary and reproductive system cancer, biliary tract system cancer, sarcoma, digestive system cancer, leukemia, lymphoma, myelodysplastic syndrome, carcinoma in situ, or cytoma.

In one embodiment of the present invention, the tumor includes sarcoma, lymphoma and cancer, and specifically may be lung cancer, thyroid cancer, oral cancer, pharyngeal cancer, peritoneal cancer, glioma, neurofibromatosis, skin cancer, melanoma, multiple myeloma, squamous lung cancer, esophageal squamous cancer, ductal breast cancer, brain cancer, ovarian cancer, uterine cancer, endometrial cancer, prostate cancer, testicular cancer, bladder cancer, kidney cancer, renal pelvis cancer, bile duct cancer, gallbladder cancer, osteosarcoma, liposarcoma, Ewing's sarcoma, liver cancer, stomach cancer, esophageal cancer, large intestine cancer, pancreatic cancer, cardia cancer, gastrointestinal stromal tumor, large intestine villous adenoma, acute leukemia, chronic leukemia, non-Hodgkin's malignant lymphoma (NHL), T/NK cell lymphoma, Hodgkin's lymphoma (HL), myelodysplasia syndrome, carcinoma in situ, or cytoma.

### Effect of the invention

The compound of the present invention has an inhibitory effect on the TAM family kinase and can be used for the treatment and/or prevention of diseases mediated by abnormal expression of TAM family kinase receptors and/or ligands thereof. In addition, the compound of the present invention can also target Ron kinase and exhibits an inhibitory effect on Ron kinase. In addition, the compound of the present invention can be used for the treatment and/or prevention of diseases mediated by abnormal expression of TAM family kinase and/or Ron kinase receptors and/or ligands thereof. By means of targeted inhibition of TAM family kinases and/or Ron kinases, the compound of the present invention can reverse the immunosuppressive environment in a tumor microenvironment, inhibit the growth, migration and/or drug resistance performance of tumors, and exert anti-tumor immunological effects and anti-tumor efficacy. In addition, the compound of the present invention can act selectively on the TAM family kinases and/or Ron kinases, while avoiding unnecessary inhibitory effects on other kinases.

Furthermore, with a long half-life and excellent metabolic stability *in vivo* and excellent druggability, the compound of the present invention can improve the medicament efficacy, reduce the medication burden on a patient, and improve the compliance of a patient.

### DETAILED DESCRIPTION

The embodiments of the present invention will be described in more detail in conjunction with specific implementations below, but those skilled in the art will understand that the specific implementations described below are only used to illustrate the present invention and should not be regarded as limiting the protection scope of the present invention. On the contrary, the present invention is intended to encompass all alternatives, modifications and equivalents that can be included within the scope of the present invention as defined by the claims. Unless otherwise specified, the various embodiments of the present invention can be combined in any manner, and the conversions, modifications, and changes of the technical solutions thus obtained are also included in the scope of the present invention, and do not exceed the scope of the present invention.

In the context of the present invention, unless clearly defined otherwise, or the meaning is beyond the understanding of those skilled in the art, a hydrocarbon or hydrocarbon derivative group with 3 or more carbon atoms (such as propyl, propoxy, butyl, butane, butene, butenyl and hexane) is deemed as normal either with or without the prefix of "normal". For example, propyl is generally considered as *n*-propyl, and butyl is generally considered as *n*-butyl, unless otherwise specified.

All publications, patent applications, patents, and other references mentioned in this specification are hereby incorporated by reference. Unless otherwise defined, all technical and scientific terms used in this specification have the meanings conventionally understood by those skilled in the art. In case of conflict, the definition in this specification shall control.

In the context of this specification, in addition to the contents clearly stated, any matter or item not mentioned is directly applicable to those known in the art without any change. Moreover, any embodiment described herein can be freely combined with one or more other embodiments described herein, and the resulting technical solutions or technical ideas are regarded as part of the original disclosure or original record of the present invention, and should not be regarded as new contents that have not been disclosed or anticipated herein, unless those skilled in the art believe that the combination is obviously unreasonable.

In the present invention, the expression of "C_{a-b} group" (a and b represent an integer ≥ 1, and a < b) means that the "group" has a to b carbon atoms, for example, C₁₋₄ alkyl represents alkyl with 1 to 4 carbon atoms, C₁₋₄ alkoxy represents alkoxy with 1 to 4 carbon atoms, C₃₋₁₀ cycloalkyl represents cycloalkyl with 3 to 10 carbon atoms, and C₁₋₄ alkoxy C₁₋₄ alkyl represents a group formed by bonding alkoxy having 1 to 4 carbon atoms with alkyl having 1 to 4 carbon atoms.

In the present invention, "group" represents a monovalent group or a divalent or higher group meeting the valence as required. For example, "cycloalkyl" (also expressed as cycloalkyl group) includes a monovalent group obtained by removing one hydrogen atom from cycloalkane, as well as a divalent or higher group obtained by removing two or more hydrogen atoms from the same carbon atom or two or more different carbon atoms of cycloalkane. As a terminal group, "cycloalkyl" is a monovalent group; and as a linking group in the structure, "cycloalkyl" is a divalent or higher group. In the present invention, a monovalent or divalent or higher group generally means a monovalent group or a divalent group, but the group may be higher in valence (for example, trivalent, tetravalent, pentavalent, or hexavalent) as required. A person skilled in the art can definitely determine the valence of a "group". The group "derived by removing one or more hydrogen atoms" described in the present invention means a monovalent group obtained by removing one hydrogen atom, a divalent group obtained by removing two hydrogen atoms, a trivalent group obtained by removing three hydrogen atoms, a tetravalent group obtained by removing four hydrogen atoms, and the like, and the number of hydrogen atoms to be removed can be determined according to the valence (for example, monovalent, divalent, trivalent, or tetravalent) of the group.

The "halogen" described in the present invention refers to fluorine, chlorine, bromine, and iodine. It is preferably fluorine, chlorine and bromine.

The "halogenated" described in the present invention means that a hydrogen atom on any carbon atom in a substituent is substituted with one or more of the same or different halogen atoms. "Halogen" is defined as above.

The "C₁₋₆ alkyl" described in the present invention refers to linear or branched alkyl derived by removing one or more hydrogen atoms from an alkane moiety containing 1 to 6 carbon atoms, and includes linear C₁₋₆ alkyl and branched C₁₋₆ alkyl. In fact, it is well-known by those skilled in the art that C₁₋₆ alkyl has at least three carbon atoms when having a branch chain (branched C₁₋₆ alkyl). Examples of "C₁₋₆ alkyl" may include, for example, methyl, ethyl, *n*-propyl, isopropyl, *n*-butyl, isobutyl, *sec*-butyl, *tert-butyl, n*-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like. "C₁₋₆ alkyl" preferably includes "C₁₋₄ alkyl", and the "C₁₋₄ alkyl" refers to the above examples containing 1 to 4 carbon atoms.

The "hydroxyl C₁₋₆ alkyl", "cyano C₁₋₆ alkyl", "amino C₁₋₆ alkyl", "C₁₋₆ alkylamino C₁₋₆ alkyl", "halogenated C₁₋₆ alkyl" and "C₁₋₆ alkoxy C₁₋₆ alkyl" described in the present invention refer to groups formed by substituting hydrogen atoms on C₁₋₆ alkyl independently with one or more hydroxyl, cyano, amino, C₁₋₆ alkylamino, halogen and C₁₋₆ alkoxy.

The group containing "C₁₋₆ alkyl" such as "C₁₋₆ alkylamino", "(C₁₋₆ alkyl)₂ amino", "C₁₋₆ alkylaminocarbonyl", "C₁₋₆ alkylcarbonyl", "C₁₋₆ alkylcarbonyloxy", "C₁₋₆ alkylsulfonylamino", "C₁₋₆ alkylsulfonyl", or "C₁₋₆ alkylthio" means a group formed by linking C₁₋₆ alkyl to each of the corresponding groups such as -NH-, -CO-O-, -NH-CO-, -CO-, -SO₂NH-, -SO₂-, and -S-, for example, groups formed by linking each of "C₁₋₆ alkyl" listed above to corresponding groups, such as -NH-, -CO-O-, -NH-CO-, -CO-, -SO₂NH-, -SO₂-, and -S-.

The "C₂₋₈ alkenyl" described in the present invention refers to linear or branched alkenyl derived by removing one or more hydrogen atoms from an alkene moiety containing 2 to 8 carbon atoms and at least one carbon-carbon double bond, for example, vinyl, 1-propenyl, 2-propenyl, 1-butenyl, 2-butenyl, 1,3-butadiene-1-yl, 1-pentene-3-yl, 2-pentene-1-yl, 3-pentene-1-yl, 3-pentene-2-yl, 1,3-pentadiene-1-yl, 1,4-pentadiene-3-yl, 1-hexene-3-yl, and 1,4-hexadiene-1-yl. Preferably, "C₂₋₈ alkenyl" contains a carbon-carbon double bond.

The "C₂₋₈ alkynyl" described in the present invention refers to linear or branched alkynyl derived by removing one or more hydrogen atoms from an alkyne moiety containing 2 to 8 carbon atoms and at least one carbon-carbon triple bond, for example, ethynyl, propynyl, 2-butyn-1-yl, 2-pentyn-1-yl, 3-pentyn-1-yl, 4-methyl-2-pentyn-1-yl, 2-hexyn-1-yl, 2-hexyn-2-yl, 3-hexyn-1-yl, and 3-hexyn-2-yl. Preferably, "C₂₋₈ alkynyl" contains a carbon-carbon triple bond.

The "C₁₋₆ alkoxy" described in the present invention refers to the group obtained by linking an oxygen atom to the parent structure of "C₁₋₆ alkyl" defined above, i.e., "C₁₋₆ alkyl-O-" group, for example, methoxy, ethoxy, *n*-propoxy, isopropoxy, *n*-butoxy, tert-butoxy, *n*-pentyloxy, neopentyloxy and *n*-hexyloxy. The "C₁₋₄ alkoxy" described in the present invention refers to the above examples containing 1 to 4 carbon atoms, i.e., "C₁₋₄ alkyl-O-" group.

The "halogenated C₁₋₆ alkoxy", "C₁₋₆ alkoxy C₁₋₆ alkoxy" and "C₁₋₆ alkyl C₁₋₆ alkoxy" containing "C₁₋₆ alkoxy" described in the present invention refer to groups obtained by substituting one or more hydrogen atoms on C₁₋₆ alkoxy independently with one or more halogen, C₁₋₆ alkoxy and C₁₋₆ alkyl.

The "(group)₁₋₂ amino-" or "-amino (group)₁₋₂" described in the present invention means that one or two hydrogen of amino (-NH₂) can be substituted with the group. For example, "-amino (optionally substituted C₁₋₆ alkyl)₁₋₂" represents -NH (optionally substituted C₁₋₆ alkyl), or -N (optionally substituted C₁₋₆ alkyl)₂, and "(optionally substituted C₁₋₄ alkyl)₁₋₂ amino-" represents (optionally substituted C₁₋₄ alkyl) NH-, or (optionally substituted C₁₋₄ alkyl)₂N-.

The "polycyclic ring" described in the present invention refers to a multi-ring system structure formed by two or more ring structures linked by an ortho-fused, spiro- or bridged linkage. The ortho-fused ring refers to a polycyclic structure formed by two or more ring structures sharing two adjacent ring atoms (i.e., sharing a bond) with each other. The "bridged ring" refers to a polycyclic structure formed by two or more ring structures sharing two non-adjacent ring atoms with each other. The spiro-ring refers to a polycyclic structure formed by two or more ring structures sharing a ring atom with each other.

The "cycloalkyl" or "cycloalkyl group" (hereinafter, collectively referred to as "cycloalkyl") in the present invention refers to a monovalent, divalent or higher (as required) group derived from cycloalkane including monocyclic cycloalkane or polycyclic cycloalkane, and it is, for example, "3-12 membered cycloalkyl", i.e., may have 3, 4, 5, 6, 7, 8, 9, 10, 11 or 12 ring-forming carbon atoms. Unless otherwise specified, a certain membered cycloalkyl includes all possibly formed monocyclic and polycyclic (including fused in the form of ortho-, spiro- or bridged) cases. Cycloalkyl may be a 3-12 membered monovalent, divalent or higher (as required) group, a 3-10 membered monovalent, divalent or higher (as required) group, a 3-8 membered monovalent, divalent or higher (as required) group, a 3-6 membered monovalent, divalent or higher (as required) group, a 4-6 membered monovalent, divalent or higher (as required) group, or a 5-7 membered monovalent, divalent or higher (as required) group.

Specifically, (monovalent, divalent or higher) monocyclic cycloalkyl may include 3-12 membered cycloalkyl, 3-10 membered cycloalkyl, 3-8 membered cycloalkyl, 3-6 membered cycloalkyl, 4-6 membered cycloalkyl, 5-6 membered cycloalkyl and 5-7 membered cycloalkyl. Examples include, but are not limited to: cyclopropyl, cyclobutyl, cyclopentyl, cyclohexyl, cycloheptyl, cyclooctyl, cyclopentyl-1,3-diyl, cyclohexyl- 1,4-diyl, cycloheptyl-1,4-diyl, etc.

(Monovalent, divalent or higher) polycyclic cycloalkyl includes ortho-fused cycloalkyl, bridged cycloalkyl and spiro-cycloalkyl.

(Monovalent, divalent or higher) ortho-fused cycloalkyl may be 6-11 membered ortho-fused cycloalkyl or 7-10 membered ortho-fused cycloalkyl, and representative examples include, but are not limited to, monovalent, divalent or higher groups derived from bicyclo[3.1.1]heptane, bicyclo[2.2.1]heptane, bicyclo[2.2.2]octane, bicyclo[3.2.2]nonane, bicyclo[3.3.1]nonane and bicycle[4.2.1]nonane.

(Monovalent, divalent or higher) bridged cycloalkyl may be a monovalent group obtained by removing one hydrogen atom from a 6-12 membered bridged ring or a 7-11 membered bridged ring, or a divalent or higher group obtained by removing two or more hydrogen atoms from the same carbon atom or different carbon atoms as required.

Examples of bridged ring include, but are not limited to:

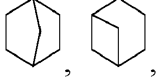

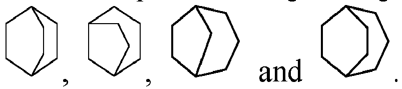

(Monovalent, divalent or higher) spiro-cycloalkyl may be a monovalent group obtained by removing one hydrogen atom from a 7-12 membered spiro-ring or a 7-11 membered spiro-ring, or a divalent or higher group obtained by removing two or more hydrogen atoms from the same carbon atom or different carbon atoms as required. Examples of spiro ring include, but are not limited to: 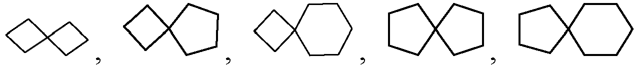 and

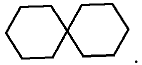

The "cycloalkenyl" described in the present invention refers to a group obtained by having at least one double bond in the above cycloalkyl. It may be, for example, "3-12 membered cycloalkenyl", i.e., may have 3, 4, 5, 6, 7, 8, 9, 10, 11 or 12 ring-forming carbon atoms. Unless otherwise specified, a certain membered cycloalkenyl encompasses all possible monocyclic or polycyclic cycloalkenyl (including fused in the form of ortho-, spiro- and bridged). "3-12 membered cycloalkenyl" may include 3-12 membered cycloalkenyl, 3-8 membered cycloalkenyl, 4-6 membered cycloalkenyl, 7-11 membered spiro cycloalkenyl, 7-11 membered ortho-fused cycloalkenyl, 6-11 membered bridged cycloalkenyl, etc. Examples of cycloalkenyl include, but are not limited to, cyclobutenyl, cyclopentenyl, cyclopentadienyl, cyclohexenyl, 1,4-cyclohexadien-1-yl, cycloheptenyl, 1,4-cycloheptadien-1-yl, cyclooctenyl and 1,5-cyclooctadien-1-yl.

The "heterocyclic ring" described in the present invention includes a non-aromatic cyclic hydrocarbon containing at least one heteroatom (which may be 1 to 5, 1 to 4, 1 to 3, 1 to 2 or 1) selected from O, S and N as a ring-forming atom in the ring. It may be a heterocyclic ring having 3, 4, 5, 6, 7, 8, 9, 10, 11, 12, 13 or 14 ring-forming atoms. There may optionally be at least one double bond in the ring. The heterocyclic ring in the present invention may be a monocyclic system or a polycyclic system (fused in the form of ortho-, spiro- or bridged). Examples of heterocyclic ring may include pyrroline, pyrrolidine, piperidine, piperazine, morpholine, thiomorpholine, tetrahydropyran, dihydropyridine, dihydropyridazine, dioxane, oxathiolane, thiacyclopentane, tetrahydrofuran, tetrahydropyran, tetrahydrothiazole, tetrahydroisothiazole and other monocyclic heterocyclic rings; and indoline, isoindoline, benzopyran, benzodioxane, tetrahydroquinoline, benzo[*d*]oxazol-2(3*H*)-one, tetrahydrobenzothiophene and other polycyclic heterocyclic rings. Furthermore, it may include heterocyclic rings obtained by substituting at least one ring carbon atom in the 7-12 membered spiro ring, 7-11 membered spiro ring, 6-12 membered bridged ring or 7-11 membered bridged ring described above with a heteroatom selected from O, S and N, and preferably with 1 to 4 heteroatoms. Furthermore, it may also include 6-12 membered ortho-fused cyclyl, 7-10 membered ortho-fused cyclyl, 6-10 membered ortho-fused cyclyl, 6-12 membered saturated ortho-fused cyclyl, 6-12 membered spiro-heterocyclyl, 7-11 membered spiro-heterocyclyl, 6-12 membered saturated spiro-heterocyclyl, 7-11 membered saturated spiro-heterocyclyl, 6-12 membered bridged heterocyclyl, 7-11 membered bridged heterocyclyl, 6-12 membered saturated bridged heterocyclyl, and 7-8 membered saturated bridged heterocyclyl, which are described below in the present invention.

The "heterocyclyl" or "heterocyclic group" (hereinafter, generically referred to as "heterocyclyl") described in the present invention refers to a monovalent, divalent or higher group derived from the above "heterocyclyl". The "heterocyclyl" described in the present invention may be a monovalent, divalent or higher non-aromatic cyclic group obtained by substituting at least one ring carbon atom in the cycloalkyl or cycloalkenyl described above with at least one heteroatom selected from O, S and N, and preferably with 1 to 4 heteroatoms. In addition, heterocyclyl also includes the case where a carbon atom or a sulfur atom is substituted with oxygen or nitrogen, for example, the case where the carbon atom or the sulfur atom is substituted with C(=O), S(=O), S(=O)₂ or S(=O)(=N).

Specifically, "heterocyclyl" may be a heterocyclic group having 3, 4, 5, 6, 7, 8, 9, 10, 11, 12, 13 or 14 ring-forming atoms. It may include 3-14 membered heterocyclyl, 3-10 membered heterocyclyl, 4-10 membered heterocyclyl, 3-8 membered heterocyclyl, 4-8 membered heterocyclyl, 4-6 membered heterocyclyl, 3-12 membered heterocyclyl, 4-12 membered heterocyclyl, and 5-9 membered heterocyclyl, including monocyclic heterocyclyl or polycyclic heterocyclyl.

In addition, "heterocyclyl" refers to a monovalent, divalent or higher (as required) monocyclic heterocyclyl, a monovalent, divalent or higher (as required) bicyclic heterocyclyl system, or a monovalent, divalent or higher (as required) polycyclic heterocyclyl system (also referred to as a polycyclic system), and includes saturated, partially saturated heterocyclyl, but does not include an aromatic ring. Unless otherwise specified, all possibly formed monocyclic, polycyclic (including fused in the form of ortho-, spiro- or bridged), saturated, partially saturated cases are included. It may be, for example, "3-14 membered heterocyclyl".

The monovalent, divalent or higher (as required) monocyclic heterocyclyl may include 3-14 membered heterocyclyl, 3-12 membered heterocyclyl, 3-10 membered heterocyclyl, 4-10 membered heterocyclyl, 3-8 membered heterocyclyl, 3-8 membered saturated heterocyclyl, 4-8 membered heterocyclyl, 3-6 membered heterocyclyl, 4-6 membered heterocyclyl, 4-7 membered heterocyclyl, 5-7 membered heterocyclyl, 5-6 membered heterocyclyl, 5-6 membered oxygen-containing heterocyclyl, 3-8 membered nitrogen-containing heterocyclyl, 5-6 membered nitrogen-containing heterocyclyl, 5-6 membered saturated heterocyclyl and the like. Furthermore, it may also include 3-14 membered oxygen-containing heterocyclyl, 3-14 membered nitrogen-containing heterocyclyl, 3-12 membered oxygen-containing heterocyclyl, 3-12 membered sulfur-containing heterocyclyl, 3-12 membered sulfonyl-containing (S(O)₂) heterocyclyl, 3-12 membered sulfinyl-containing (S(O)) heterocyclyl, etc. Examples of "heterocyclyl" include, but are not limited to, azacyclopropyl, ozacyclopropyl, thiocyclopropyl, azacyclobutyl, oxacyclobutyl, thiocyclobutyl, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyl, tetrahydro-2*H*-thiopyranyl, piperidyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H*-imidazolyl, 4,5-dihydro-1*H*-imidazolyl, 4,5-dihydro-1*H*-pyrazolyl, 4,5-dihydro-3*H*-pyrazolyl, 4,5-dihydrothiazolyl, 2,5-dihydrothiazolyl, 2*H*-pyranyl, 4*H*-pyranyl, 2*H*-thiopyranyl, 4*H*-thiopyranyl, 2,3,4,5-tetrahydropyridyl, 1,2-isoxazinyl, 1,4-isoxazinyl, 6*H*-1,3-oxazinyl or the like.

The monovalent, divalent or higher (as required) polycyclic heterocyclyl includes ortho-fused heterocyclyl, spiro-heterocyclyl and bridged heterocyclyl, which may be saturated, partially saturated or unsaturated, but non-aromatic. The polycyclic heterocyclyl may include heterocyclyl obtained by fusing the above heterocyclyl to 6-14 membered aryl (e.g., benzene ring), 3-12 membered cycloalkyl, 3-12 membered cycloalkenyl, 3-14 membered heterocyclyl or 3-14 membered heteroaryl, 5-6 membered monocyclic cycloalkyl, 5-6 membered monocyclic cycloalkenyl, 5-6 membered monocyclic heterocyclyl or 5-6 membered monocyclic heteroaryl.

The ortho-fused heterocyclyl may include 6-12 membered ortho-fused heterocyclyl, 7-10 membered ortho-fused heterocyclyl, 6-10 membered ortho-fused heterocyclyl, and 6-12 membered saturated ortho-fused heterocyclyl, and representative examples include, but are not limited to: 3-azabicyclo[3.1.0]hexyl, 3,6-diazabicyclo[3.2.0]heptyl, 3,8-diazabicyclo[4.2.0]octyl, 3,7-diazabicyclo[4.2.0]octyl, octahydropyrrolo[3,4-*c*]pyrrolyl, octahydropyrrolo[3,4-*b*]pyrrolyl, octahydropyrrolo[3,4-*b*][1,4]oxazinyl, octahydro-1H-pyrrolo[3,4-*c*]pyridyl, 2,3-dihydrobenzofuran-2-yl, 2,3-dihydrobenzofuryl-3-yl, indolin-1-yl, indolin-2-yl, indolin-3-yl, 2,3-dihydrobenzothiophen-2-yl, octahydro-1*H*-indolyl, octahydrobenzofuryl, octahydrocyclopenta[c]pyrrolyl, hexahydrocyclopenta[c]furyl, 2,2-dioxohexahydrocyclopenta[c]thienyl and 2-imino-2-oxo-octahydrocyclopenta[*c*]thienyl.

The spiro-heterocyclyl may be a monovalent group obtained by removing one hydrogen atom from a 6-12 membered spiro heterocyclic ring, a 7-11 membered spiro heterocyclic ring, a 6-12 membered saturated spiro heterocyclic ring or a 7 membered saturated spiro heterocyclic ring, or a divalent or higher group obtained by removing one hydrogen atom from the same carbon atom or different carbon atoms as required, and examples of spiro-heterocyclyl include, but are not limited to:

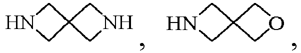

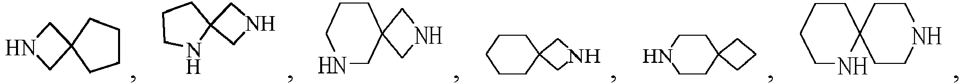

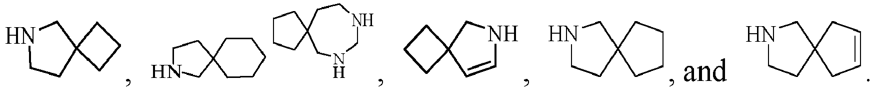

The bridged heterocyclyl may be a monovalent group obtained by removing one hydrogen atom from a 6-12 membered bridged heterocyclic ring, a 7-11 membered bridged heterocyclic ring, a 6-12 membered saturated bridged heterocyclic ring or a 7-8 membered saturated bridged heterocyclic ring, or a divalent or higher group obtained by removing one hydrogen atom from the same carbon atom or different carbon atoms as required, and examples of bridged heterocyclyl include, but are not limited to: 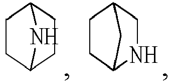 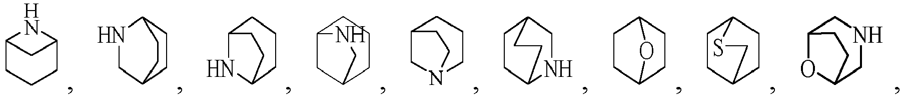 and

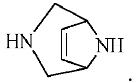

The "aromatic ring" described in the present invention refers to an aromatic carbocyclic hydrocarbon, and may be a monovalent, divalent or higher (as required) group derived from an aromatic carbocyclic hydrocarbon including a 6-14 membered aromatic ring, a 6-10 membered aromatic ring, a 6-8 membered monocyclic aromatic hydrocarbon and a 8-14 membered polycyclic aromatic hydrocarbon. The 6-8 membered monocyclic aryl is, for example, phenyl. The 8-14 membered polycyclic aryl is, for example, naphthyl, phenanthryl, or anthryl. Divalent aryl may include, for example, phenylene, naphthylene and the like.

The "aryl" or "aromatic group" (hereinafter, generically referred to as "aryl") described in the present invention refers to a group containing a monovalent, divalent or higher (as required) group derived from an aromatic carbocyclic hydrocarbon. It includes 6-14 membered aryl and 6-10 membered aryl. The 6-14 membered aryl is, for example, phenyl, naphthyl, phenanthryl, or anthracyl. The 6-10 membered aryl is, for example, phenyl or naphthyl. Divalent aryl may include, for example, phenylene, naphthylene and the like.

The "heteroaryl ring" described in the present invention refers to an aromatic cyclic hydrocarbon having at least one heteroatom (which may be 1 to 5, 1 to 4, 1 to 3, 1 to 2, or 1) selected from O, S and N as a ring-forming atom. It may be a 5, 6, 7, 8, 9, 10, 11, 12, 13 or 14 membered aromatic cyclic group, and preferably one having 1 to 3 heteroatoms. In addition, the heteroaromatic ring of the present invention may be a monocyclic or polycyclic system (fused in the form of ortho-, spiro- or bridged). Specifically, examples may include pyrrole, pyrazine, pyrazole, indole, tetrazole, furan, thiophene, pyridine, imidazole, triazole, triazine, pyridazine, pyrimidine, pyrazine, isoxazole, thiazole, isothiazole, thiadiazole, oxazole, oxadiazole and other monocyclic heteroaromatic rings. Examples may also include isoindole, indazole, indolizine, isoindoline, quinoline, isoquinoline, cinnoline, 2,3-diazanaphthalene, quinazoline, naphthyridine, quinoxaline, purine, pteridine, benzimidazole, benzisoxazole, benzoxazole, benzoxadiazole, benzisothiazole, benzothiazole, benzothiadiazole, benzofuran, isobenzofuran, benzothiophene, benzotriazole, imidazopyridine, triazolopyridine, imidazothiazole, pyrazinopyridazine, benzimidazoline and other polycyclic heteroaromatic rings.

The "heteroaryl" or "heteroaryl group" (hereinafter, collectively referred to as "heteroaryl") described in the present invention refers to a monovalent, divalent or higher group derived from the "heteroaromatic ring". In addition, the "heteroaryl" described in the present invention may also be aromatic cyclic hydrocarbyl containing at least one heteroatom selected from O, S and N and 5, 6, 7, 8, 9, 10, 11, 12, 13 or 14 ring-forming atoms. That is, the heteroaryl may be 5-14 membered heteroaryl, 5-10 membered heteroaryl, 5-9 membered heteroaryl, 5-6 membered heteroaryl, 6 membered heteroaryl, or 6 membered nitrogen-containing heteroaryl. The heteroaryl may have 1, 2, 3, 4 or 5 heteroatoms as ring-forming atoms. In addition, the heteroaryl also includes the case where a carbon atom or a sulfur atom is substituted with oxygen or nitrogen, for example, the case where the carbon atom or the sulfur atom is substituted with C(=O), S(=O), S(=O)₂ or S(=O)(=N). Heteroaryl includes monocyclic heteroaryl and polycyclic heteroaryl. Unless otherwise specified, a certain membered heteroaryl includes all possibly formed monocyclic, polycyclic, fully aromatic and partially aromatic cases. The monocyclic heteroaryl may include 5-7 membered heteroaryl, 5-6 membered heteroaryl, 6 membered heteroaryl, and 6 membered nitrogen-containing heteroaryl, and examples include, but are not limited to, furyl, imidazolyl, isoxazolyl, thiazolyl, isothiazolyl, oxadiazolyl, oxazolyl, pyridyl, pyridazinyl, pyrimidinyl, pyrazinyl, pyrazolyl, pyrrolyl, tetrazolyl, thiadiazolyl, thienyl, triazolyl, and triazinyl. In certain examples, polycyclic heteroaryl refers to a group in which a monocyclic heteroaromatic ring is fused to phenyl, cycloalkenyl, heteroaryl, cycloalkyl or heterocyclyl. Polycyclic heteroaryl may be 8-12 membered ortho-fused heteroaryl, 9-10 membered ortho-fused heteroaryl, 9 membered ortho-fused heteroaryl or 9 membered nitrogen-containing heteroaryl (9 membered nitrogen-containing ortho-fused heteroaryl), and examples include, but are not limited to, benzimidazolyl, benzofuranyl, benzothienyl, benzoxadiazolyl, benzothiadiazolyl, benzothiazolyl, cinnolinyl, 5,6-dihydroquinolin-2-yl, 5,6-dihydroisoquinolin-1-yl, furopyridinyl, indazolyl, indolyl, isoindolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4-yl, 5,6,7,8-tetrahydroisoquinolin-1-yl, thienopyridinyl, 4,5,6,7-tetrahydrobenzo[*c*][1,2,5]oxadiazolyl and 6,7-dihydrobenzo[c][1,2,5]oxadiazol-4(5*H*)keto. The heteroaryl may also be divalent groups derived from the above groups.

The "5-14 membered cyclic group" described in the present invention refers to a group having 5, 6, 7, 8, 9, 10, 11, 12, 13 or 14 ring-forming atoms, which may be the case where the above-mentioned cycloalkyl group, cycloalkenyl group, heterocyclic group, aryl group and heteroaryl group have 5 to 14 ring-forming atoms. Specifically, the 5-14 membered cyclic group may be 5-10 membered cyclic group or 5-6 membered cyclic group. In addition, the "5-6 membered cyclic group" described in the present invention refers to a chemically feasible cyclic structure with 5 to 6 ring atoms which may be optionally selected from C, N, O, S, C(=O), S(=O), S(=O)₂ and S(=O)(=N), and the formed cyclic structure may be a monocyclic ring, a fused polycyclic ring, a saturated ring, a partially saturated ring, or an aromatic ring. Examples include, but are not limited to, groups derived from pyrroline, pyrrolidine, piperidine, piperazine, morpholine, thiomorpholine, tetrahydropyran, dihydropyridine, dihydropyridazine, dioxane, oxathiolane, thiacyclopentane, tetrahydrofuran, tetrahydropyran, tetrahydrothiazole, tetrahydroisothiazole, pyrrole, pyrazine, pyrazole, indole, tetrazole, furan, thiophene, pyridine, imidazole, triazole, tetrazole, triazine, pyridazine, pyrimidine, pyrazine, isoxazole, thiazole, isothiazole, thiadiazole, oxazole, oxadiazole, benzene, etc. Preferably, it is 5-6 membered oxygen-containing cyclic group, i.e., cyclic group having at least one O and 5 or 6 ring-forming atoms.

The "one or more" described in the present invention refers to the number of substituents with which all positions can be chemically substituted in the substituted group, preferably 1 to 6, more preferably 1 to 5, more preferably 1 to 3, more preferably 1 to 2, and more preferably 1.

For the "optionally substituted" described in the present invention, the number of substituents may be 0 (i.e., unsubstituted), or 1 to the number of all chemically substitutable positions in the group to be substituted, preferably 1 to 6, more preferably 1 to 5, more preferably 1 to 4, more preferably 1 to 3, and more preferably 1 to 2 or 1.

The present invention provides a compound of general formula (I), or a pharmaceutically acceptable salt, an ester, a stereoisomer or a tautomer thereof: 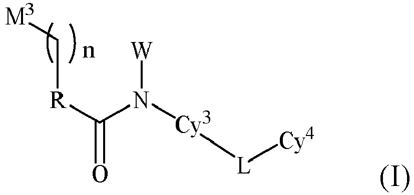 wherein W is selected from hydrogen and optionally substituted C₁₋₆ alkyl;
R represents a group shown as the following general formula (b) or (b'): 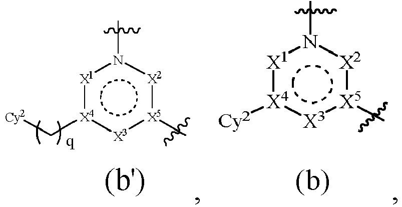 the 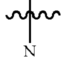 moiety is linked to the M³ group through a linking group;
X¹, X² and X³ are each independently selected from CR^{a}R^{a}, C=O and NR^{b}, and at least one of the above is C=O;
X⁴ and X⁵ are each independently selected from CR^{a} and N;
M³ is selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl; when N of the 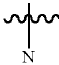 moiety is linked to a double bond in formula (b'), M³ is absent;
Cy² is selected from 3-12 membered cycloalkyl optionally substituted with one or more R², 3-12 membered cycloalkenyl optionally substituted with one or more R², 3-14 membered heterocyclyl optionally substituted with one or more R², 6-14 membered aryl optionally substituted with one or more R², and 5-10 membered heteroaryl optionally substituted with one or more R², and R² is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{C}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
Cy³ is selected from 5-10 membered heteroaryl optionally substituted with one or more R³, and R³ is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{C}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
Cy⁴ is selected from 3-12 membered cycloalkyl optionally substituted with one or more R⁴, 3-14 membered heterocyclyl optionally substituted with one or more R⁴, 5-14 membered heteroaryl optionally substituted with one or more R⁴ and 6-14 membered aryl optionally substituted with one or more R⁴, and R⁴ is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{C}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl and optionally substituted 5-10 membered heteroaryl, or, two R⁴, together with the atoms attached thereto, can form a 5-14 membered cyclic group;
L is selected from -NR^{b}-, -O-, -S-, and -(CR^{a}R^{a})ₘ-, and m is selected from integers between 1 and 3;
R^{a} is present or absent, and when present, is each independently selected from hydrogen, cyano, hydroxyl, sulfydryl, halogen, carboxyl, nitro, -NR^{b}R^{c}, -C(O)R^{d}, -C(O)NR^{b}R^{c}, -OC(O)NR^{b}R^{C}, -NR^{b}C(O)R^{d}, -NR^{b}C(O)OR^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
R^{b} and R^{c} are present or absent, and when present, are each independently selected from hydrogen, hydroxyl, optionally substituted C₁₋₆ alkyl, optionally substituted -C₁₋₆ alkyl-R', -C(O)-R ', -SO₂-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
R^{d} is present or absent, and when present, is each independently selected from hydroxyl, halogen, amino, -amino (optionally substituted C₁₋₆ alkyl)₁₋₂, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', optionally substituted 3-12 membered cycloalkyl, optionally substituted 3-12 membered cycloalkenyl, optionally substituted 3-14 membered heterocyclyl, optionally substituted 6-14 membered aryl, and optionally substituted 5-10 membered heteroaryl;
R' is each independently selected from optionally substituted 3-12 membered cycloalkyl, optionally substituted 3-12 membered cycloalkenyl, optionally substituted 3-14 membered heterocyclyl, optionally substituted 6-14 membered aryl and optionally substituted 5-10 membered heteroaryl;
q is an integer between 0 and 4;
n is an integer between 0 and 4;
the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo.

In one embodiment of the present invention, when M³ represents a group carrying aryl, Cy² does not represent heterocyclyl.

In one embodiment of the present invention, Cy² does not represent heterocyclyl.

In one embodiment of the present invention, when M³ represents a group carrying aryl, Cy² does not represent heteroaryl.

In one embodiment of the invention, Cy² does not represent heteroaryl.

In one embodiment of the invention, when M³ represents optionally substituted C₁₋₆ alkyl, X⁴ represents CR^{a}.

In one embodiment of the invention, when M³ represents a group carrying aryl, X⁴ represents CR^{a}.

In one embodiment of the invention, when M³ represents a group carrying cycloalkyl, X⁴ represents CR^{a}.

In one embodiment of the invention, when M³ represents optionally substituted C₁₋₆ alkyl and X¹ represents CR^{a}R^{a}, the R^{a} group on X¹ does not have carbonyl.

In one embodiment of the present invention, when M³ represents a group carrying aryl, X² does not represent C=O.

In one embodiment of the present invention, M³ is selected from optionally substituted branched C₁₋₆ alkyl and optionally substituted 3-8 membered cycloalkyl.

In one embodiment of the present invention, X¹ is NR^{b}, X² is CR^{a}R^{a}, and X³ is C=O; or X¹ is CR^{a}R^{a}, X² is NR^{b}, and X³ is C=O; or X¹ is C=O, X² is CR^{a}R^{a}, and X³ is C=O; or X¹ is CR^{a}R^{a}, X² is C=O, and X³ is C=O; or X¹ is CR^{a}R^{a}, X² is CR^{a}R^{a}, and X³ is C=O; or X¹ is C=O, X² is CR^{a}R^{a}, and X³ is CR^{a}R^{a}; or X¹ is CR^{a}R^{a}, X² is C=O, and X³ is CR^{a}R^{a}.

In one embodiment of the present invention, X⁴ is selected from N, and X⁵ is selected from CR^{a}; or X⁴ is selected from CR^{a}, and X⁵ is selected from CR^{a}; or X⁴ is selected from N, and X⁵ is selected from N; or X⁴ is selected from CR^{a}, and X⁵ is selected from N.

In one embodiment of the present invention, X¹ does not represent C=O.

In one embodiment of the present invention, X² does not represent C=O.

In one embodiment of the present invention, X¹ is NR^{b}.

In one embodiment of the present invention, X² is NR^{b}.

In one embodiment of the present invention, X³ is C=O.

In one embodiment of the present invention, formulas (b) and (b') carry only 1 carbonyl.

In one embodiment of the present invention, at most two of X¹, X², X³, X⁴ and X⁵ are NR^{b}.

In one embodiment of the present invention, X⁴ represents CR^{a}.

In one embodiment of the present invention, X⁵ represents CR^{a}.

In one embodiment of the present invention, M³ represents methyl, ethyl, propyl, isopropyl, butyl, isobutyl, *sec*-butyl, *tert-butyl,* cyclopropyl, cyclobutyl, cyclopentyl, vinyl, allyl or propenyl.

In one embodiment of the present invention, Cy² is selected from 6-14 membered aryl optionally substituted with one or more R² and 5-10 membered heteroaryl optionally substituted with one or more R².

In one embodiment of the present invention, Cy² is selected from phenyl optionally substituted with one or more R² and 5-6 membered heteroaryl optionally substituted with one or more R².

In one embodiment of the present invention, Cy² is selected from 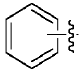 and 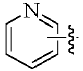 optionally substituted with one or more R².

In one embodiment of the present invention, Cy² represents a group without heterocyclyl. In one embodiment of the present invention, when M³ is a group carrying aryl, Cy² represents a group without carbonyl.

In one embodiment of the present invention, Cy³ is selected from 5-10 membered heteroaryl optionally substituted with one or more R³.

In one embodiment of the present invention, Cy³ is selected from 5-6 membered heteroaryl optionally substituted with one or more R³.

In one embodiment of the present invention, Cy³ represents a group shown in 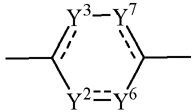 optionally substituted with one or more R³, wherein 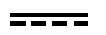 represents a single bond or a double bond, Y², Y³, Y⁶ and Y⁷ are each independently selected from CR^{a}R^{a} and NR^{b}, and at least one of the above is NRb, and preferably, Cy³ represents the group shown in 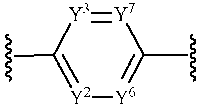 optionally substituted with one or more R³, wherein Y², Y³, Y⁶ and Y⁷ are each independently selected from CH and N, and at least one of the above is N.

In one embodiment of the present invention, Cy³ is selected from 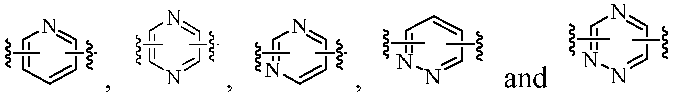 optionally substituted with one or more R³.

In one embodiment of the present invention Cy³ is selected from the following groups optionally substituted with one or more R³: 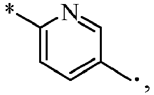 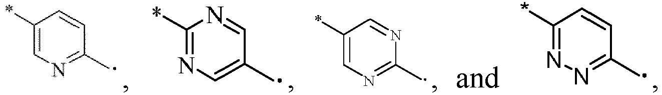 wherein the ^{∗} end is linked to N, and the · end is linked to L.

In one embodiment of the present invention, Cy⁴ is selected from 5-9 membered heteroaryl optionally substituted with one or more R⁴.

In one embodiment of the present invention, Cy⁴ is selected from 5-9 membered heterocyclyl optionally substituted with one or more R⁴.

In one embodiment of the present invention, Cy⁴ is selected from 5-10 membered heterocyclyl optionally substituted with one or more R⁴, 5-10 membered heteroaryl optionally substituted with one or more R⁴, and 6-10 membered aryl optionally substituted with one or more R⁴.

In one embodiment of the present invention, Cy⁴ represents a group shown in 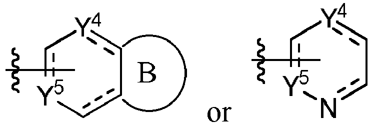 optionally substituted with one or more R⁴, wherein 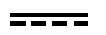 represents a single bond or a double bond, Y⁴ and Y⁵ are each independently selected from CR^{a}R^{a} and NR^{b}, ring B forms a five membered ring, and when Y⁴ and Y⁵ are CR^{a}R^{a}, ring B forms a five membered heteroaryl ring containing NR^{b}, and preferably, Cy⁴ represents 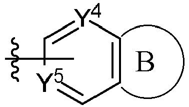 or 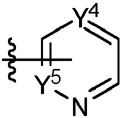 optionally substituted with one or more R⁴, wherein Y⁴ and Y⁵ are each independently CH, and ring B is a five membered heteroaryl ring containing 1 to 2 NR^{b}.

In one embodiment of the present invention, Cy⁴ is selected from 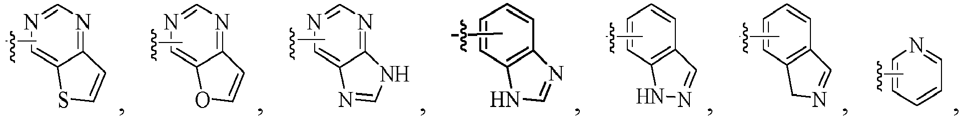 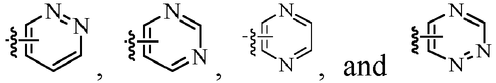 optionally substituted with one or more R⁴.

In one embodiment of the present invention, R² is each independently selected from hydrogen, hydroxyl, halogen, carboxyl, nitro, -NR^{b}R^{c}, -C(O)R^{d}, -C(O)NR^{b}R^{c}, -NR^{b}C(O)OR^{d}, optionally substituted C₁₋₆ alkyl, and optionally substituted C₁₋₆ alkoxy.

In one embodiment of the present invention, R² is each independently selected from hydrogen, hydroxyl, halogen, C₁₋₆ alkyl, hydroxyl C₁₋₆ alkyl, amino C₁₋₆ alkyl, halogenated C₁₋₆ alkyl, C₁₋₆ alkoxy, and halogenated C₁₋₆ alkoxy; preferably, R² is each independently selected from hydrogen, hydroxyl, fluoro, chloro, bromo and C₁₋₄ alkyl.

In one embodiment of the present invention, R³ is each independently selected from hydrogen, hydroxyl, halogen, carboxyl, nitro, -NR^{b}R^{c}, -C(O)R^{d}, -C(O)NR^{b}R^{c}, -NR^{b}C(O)R^{d}, optionally substituted C₁₋₆ alkyl, and optionally substituted C₁₋₆ alkoxy.

In one embodiment of the present invention, R³ is each independently selected from hydrogen, hydroxyl, halogen, C₁₋₆ alkyl, hydroxyl C₁₋₆ alkyl, amino C₁₋₆ alkyl, halogenated C₁₋₆ alkyl, C₁₋₆ alkoxy, and halogenated C₁₋₆ alkoxy; preferably, R³ is each independently selected from hydrogen, hydroxyl, fluoro, chloro, bromo and C₁₋₄ alkyl.

In one embodiment of the present invention, R⁴ is each independently selected from hydrogen, hydroxyl, halogen, carboxyl, nitro, -NR^{b}R^{c}, -C(O)R^{d}, -C(O)NR^{b}R^{c}, -NR^{b}C(O)R^{d}, optionally substituted C₁₋₆ alkyl, optionally substituted C₁₋₆ alkoxy, and optionally substituted 5-10 membered heteroaryl.

In one embodiment of the present invention, R⁴ is each independently selected from hydrogen, hydroxyl, halogen, carboxyl, nitro, -NR^{b}R^{c}, -C(O)R^{d}, -C(O)NR^{b}R^{c}, -NR^{b}C(O)R^{d}, optionally substituted C₁₋₆ alkyl, optionally substituted C₁₋₆ alkoxy, and optionally substituted 5-6 membered heteroaryl.

In one embodiment of the present invention, preferably, R⁴ is each independently selected from hydrogen, hydroxyl, halogen, amino, (C₁₋₆ alkyl)₁₋₂ amino-, C₁₋₆ alkyl, hydroxyl C₁₋₆ alkyl, amino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkyl C₁₋₆ alkoxy, C₁₋₆ alkoxy C₁₋₆ alkoxy, pyrrolyl, furanyl, thienyl, oxazolyl, isoxazolyl, pyrazolyl, imidazolyl, thiazolyl, pyridyl, pyrimidinyl and pyridazinyl.

In one embodiment of the present invention, R⁴ is selected from hydrogen, hydroxyl, sulfydryl, halogen, amino, (C₁₋₄ alkyl)₁₋₂ amino-, C₁₋₄ alkyl, halogenated C₁₋₄ alkyl, C₁₋₄ alkoxy, C₁₋₄ alkoxy C₁₋₄ alkyl, C₁₋₄ alkyl C₁₋₄ alkoxy, C₁₋₄ alkoxy C₁₋₄ alkoxy, halogenated C₁₋₄ alkoxy, pyrrolyl, furyl, thienyl, oxazolyl, isoxazolyl, pyrazolyl and imidazolyl.

In one embodiment of the present invention, R^{a} present or absent, and when present, is each independently selected from hydrogen, halogen, hydroxyl, carboxyl, nitro, -NR^{b}R^{c}, -C(O)R^{d}, -C(O)NR^{b}R^{c}, -NR^{b}C(O)R^{d}, optionally substituted C₁₋₆ alkyl, and optionally substituted C₁₋₆ alkoxy.

In one embodiment of the present invention, R^{a} is present or absent, and when present, is each independently selected from hydrogen, hydroxyl, halogen, carboxyl, nitro, C₁₋₆ alkyl, C₁₋₆ alkoxy and halogenated C₁₋₆ alkyl.

In one embodiment of the present invention, R^{a} is present or absent, and when present, is each independently selected from hydrogen, C₁₋₆ alkyl and C₁₋₆ alkoxy.

In one embodiment of the present invention, R^{b} and R^{c} are present or absent, and when present, are each independently selected from hydrogen and optionally substituted C₁₋₆ alkyl.

In one embodiment of the present invention, R^{b} and R^{c} are present or absent, and when present, are each independently selected from hydrogen, C₁₋₆ alkyl and halogenated C₁₋₆ alkyl.

In one embodiment of the present invention, R^{b} and R^{c} are present or absent, and when present, are each independently selected from hydrogen and C₁₋₆ alkyl.

In one embodiment of the present invention, R^{d} is present or absent, and when present, is each independently selected from hydroxyl, halogen, amino, -amino (optionally substituted C₁₋₆ alkyl)₁₋₂, optionally substituted C₁₋₆ alkyl, and optionally substituted C₁₋₆ alkoxy.

In one embodiment of the present invention, R^{d} is present or absent, and when present, is each independently selected from hydroxyl, amino, -amino (C₁₋₆ alkyl)₁₋₂, C₁₋₆ alkyl, halogenated C₁₋₆ alkyl, C₁₋₆ alkoxy and halogenated C₁₋₆ alkoxy.

In one embodiment of the present invention, R^{d} is present or absent, and when present, is each independently selected from hydroxyl, amino, C₁₋₄ alkyl, halogenated C₁₋₄ alkyl, C₁₋₄ alkoxy and halogenated C₁₋₄ alkoxy.

In one embodiment of the present invention, R' is selected from optionally substituted 3-8 membered cycloalkyl, optionally substituted 3-8 membered cycloalkenyl, optionally substituted 3-10 membered heterocyclyl, optionally substituted 6-10 membered aryl and optionally substituted 5-6 membered heteroaryl.

In one embodiment of the present invention, R' is selected from optionally substituted 3-6 membered cycloalkyl, optionally substituted 4-7 membered heterocyclyl, optionally substituted 6-10 membered aryl and optionally substituted 5-6 membered heteroaryl.

In one embodiment of the present invention, R' is selected from cyclopropyl, cyclobutyl, cyclopentyl, cyclohexyl, phenyl, naphthyl, oxacyclopropyl, oxacyclobutyl, oxacyclopentyl, oxacyclohexyl, oxacycloheptyl, pyrrolyl, furanyl, thienyl, oxazolyl, isoxazolyl, pyrazolyl, thiazolyl, pyridyl, pyrimidinyl and pyridazinyl.

In one embodiment of the present invention, the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-8 membered cycloalkyl, 6-10 membered aryl, 3-8 membered heterocyclyl, 5-6 membered heteroaryl and oxo.

In one embodiment of the present invention, the substituents involved in the phrase "optionally substituted" are each independently selected from hydroxyl, sulfydryl, amino, carboxyl, cyano, nitro, halogen, C₁₋₄ alkyl, C₁₋₄ alkoxy C₁₋₄ alkyl, C₁₋₄ alkoxy, C₁₋₄ alkyl C₁₋₄ alkoxy, C₁₋₄ alkoxy C₁₋₄ alkoxy, C₁₋₄ alkylamino, (C₁₋₄ alkyl)₂amino, C₁₋₄ alkylaminocarbonyl, (C₁₋₄ alkyl)₂aminocarbonyl, C₁₋₄ alkylcarbonyl, C₁₋₄ alkylcarbonyloxy, C₁₋₄ alkylcarbonylamino, C₁₋₄ alkylsulfonylamino, halogenated C₁₋₄ alkyl, halogenated C₁₋₄ alkoxy, C₁₋₄ alkylsulfonyl, C₁₋₄ alkylthio, cyclopropyl, cyclobutyl, cyclopentyl, cyclohexyl, cycloheptyl, phenyl, naphthyl, oxacyclopropyl, oxacyclobutyl, oxacyclopentyl, oxacyclohexyl, oxacycloheptyl, pyrrolyl, furanyl, thienyl, oxazolyl, isoxazolyl, pyrazolyl, thiazolyl, pyridyl, pyrimidinyl, pyridazinyl and oxo.

In one embodiment of the present invention, n is 0, 1, 2, 3 or 4. When n is 0, M³ is directly bonded to the N atom of formula (b) or formula (b').

In one embodiment of the present invention, q is 0, 1, 2, 3 or 4. When q is 0, Cy² is directly bonded to X⁴.

In one embodiment of the present invention, L is selected from -NR^{b}-, -O- and -S-, and preferably -O-.

In the present invention, when N of the 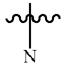 moiety is linked to a double bond in formula (b) or formula (b'), M³ is absent. That is, in this case, the N atom of the 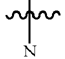 moiety is present in formula (b) or formula (b'); since it is linked to a double bond (or a large π bond is formed when a heteroaromatic ring is formed), the N atom is not bonded to other atoms than adjacent ring-forming atoms. The "ester" described in the present invention refers to a pharmaceutically acceptable ester formed by the compound of the present invention, and more specifically to formate, acetate, propionate, butyrate, acrylate, ethyl succinate and other esters of the compound of the present invention, but not limited thereto.

The "pharmaceutically acceptable salt" described in the present invention refers to a pharmaceutically acceptable addition salt of acid and base or a solvate thereof. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid and CH₃-(CH₂)ₙ-COOH (wherein n is 0 to 4)), etc. The following salts of bases are also included: sodium salt, potassium salt, calcium salt, ammonium salt, and the like. Those skilled in the art know a variety of pharmaceutically acceptable non-toxic addition salts.

The hydrogen atom, fluorine atom, carbon atom, nitrogen atom, oxygen atom, sulfur atom and the like in the present invention also include the respective radioactive isotopes or stable isotopes thereof.

The tumor described in the present invention includes sarcoma, lymphoma and cancer, and may be respiratory system cancer, mesothelioma, nervous system tumor, skin malignancy, bone cancer, squamous cell carcinoma, breast cancer, head and neck cancer, urinary and reproductive system cancer, biliary tract system cancer, sarcoma, digestive system cancer, leukemia, lymphoma, myelodysplastic syndrome, carcinoma in situ, or cytoma.

The tumor described in the present invention specifically may be lung cancer, thyroid cancer, oral cancer, pharyngeal cancer, peritoneal cancer, glioma, neurofibromatosis, skin cancer, melanoma, multiple myeloma, squamous lung cancer, esophageal squamous cancer, ductal breast cancer, brain cancer, ovarian cancer, uterine cancer, endometrial cancer, prostate cancer, testicular cancer, bladder cancer, kidney cancer, renal pelvis cancer, bile duct cancer, gallbladder cancer, osteosarcoma, liposarcoma, Ewing's sarcoma, liver cancer, stomach cancer, esophageal cancer, large intestine cancer, pancreatic cancer, cardia cancer, gastrointestinal stromal tumor, large intestine villous adenoma, acute leukemia, chronic leukemia, non-Hodgkin's malignant lymphoma (NHL), T/NK cell lymphoma, Hodgkin's lymphoma (HL), myelodysplasia syndrome, carcinoma in situ, or cytoma.

In the present invention, the expression of "A and/or B" refers to A alone or both A and B. For example, "TAM family kinase and/or Ron kinase" refers to "TAM family kinase" alone or both "TAM family kinase" and "Ron kinase".

The stereoisomer of the compound of formula (I) of the present invention refers to an enantiomer in the case that atoms are asymmetric in the compound of formula (I), and a cis-trans isomer in the case that a carbon-carbon double bond or a cyclic structure exists in a compound. All enantiomers, diastereomers, racemic isomers, cis-trans isomers, geometric isomers, epimers and mixtures thereof of the compound of formula (I) are included in the scope of the present invention. The definition of the compounds of the present invention encompasses all possible stereoisomers and mixtures thereof. In particular, it includes racemic forms and isolated optical isomers with specified activities. The racemic forms can be resolved by physical methods, such as fractional crystallization, separation or crystallization of diastereomeric derivatives, or chiral column chromatography. An individual optical isomer can be obtained from the racemate by conventional methods (e.g., salifying with an optically active acid, followed by crystallization).

The "tautomer" of the compound of formula (I) of the present invention refers to the functional isomer produced when an atom in the compound of formula (I) moves rapidly between two positions. When the hydrogen at the α position of the carbonyl-containing functional group is on the α carbon, a keto tautomer is produced; and when the hydrogen at the α position of the carbonyl-containing functional group is on the oxygen of the carbonyl, an enol tautomer is produced.

The pharmaceutical composition of the present invention comprises at least one of the compounds of formula (I), and a pharmaceutically acceptable salt, an ester, a stereoisomer and a tautomer thereof.

The pharmaceutical composition of the present invention comprises the compound of formula (I), or a pharmaceutically acceptable salt, an ester, a stereoisomer or a tautomer thereof, and optionally one or more pharmaceutically acceptable carriers.

The pharmaceutical composition of the present invention can be administered to a patient or subject in need of treatment and/or prevention in any suitable administration means known in the art, for example, oral, parenteral (including subcutaneous, intramuscular, intravenous, intra-arterial, intradermal, intrathecal, and epidural), transdermal, rectal, nasal, transpulmonary, topical (including buccal and sublingual), vaginal, intraperitoneal, intrapulmonary, intranasal administration, and the like.

The pharmaceutical composition of the present invention can be prepared into a conventional solid formulation, such as tablet, capsule, pill and granule, and can also be prepared into an oral liquid formulation, such as oral solution, oral suspension and syrup. In the preparation of an oral formulation, one or more of suitable excipient, diluent, sweetener, solubilizer, lubricant, binder, tablet disintegrant, stabilizer, preservative and encapsulating material may be added. For parenteral administration, the pharmaceutical composition can be prepared as an injection, a sterile powder for injection and a concentrated solution for injection. The injection can be produced by a conventional method existing in the pharmaceutical field, and during the preparation process, no additive may be added, or an appropriate additive may be added according to the property of the medicament. For rectal administration, the pharmaceutical composition can be prepared into a suppository and the like. For transpulmonary administration, the pharmaceutical composition can be prepared into an inhalant, spray or the like. In the present invention, suitable solid carriers include, but are not limited to, cellulose, glucose, lactose, mannitol, magnesium stearate, magnesium carbonate, sodium carbonate, sodium saccharin, sucrose, dextrin, talc, starch, pectin, gelatin, tragacanth, arabic gum, sodium alginate, *p*-hydroxylbenzoate, methylcellulose, sodium carboxymethyl cellulose, low melting wax, cocoa butter, and the like. Suitable liquid carriers include, but are not limited to, water, ethanol, polyol (such as glycerol, propylene glycol, and liquid polyethylene glycol), vegetable oil, glyceride, and mixtures thereof.

Methods for preparing the pharmaceutical composition of the present invention are generally known. The pharmaceutical composition of the present invention is prepared by known methods, including conventional mixing, granulating, tableting, coating, dissolving or lyophilizing.

The pharmaceutical formulation is preferably in unit dosage form. In this form, the formulation is subdivided into unit dosages containing an appropriate quantity of active components. The unit dosage form can be packaged into packages containing a discrete quantity of formulation, such as packaged tablets, capsules, or powders in a vial or an ampoule.

Dosage of a medicament depends on various factors, including the age, weight and state of a patient, as well as the route of administration. The precise dosage administered is determined based on the judgment of a treating physician. A usual dose for administration of the active compound may be, for example, from about 0.01 mg to about 100 mg per day, from about 0.05 mg to about 75 mg per day, from about 0.1 mg to about 50 mg per day, or from about 5 mg to about 10 mg per day. The desired dosage also depends on the specific compound employed, the severity of the disease, the route of administration, the weight and health status of a patient, and the judgment of a treating physician.

### Preparation Method for Compound of General Formula (I) of the Present Invention

The compound of the present invention can be prepared by a variety of methods including standard chemical methods. Unless otherwise stated, any variable defined above will continue to have the meaning defined above. Exemplary general synthesis methods are elaborated in the following schemes, and can be easily modified to prepare other compounds disclosed herein. Those skilled in the art can perform the following reactions according to a conventional method (such as Organic Synthesis 2nd, Michael B. Smith etc.) taught in the art. The specific compounds of the present invention were specifically prepared in examples.

The method for preparing the compound of general formula (I) can be prepared, for example, by the following method, but is not limited thereto. 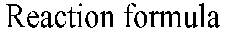 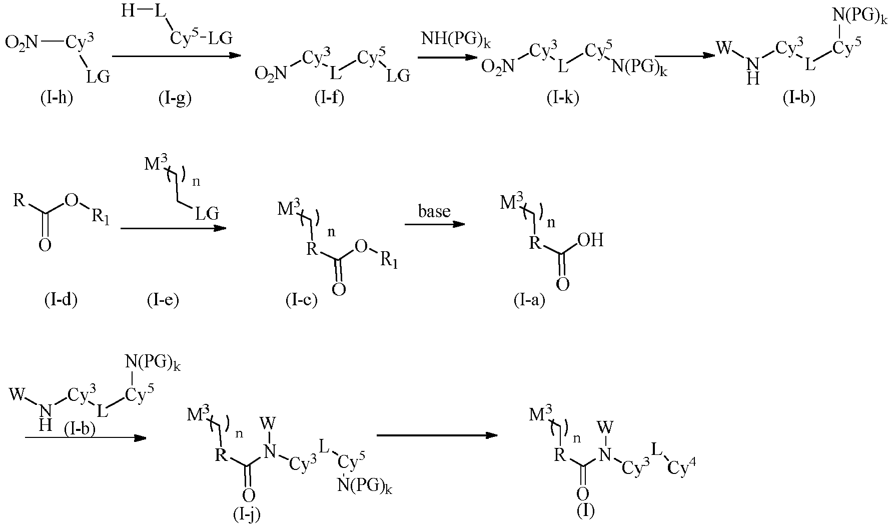 wherein, as shown above, the compound of formula (I-j) prepared from formula (I-a) and formula (I-b) is further deprotected to give the compound of formula (I). 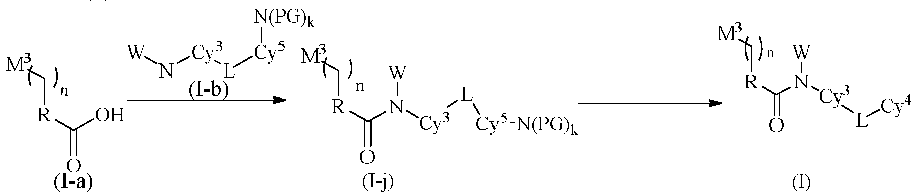 wherein, formula (I-a) is added into a solvent, a peptide coupling reagent, a base and formula (I-b) are added, the mixture is stirred until the reaction is completed, and then formula (I-j) is obtained by separation; or
formula (I-a) and formula (I-b) are added into a base, a coupling reagent is added dropwise, the mixture is stirred until the reaction is completed, then formula (I-j) is obtained by separation,
formula (I-j) is added into a solvent, an acid is added, the mixture is stirred until the reaction is completed, and formula (I) is obtained by separation;
wherein M³, W, R, n, Cy³, L and Cy⁴ are as defined above;
Cy⁵ is derived from the group defined by Cy⁴;
k is an integer between 0 and 2;
PG is a protective group, and the protective group known in the art may be used, and may be selected from, for example, *tert*-butoxycarbonyl, benzyloxycarbonyl, acetyl, and *p*-methoxybenzyl;
LG is a leaving group or boric acid or boric acid ester, and the leaving group known in the art may be used, and may be selected from, for example, chlorine, bromine, iodine, mesylate, and besylate;
R₁ is C₁₋₆ alkyl.

As shown above, k represents the number of PG groups linked to an N atom to which a hydrogen atom is optionally linked such that the valence of the N atom can be trivalent.

The solvent is selected from: one of *N*,*N*-dimethylformamide, *N*,*N*-dimethylacetamide, dimethylsulfoxide, toluene, benzene, dimethylbenzene, trimethylbenzene, cyclohexane, hexane, dichloromethane, chloroform, 1,2-dichloroethane, tetrahydrofuran, diethyl ether, dioxane, 1,2-dimethoxyethane, methyl acetate, ethyl acetate, acetone, methyl ethyl ketone, acetonitrile, methanol, ethanol, isopropanol, *tert*-butanol and water, and a mixture thereof;
the base is selected from: one of methylamine, ethylamine, propylamine, *N*,*N*-diisopropylethylamine, trimethylamine, triethylamine, dicyclohexylamine, ethanolamine, diethanolamine, triethanolamine, meglumine, diethanolamine, ethylenediamine, pyridine, picoline and quinoline, and a mixture thereof;
the acid is selected from: formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, *p*-toluenesulfonic acid, methanesulfonic acid, and ethanesulfonic acid; hydrochloric acid, sulfuric acid, nitric acid, carbonic acid, hydrobromic acid, phosphoric acid, and hydroiodic acid;
the peptide coupling reagent is selected from: one of 2-(7-azobenzotriazol)-tetramethyluronium hexafluorophosphate (HATU), benzotriazol-*N*,*N*,*N*,*N*-tetramethyluronium hexafluorophosphate (HBTU), and 2-(1*H*-benzo[d][1,2,3]trisazo-1-yl)-1,1,3,3-tetramethyluronium tetrafluoroborate (TBTU), and a mixture thereof;
the coupling reagent is selected from: one of phosphorus oxychloride, dicyclohexylcarbodiimide (DCC), *N*,*N*-carbonyldiimidazole, isobutyl chloroformate, 1-*n*-propylphosphoric anhydride and the like, and a mixture thereof.

The compound of formula (I) can be prepared from the compound of formula (I-j) by a method conventional to those skilled in the art, and a method for preparing the compound of formula (I) from the compound of formula (I-j) is schematically provided as follows:
For example, the compound of formula (I-j) is added to a suitable solvent (e.g., methanol, tetrahydrofuran, *N*,*N*-dimethylformamide, or *N*,*N*-dimethylacetamide, and preferably, *N*,*N*-dimethylformamide), water is added, a suitable acid (formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, *p*-toluenesulfonic acid, methanesulfonic acid, or ethanesulfonic acid; hydrochloric acid, sulfuric acid, nitric acid, carbonic acid, hydrobromic acid, phosphoric acid, or hydroiodic acid) is added, the mixture is stirred at room temperature for an appropriate time (e.g., 10 to 30 minutes), after the reaction is completed as monitored, an appropriate amount of water is added, pH is adjusted, the reaction solution is extracted with a suitable extractant (e.g., ethyl acetate), the extract is concentrated, and then the crude product is separated by a conventional purification method (e.g., silica gel column chromatography) to give the compound of formula (I).

As shown above, the compound of formula (I-j) can be prepared by the reaction of a compound of formula (I-a) and a compound of formula (I-b), 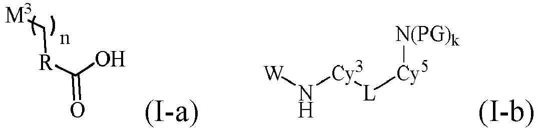 wherein M³, R, n, W, Cy³, L, PG, k and Cy⁵ are as defined above.

The compound of formula (I-j) can be prepared from the compound of formula (I-a) and the compound of formula (I-b) by a method conventional to those skilled in the art, and a method for preparing the compound of formula (I-j) by the reaction of the compound of formula (I-a) and the compound of formula (I-b) is schematically provided as follows:

For example, the compound of formula (I-a) is added to a suitable solvent (e.g., tetrahydrofuran, *N*,*N*-dimethylformamide, or *N*,*N*-dimethylacetamide, and preferably, *N*,*N*-dimethylformamide), a suitable peptide coupling reagent (preferably, 2-(7-benzotriazole oxide)-*N*,*N*,*N*,*N*-tetramethyluronium hexafluorophosphate) is added, then a suitable base (e.g., triethylamine, pyridine, or *N*,*N*-diisopropylethylamine, and preferably, *N*,*N*-diisopropylethylamine) is added, and the mixture is cooled to an appropriate temperature (e.g., 0 °C) and stirred for an appropriate time (e.g., 10 to 30 minutes), added with the compound of formula (I-b), and stirred at an appropriate temperature (e.g., from 0 °C to ambient temperature) for a suitable time (e.g., 1 to 16 hours); the reaction solution is concentrated under reduced pressure, added with an appropriate amount of water and extracted with a suitable extractant (such as ethyl acetate), the extract is concentrated, and the crude product is separated by a conventional purification method (such as silica gel column chromatography) to give the compound of formula (I-j).

For another example, the compound of formula (I-a) and the compound of formula (I-b) are added to a suitable base (e.g., pyridine), the mixture is cooled to an appropriate temperature (e.g., 0 °C), added dropwise with a coupling reagent (e.g., phosphorus oxychloride), and stirred for a suitable time (e.g., 0.5 to 3 hours) until the reaction is completed, then the reaction solution is concentrated under reduced pressure, added with an appropriate amount of water, and extracted with a suitable extractant (e.g., ethyl acetate), the extract is concentrated, and the crude product is separated by a conventional purification method (e.g., silica gel column chromatography) to give the compound of formula (I-j).

As shown above, the compound of formula (I-a) can be prepared from a compound of formula (I-c), 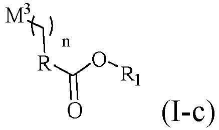 wherein R₁ is C₁₋₆ alkyl, and M³, R and n are as defined above. The compound of formula (I-a) can be prepared from the compound of formula (I-c) by a method conventional to those skilled in the art, and a method for preparing the compound of formula (I-a) from the compound of formula (I-c) is schematically provided as follows:
For example, the compound of formula (I-c) is dissolved in a suitable solvent (e.g., methanol, ethanol, tetrahydrofuran, or dioxane, and preferably, methanol), a suitable base (e.g., lithium hydroxide, sodium hydroxide, or potassium hydroxide, and preferably, lithium hydroxide) is added, the mixture is stirred at an appropriate temperature (e.g., room temperature) for an appropriate time (e.g., 0.5 to 3 hours), after the reaction is completed, the reaction solution is concentrated under reduced pressure, pH is adjusted to a suitable range (e.g., 2 to 5) with an appropriate aqueous solution of an acid (e.g., hydrochloric acid or citric acid), and then the compound of formula (I-a) is obtained by filtration.

The compound of formula (I-c) can be prepared by the reaction of a compound of formula (I-d) and a compound of formula (I-e), 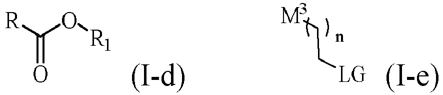 wherein R and R₁ are as defined above, and LG, M³ and n are as defined above. The compound of formula (I-c) can be prepared from the compound of formula (I-d) and the compound of formula (I-e) by a method conventional to those skilled in the art, and a method for preparing the compound of formula (I-c) by the reaction of the compound of formula (I-d) and the compound of formula (I-e) is schematically provided as follows:
For example, the compound of formula (I-d) and the compound of formula (I-e) (LG is a leaving group) are dissolved in a suitable solvent (e.g., acetonitrile, tetrahydrofuran, or *N*,*N*-dimethylformamide, and preferably, *N*,*N*-dimethylformamide), an appropriate base (e.g., sodium carbonate, potassium carbonate, or cesium carbonate, and preferably, potassium carbonate) is added, the mixture is stirred at an appropriate temperature (e.g., 50 °C) for an appropriate time (e.g., 10 to 16 hours), after the reaction is completed, water and a suitable extractant are added, and the crude product is separated by a conventional purification method (e.g., silica gel column chromatography) to give the compound of formula (I-c).

For another example, the compound of formula (I-d) and the compound of formula (I-e) (LG is boric acid or boric acid ester) are dissolved in a suitable solvent (e.g., acetonitrile, dichloromethane, chloroform, pyridine, or *N*,*N*-dimethylformamide, and preferably, *N*,*N*-dimethylformamide), an appropriate base (e.g., triethylamine, or pyridine) and an appropriate catalyst (e.g., copper acetate, or copper chloride) are added, and the mixture is reacted in air or under oxygen atmosphere at an appropriate temperature (e.g., room temperature to 50 °C) for an appropriate time (e.g., 14 hours). After the reaction is completed, the crude product is obtained through conventional post-treatment (such as extraction and concentration), and then the product is obtained through conventional purification means (such as silica gel column chromatography).

As shown above, the compound of formula (I-b) can be prepared by the reaction of a compound of formula (I-k), 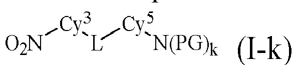 wherein Cy³, L, k, PG and Cy⁵ are as defined above. The compound of formula (I-b) can be prepared from the compound of formula (I-k) by a method conventional to those skilled in the art, and a method for preparing the compound of formula (I-b) from the compound of formula (I-k) is schematically provided as follows:
For example, the compound of formula (I-k) and a suitable reducing agent (e.g., iron powder, zinc powder, stannous chloride, or sodium dithionite, and preferably, iron powder, ammonium sulfide, or ferrous sulfate heptahydrate) are added to an appropriate solvent (e.g., ethanol, aqueous ammonium chloride solution, acetic acid, aqueous ammonia, or a mixture of ethanol and aqueous ammonium chloride solution), and the mixture is reacted at an appropriate temperature (e.g., 80 °C) for an appropriate time (e.g., 2 hours). After the reaction is completed, the reaction solution is filtered, concentrated under reduced pressure, extracted with a suitable extractant (such as dichloromethane), and concentrated under reduced pressure to give the product.

As shown above, the compound of formula (I-k) can be prepared by the reaction of a compound of formula (I-f), 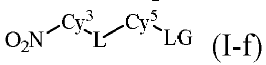 wherein L, Cy³, Cy⁵ and LG are as defined above; the compound of formula (I-k) can be prepared from the compound of formula (I-f) by a method conventional to those skilled in the art, and a method for preparing the compound of formula (I-k) from the compound of formula (I-f) is schematically provided as follows:
For example, the compound of formula (I-f) is added to an appropriate solvent (e.g., 1,4-dioxane, tetrahydrofuran, acetonitrile, *N*,*N*-dimethylformamide, or *N*,*N*-dimethylacetamide,and preferably, *N*,*N*-dimethylformamide), an appropriate catalyst (such as palladium acetate or palladium chloride) is added, an appropriate ligand (such as, BINAP, XPhos, SPhos or Ruphos) is added, an appropriate base (e.g., potassium carbonate, sodium carbonate, triethylamine, or *N*,*N*-diisopropylethylamine, and preferably, potassium carbonate) is added, NH(PG)ₖ (e.g., benzophenone imine, *p*-methoxybenzylamine, or tert-butylamine) is added, and the mixture is stirred at an appropriate temperature (e.g., room temperature to 110 °C) for an appropriate time (e.g., 14 hours). After the reaction is completed, the crude product is obtained by conventional post-treatment methods (such as filtration and extraction), and then separated by a conventional purification method (such as silica gel column chromatography) to give the product.

As shown above, the compound of formula (I-f) can be prepared by the reaction of a compound of formula (I-g) with a compound of formula (I-h), 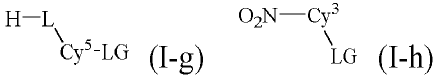 wherein L, Cy⁵, Cy³ and LG are as defined above.

The compound of formula (I-f) can be prepared from the compound of formula (I-g) and the compound of formula (I-h) by a method conventional to those skilled in the art, and a method for preparing the compound of formula (I-f) by the reaction of the compound of formula (I-g) and the compound of formula (I-h) is schematically provided as follows:
For example, the compound of formula (I-g) and the compound of formula (I-h) are added to an appropriate solvent (e.g., tetrahydrofuran, acetonitrile, *N*,*N*-dimethylformamide, or *N*,*N*-dimethylacetamide,and preferably *N*,*N*-dimethylformamide), an appropriate base (e.g., potassium carbonate, sodium carbonate, triethylamine, or *N*,*N*-diisopropylethylamine, and preferably potassium carbonate) is added, and the mixture is stirred at an appropriate temperature (e.g., room temperature to 80 °C) for an appropriate time (e.g., 14 hours). After the reaction is completed, the crude product is obtained by conventional post-treatment methods (such as filtration and extraction), and then separated by a conventional purification method (such as silica gel column chromatography) to give the product.

In certain embodiments of the present invention, when R represents a group of general formula (b), the compound of formula (I-c) can be prepared by the following reaction:

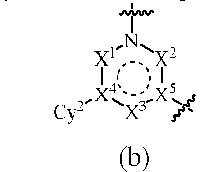

In certain embodiments, the compound of formula (I-c) can be prepared by the following reaction: 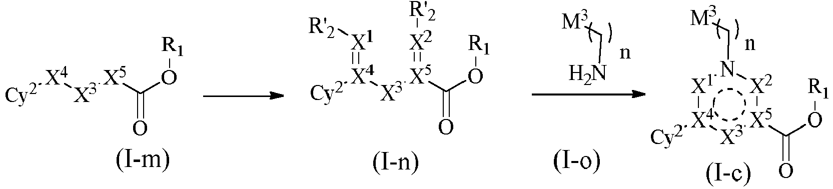 wherein X¹, X², X⁵ and X⁴ are each independently selected from C, CH and CH₂, X³ is C=O, R'₂ is C₁₋₆ alkoxy or disubstituted amino, and Cy², R¹, M³ and n are as defined above. The compound of formula (I-c) can be prepared from a compound of formula (I-m) by a method conventional to those skilled in the art, and a method for preparing the compound of formula (I-c) from the compound of formula (I-m) is schematically provided as follows:

For example, the compound of formula (I-m) is added to an appropriate solvent (e.g., toluene, dimethylbenzene, trimethylbenzene, *N*,*N*-dimethylformamide, or *N*,*N*-dimethylacetamide,and preferably toluene), a suitable reagent (e.g., *N*,*N*-dimethylformamide dimethyl acetal, trimethyl orthoformate, or triethyl orthoformate, and preferably, *N*,*N*-dimethylformamide dimethyl acetal) is added, and the mixture is heated to an appropriate temperature (e.g., 100 °C) and stirred for an appropriate time (e.g., 15 hours). After the reaction is completed, a compound of formula (I-n) is obtained by conventional post-treatment methods (e.g., filtration, extraction, and concentration).

The compound of formula (I-n) and a compound of formula (I-o) are added to a suitable solvent (e.g., ethanol, isopropanol, or toluene), and the mixture is heated to a suitable temperature (e.g., 100 °C) and reacted for an appropriate time (e.g., 3 h). After the reaction is completed, the compound of formula (I-c) is obtained by a conventional purification method (such as silica gel column chromatography).

In other embodiments, the compound of formula (I-c) can be prepared by the following reaction: 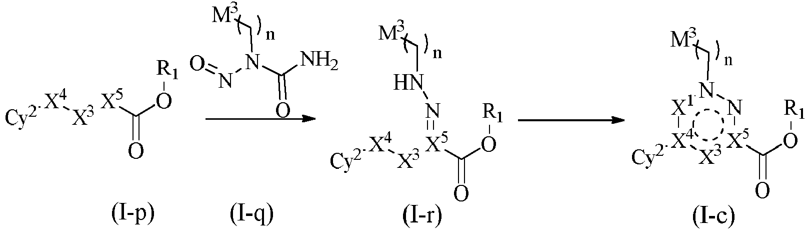 wherein X¹, X⁵ and X⁴ are each independently selected from C, CH and CH₂, X³ is C=O, and Cy², R¹, M³ and n are as defined above. The compound of formula (I-c) can be prepared from a compound of formula (I-p) by a method conventional to those skilled in the art, and a method for preparing the compound of formula (I-c) from the compound of formula (I-p) is schematically provided as follows:
For example, the compound of formula (I-p) and a compound of formula (I-q) are added to an appropriate solvent (such as dichloromethane, chloroform, or 1,2-dichloroethane, and preferably, dichloromethane), the mixture is cooled to an appropriate temperature (such as 0 °C), added with an appropriate base (such as potassium carbonate, sodium carbonate, or sodium hydroxide, and preferably, potassium carbonate), and slowly heated to room temperature for an appropriate time (such as 3 to 15 hours), and after the reaction is completed, a compound of formula (I-r) is obtained by a conventional purification method (such as silica gel column chromatography).

The compound of formula (I-r) is added to an appropriate solvent (e.g., toluene, dimethylbenzene, trimethylbenzene, *N*,*N*-dimethylformamide, or *N*,*N*-dimethylacetamide, and preferably toluene), a suitable reagent (e.g., *N*,*N*-dimethylformamide dimethyl acetal, trimethyl orthoformate, or triethyl orthoformate, and preferably, *N*,*N*-dimethylformamide dimethyl acetal) is added, and the mixture is heated to an appropriate temperature (e.g., 110 °C) and stirred for an appropriate time (e.g., 15 hours). After the reaction is completed, the compound of formula (I-c) is obtained by conventional post-treatment methods (e.g., filtration, extraction, and concentration) and purification methods (e.g., silica gel column chromatography).

In other embodiments, the compound of formula (I-c) can be prepared from the following reaction formulas: 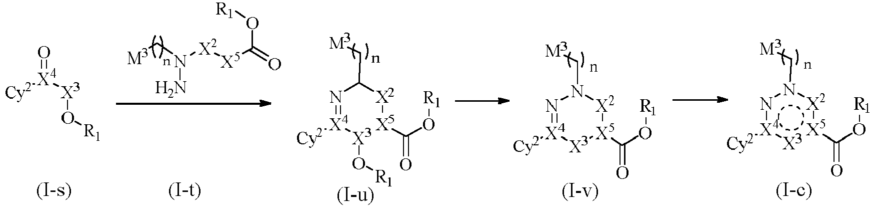 wherein X², X⁵ and X⁴ are each independently selected from C, CH and CH₂, X³ is C=O, and Cy², R¹, M³ and n are as defined above. The compound of formula (I-c) can be prepared from a compound of formula (I-s) by a method conventional to those skilled in the art, and a method for preparing the compound of formula (I-c) from the compound of formula (I-s) is schematically provided as follows:
For example, the compound of formula (I-s) and a compound of formula (I-t) are added to an appropriate solvent (e.g., methanol, ethanol, or isopropanol, and preferably, ethanol), and the mixture is reacted at an appropriate temperature (e.g., 80 °C) for an appropriate time (e.g., 15 hours). After the reaction is completed, a compound of formula (I-u) is obtained by a conventional purification method (such as silica gel column chromatography).

The compound of formula (I-u) is added to an appropriate solvent (e.g., tetrahydrofuran, 2-methyltetrahydrofuran, *N*,*N*-dimethylformamide, *N*,*N*-dimethylacetamide, or ethanol, and preferably, 2-methyltetrahydrofuran), and the mixture is cooled to an appropriate temperature (e.g., 0 °C), added with an appropriate base (e.g., sodium hydride, sodium *tert*-butoxide, potassium *tert*-butoxide, or sodium ethoxide, and preferably, potassium *tert*-butoxide), and then heated to an appropriate temperature (e.g., room temperature) and stirred for an appropriate time (e.g., 15 hours). After the reaction is completed, the pH value of the reaction solution is adjusted to an appropriate value (such as 5 to 6), and a compound of formula (I-v) is obtained by conventional post-treatment methods (such as filtration, extraction, and concentration) and a purification method (such as silica gel column chromatography).

The compound of formula (I-v) is added to an appropriate solvent (e.g., acetonitrile), an appropriate oxidant (e.g. copper chloride) is added, and the mixture is heated to an appropriate temperature (e.g., 80 °C) for an appropriate time (e.g., 2 to 4 hours). After the reaction is completed, the compound of formula (I-c) is obtained by conventional post-treatment methods (e.g., filtration, extraction, and concentration) and purification methods (e.g., silica gel column chromatography).

Wherein the compound of formula (I-t) can be prepared from the following reaction formulas: 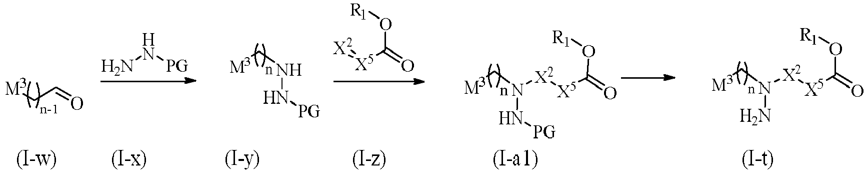 wherein X² and X⁵ are each independently selected from CH and CH₂, PG is a protective group (such as *tert*-butoxycarbonyl, benzyloxycarbonyl, acetyl, or *p*-methoxybenzyl) known in the art, R¹, M³ and n are as defined above, and n is more than or equal to 1. The compound of formula (I-t) can be prepared from a compound of formula (I-w) by a method conventional to those skilled in the art, and a method for preparing the compound of formula (I-t) from the compound of formula (I-w) is schematically provided as follows:

For example, the compound of formula (I-w) and a compound of formula (I-x) are added to a suitable solvent (e.g., dichloromethane, methanol, or ethanol, and preferably, dichloromethane), an appropriate acid (e.g., acetic acid) and a dehydrating agent (e.g., magnesium sulfate) are added, an appropriate reducing agent (e.g., sodium triacetoxyborohydride, or sodium cyanoborohydride) is then added, and the reaction is carried out at an appropriate temperature (e.g., room temperature) for an appropriate time (e.g., 15 hours). After the reaction is completed, a compound of formula (I-y) is obtained by conventional post-treatment methods (e.g., filtration, extraction, and concentration);
the compound of formula (I-y) and a compound of formula (I-z) are added to an appropriate solvent (e.g., ethanol), and the mixture is reacted at an appropriate temperature (e.g., 80 °C) for an appropriate time (e.g., 15 hours). After the reaction is completed, a compound of formula (I-a1) is obtained by conventional post-treatment methods (e.g., filtration, extraction, and concentration) and a purification method (e.g., silica gel column chromatography);
the compound of formula (I-a1) is treated with an appropriate deprotection method (e.g., acid, base, hydrogenation, and oxidation) to give the compound of formula (I-t) by conventional post-treatment methods (e.g., filtration, extraction, and concentration) and purification methods (e.g., recrystallization, and silica gel column chromatography).

In other embodiments, the compound of formula (I-a1) can be prepared from the following reaction formulas: 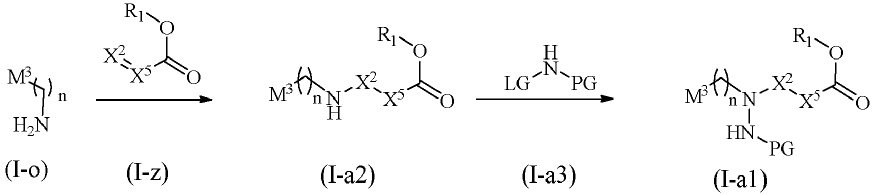 wherein X² and X⁵ are each independently selected from CH and CH₂, PG is a protective group known in the art (e.g., *tert*-butoxycarbonyl, benzyloxycarbonyl, acetyl, or *p*-methoxybenzyl), LG is a leaving group known in the art (e.g., benzenesulfonate, or methanesulfonate), and R₁, M³ and n are as defined above. The compound of formula (I-a1) can be prepared from the compound of formula (I-o) by a method conventional to those skilled in the art, and a method for preparing the compound of formula (I-a1) from the compound of formula (I-o) is schematically provided as follows:
For example, the compound of formula (I-o) is dissolved in an appropriate solvent (e.g., ethanol), and the compound of formula (I-z) is added at an appropriate temperature (e.g., 0 °C to room temperature) for an appropriate time (2 to 15 hours). After the reaction is completed, a compound of formula (I-a2) is obtained by conventional post-treatment methods (such as filtration, extraction, and concentration) and purification methods (such as recrystallization, and silica gel column chromatography).

The compound of formula (I-a2) is added to an appropriate solvent (e.g., dichloromethane, tetrahydrofuran, or 2-methyltetrahydrofuran), an appropriate base (e.g., triethylamine, *N*,*N*-diisopropylethylamine, or *N*-methylmorpholine) is added, and the mixture is cooled to an appropriate temperature (e.g., -5 °C), added with a compound of formula (I-a3), and heated to room temperature and reacted for an appropriate time (e.g., 14 hours). After the reaction is completed, the compound of formula (I-a1) is obtained by conventional post-treatment methods (such as filtration, extraction, and concentration) and purification methods (such as recrystallization, and silica gel column chromatography).

In some embodiments of the present invention, when R represents a group of general formula (b), 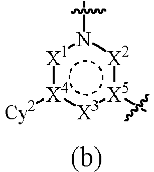 wherein X¹, X⁴ and X⁵ are each independently selected from C, CH and CH₂, X² is N, X³ is C=O, and Cy² is as defined above. The compound of formula (I-d) can be prepared from the following reaction formulas: 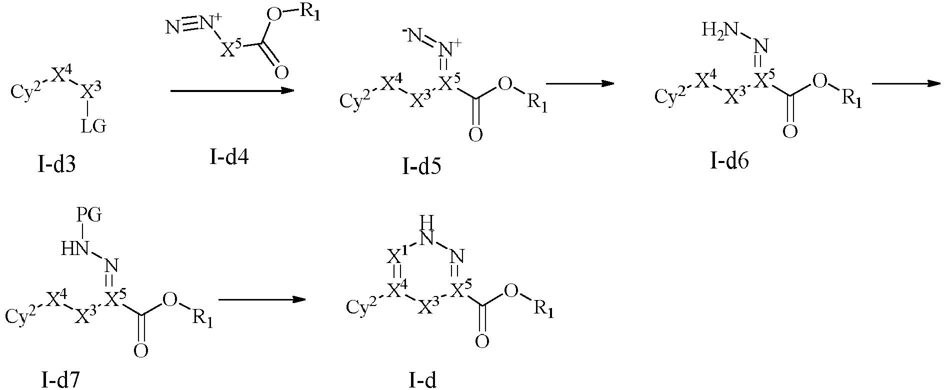 wherein LG is a leaving group (e.g., chloro) known in the art, PG is a protective group (preferably, *tert*-butoxycarbonyl) known in the art, and R¹ is as defined above. The compound of formula (I-d) can be prepared from a compound of formula (I-d3) by a method conventional to those skilled in the art, and a method for preparing the compound of formula (I-d) from the compound of formula (I-d3) is schematically provided as follows:

For example, the compound of formula (I-d3) is dissolved in an appropriate solvent (e.g., ethyl acetate), and the mixture is cooled to an appropriate temperature (e.g., 0 °C), added with a compound of formula (I-d4), and heated to room temperature and reacted for an appropriate time (e.g., 15 hours). After the reaction is completed, the reaction is quenched with an appropriate base (such as potassium carbonate), and a compound of formula (I-d5) is obtained by conventional post-treatment methods (such as filtration, extraction, and concentration) and purification methods (such as recrystallization, and silica gel column chromatography).

The compound of formula (I-d5) is dissolved in an appropriate solvent (e.g., methyl *tert*-butyl ether), and the mixture is cooled to an appropriate temperature (e.g., 0 °C), added with an appropriate reducing agent (e.g., triphenylphosphine, or tri-*n*-butylphosphine, and preferably, tri-*n*-butylphosphine), and heated to room temperature and reacted for an appropriate time (e.g. 15 hours). After the reaction is completed, a compound of formula (I-d6) is obtained by conventional post-treatment methods (such as filtration, extraction, and concentration) and purification methods (such as recrystallization, and silica gel column chromatography).

The compound of formula (I-d6) is dissolved in an appropriate solvent (e.g., dichloromethane, or tetrahydrofuran), and the mixture is cooled to an appropriate temperature (e.g., 0 °C), added with an appropriate base (e.g., triethylamine, *N*,*N*-diisopropylethylamine, potassium carbonate, or sodium carbonate, and preferably, triethylamine) and an appropriate protective group reagent (e.g., di-tert-butyl dicarbonate), and heated to room temperature and reacted for an appropriate time (e.g., 15 hours). After the reaction is completed, a compound of formula (I-d7) is obtained by conventional post-treatment methods (such as filtration, extraction, and concentration) and purification methods (such as recrystallization, and silica gel column chromatography).

The compound of formula (I-d7) is added to an appropriate solvent (e.g., toluene, dimethylbenzene, trimethylbenzene, *N*,*N*-dimethylformamide, or *N*,*N*-dimethylacetamide, and preferably, toluene), a suitable reagent (e.g., *N*,*N*-dimethylformamide dimethyl acetal, trimethyl orthoformate, or triethyl orthoformate, and preferably, *N*,*N*-dimethylformamide dimethyl acetal) is added, and the mixture is heated to an appropriate temperature (e.g., 60 °C) and stirred for an appropriate time (e.g., 15 hours). After the reaction is completed, the compound of formula (I-d) is obtained by conventional post-treatment methods (such as filtration, extraction, and concentration) and purification methods (such as recrystallization, and silica gel column chromatography).

In certain embodiments of the present invention, the compound of general formula (I) can be prepared by the following method. 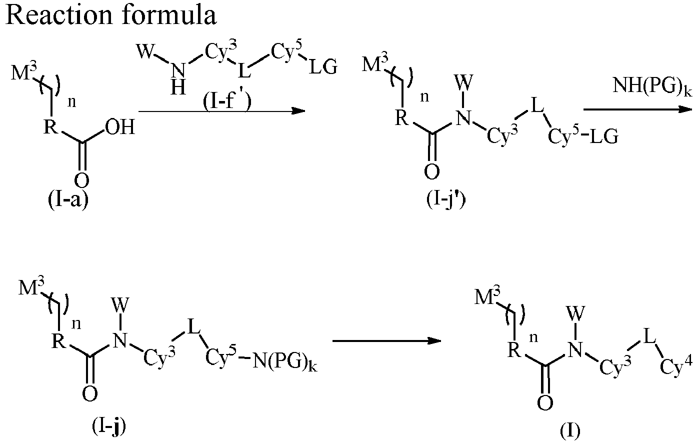
wherein, formula (I-a) is added into a solvent, a peptide coupling reagent, a base and formula (I-f) are added, the mixture is stirred until the reaction is completed, and then formula (I-j') is obtained by separation; or
formula (I-a) and formula (I-f) are added into a base, a coupling reagent is added dropwise, the mixture is stirred until the reaction is completed, and then formula (I-j') is obtained by separation,
formula (I-j') is added into a solvent, a base, a palladium catalyst, a ligand and NPG are added, the mixture is stirred until the reaction is completed, and then formula (I-j) is obtained by separation;
formula (I-j) is added into a solvent, an acid is added, the mixture is stirred until the reaction is completed, and then formula (I) is obtained by separation;
wherein M³, R, n, Cy³, Cy⁴, L, LG, PG, k, and Cy⁵ are as defined above.

The solvent is selected from: one of *N*,*N*-dimethylformamide, *N*,*N*-dimethylacetamide, dimethylsulfoxide, toluene, benzene, dimethylbenzene, trimethylbenzene, cyclohexane, hexane, dichloromethane, chloroform, 1,2-dichloroethane, tetrahydrofuran, diethyl ether, dioxane, 1,2-dimethoxyethane, methyl acetate, ethyl acetate, acetone, methyl ethyl ketone, acetonitrile, methanol, ethanol, isopropanol, *tert*-butanol and water, and a mixture thereof;
the catalyst is selected from: palladium chloride, palladium acetate, tris(dibenzylideneacetone)dipalladium, and a mixture thereof;
the ligand is selected from: 1,1'-binaphthyl-2,2'-bisdiphenylphosphine, triphenylphosphine, and a mixture thereof;
the base is selected from: one of methylamine, ethylamine, propylamine, *N*,*N*-diisopropylethylamine, trimethylamine, triethylamine, dicyclohexylamine, ethanolamine, diethanolamine, triethanolamine, meglumine, diethanolamine, ethylenediamine, pyridine, picoline and quinoline, and a mixture thereof;
the acid is selected from: formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, *p*-toluenesulfonic acid, methanesulfonic acid, and ethanesulfonic acid; hydrochloric acid, sulfuric acid, nitric acid, carbonic acid, hydrobromic acid, phosphoric acid, and hydroiodic acid;
the peptide coupling reagent is selected from: one of 2-(7-azobenzotriazol)-tetramethyluronium hexafluorophosphate (HATU), benzotriazol-*N*,*N*,*N'*,*N'*-tetramethyluronium hexafluorophosphate (HBTU), and 2-(1*H*-benzo[d][1,2,3]trisazo-1-yl)-1,1,3,3-tetramethyluronium tetrafluoroborate (TBTU), and a mixture thereof;
the coupling reagent is selected from: one of phosphorus oxychloride, dicyclohexylcarbodiimide (DCC), *N*,*N'*-carbonyldiimidazole, isobutyl chloroformate, 1-*n*-propylphosphoric anhydride and the like, and a mixture thereof.

In certain embodiments of the present invention, the compound of general formula (I) can be prepared by the following method.

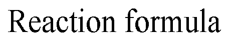

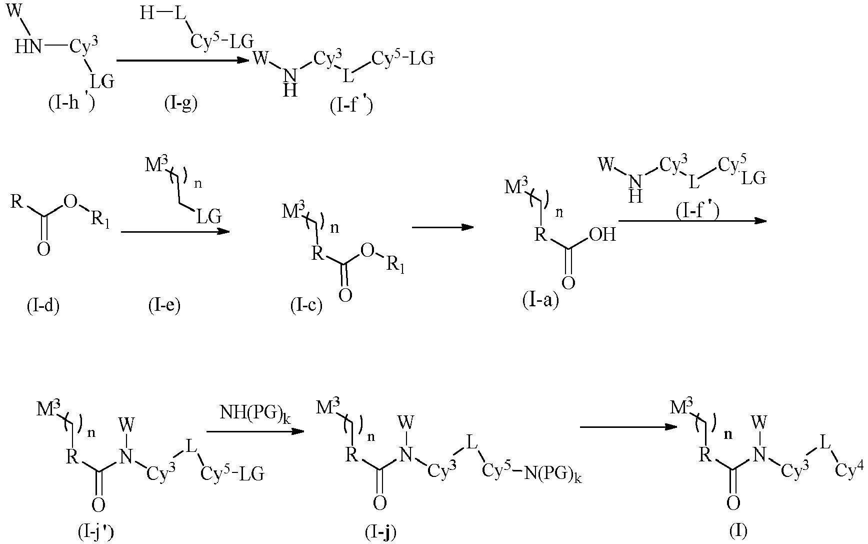

As shown above, the compound of formula (I) can be prepared by subjecting the compound of formula (I-j) to deprotection reaction,
wherein M³, R, n, W, Cy³, L, PG, k, R₁ and Cy⁵ are as defined above.

The compound of formula (I) can be prepared from the compound of formula (I-j) by a method conventional to those skilled in the art, and a method for preparing the compound of formula (I) from the compound of formula (I-j) is schematically provided as follows:
For example, the compound of formula (I-j) is added to a suitable solvent (e.g., methanol, tetrahydrofuran, *N*,*N*-dimethylformamide, or *N*,*N*-dimethylacetamide, and preferably, *N*,*N*-dimethylformamide), water is added, a suitable acid (formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, *p*-toluenesulfonic acid, methanesulfonic acid, or ethanesulfonic acid; hydrochloric acid, sulfuric acid, nitric acid, carbonic acid, hydrobromic acid, phosphoric acid, or hydroiodic acid) is added, the mixture is stirred at room temperature for an appropriate time (e.g., 10 to 30 minutes), after the reaction is completed as monitored, an appropriate amount of water is added, pH is adjusted, the reaction solution is extracted with a suitable extractant (e.g., ethyl acetate), the extract is concentrated, and then the crude product is separated by a conventional purification method (e.g., silica gel column chromatography) to give the compound of formula (I-j).

As shown above, the compound of formula (I-j) can be prepared by the reaction of a compound of formula (I-j'), 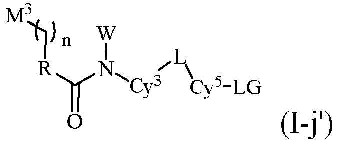 wherein M³, R, n, Cy³, L and Cy⁵ are as defined above.

The compound of formula (I-j) can be prepared from the compound of formula (I-j') by a method conventional to those skilled in the art, and a method for preparing the compound of formula (I-j) from the compound of formula (I-j') is schematically provided as follows:
For example, the compound of formula (I-j') is added to an appropriate solvent (e.g., 1,4-dioxane, tetrahydrofuran, acetonitrile, *N*,*N*-dimethylformamide, or *N*,*N*-dimethylacetamide, and preferably, *N*,*N*-dimethylformamide), an appropriate catalyst (such as palladium acetate or palladium chloride) is added, an appropriate ligand (such as, BINAP, XPhos, SPhos or Ruphos) is added, an appropriate base (e.g., potassium carbonate, sodium carbonate, triethylamine, or *N*,*N*-diisopropylethylamine, and preferably, potassium carbonate) is added, NPG (e.g., benzophenone imine, *p*-methoxybenzylamine, or tert-butylamine) is added, and the mixture is stirred at an appropriate temperature (e.g., room temperature to 110 °C) for an appropriate time (e.g., 14 hours). After the reaction is completed, the crude product is obtained by conventional post-treatment methods (such as filtration and extraction), and then separated by a conventional purification method (such as silica gel column chromatography) to give the compound of formula (I-j).

As shown above, the compound of formula (I-j') can be prepared by the reaction of the compound of formula (I-a) and a compound of formula (I-f), 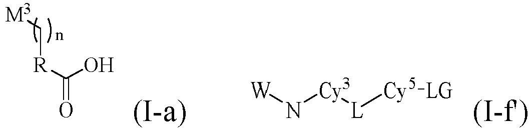 wherein M³, R, n, Cy³, L and Cy⁵ are as defined above.

The compound of formula (I-j') can be prepared from the compound of formula (I-a) and the compound of formula (I-f) by a method conventional to those skilled in the art, and a method for preparing the compound of formula (I-j') by the reaction of the compound of formula (I-a) and the compound of formula (I-f) is schematically provided as follows:
For example, the compound of formula (I-a) is added to a suitable solvent (e.g., tetrahydrofuran, *N*,*N*-dimethylformamide, or *N*,*N*-dimethylacetamide, and preferably, *N*,*N*-dimethylformamide), a suitable peptide coupling reagent (preferably, 2-(7-benzotriazole oxide)-*N*,*N*,*N'*,*N*'-tetramethyluronium hexafluorophosphate) is added, then a suitable base (e.g., triethylamine, pyridine, or *N*,*N*-diisopropylethylamine, and preferably, *N*,*N*-diisopropylethylamine) is added, and the mixture is cooled to an appropriate temperature (e.g., 0 °C) and stirred for an appropriate time (e.g., 10 to 30 minutes), added with the compound of formula (I-f), and stirred at an appropriate temperature (e.g., from 0 °C to ambient temperature) for a suitable time (e.g., 1 to 16 hours); the reaction solution is concentrated under reduced pressure, added with an appropriate amount of water and extracted with a suitable extractant (such as ethyl acetate), the extract is concentrated, and the crude product is separated by a conventional purification method (such as silica gel column chromatography) to give the compound of formula (I-j').

For another example, the compound of formula (I-a) and the compound of formula (I-f) are added to a suitable base (e.g., pyridine), the mixture is cooled to an appropriate temperature (e.g., 0 °C), added dropwise with a coupling reagent (e.g., phosphorus oxychloride), and stirred for a suitable time (e.g., 0.5 to 3 hours) until the reaction is completed, then the reaction solution is concentrated under reduced pressure, added with an appropriate amount of water, and extracted with a suitable extractant (e.g., ethyl acetate), the extract is concentrated, and the crude product is separated by a conventional purification method (e.g., silica gel column chromatography) to give the compound of formula (I-j').

As shown above, the compound of formula (I-a) can be prepared from the compound of formula (I-c), 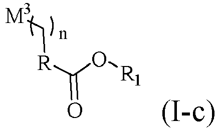 wherein R₁ is C₁₋₆ alkyl, and M³, R and n are as defined above. The compound of formula (I-a) can be prepared from the compound of formula (I-c) by a method conventional to those skilled in the art, and a method for preparing the compound of formula (I-a) from the compound of formula (I-c) is schematically provided as follows:
For example, the compound of formula (I-c) is dissolved in a suitable solvent (e.g., methanol, ethanol, tetrahydrofuran, or dioxane, and preferably, methanol), a suitable base (e.g., lithium hydroxide, sodium hydroxide, or potassium hydroxide, and preferably, lithium hydroxide) is added, the mixture is stirred at an appropriate temperature (e.g., room temperature) for an appropriate time (e.g., 0.5 to 3 hours), after the reaction is completed, the reaction solution is concentrated under reduced pressure, pH is adjusted to a suitable range (e.g., 2 to 5) with an appropriate aqueous solution of an acid (e.g., hydrochloric acid or citric acid), and then the compound of formula (I-a) is obtained by filtration.

As shown above, the compound of formula (I-c) can be prepared by the reaction of the compound of formula (I-d) and the compound of formula (I-e), 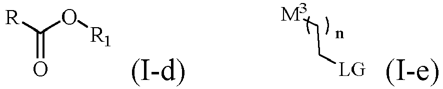 wherein R, R₁, M³ and n are as defined above, LG is a leaving group (e.g., chloro, bromo, iodo, mesylate or besylate etc) or boric acid or boric acid ester, and M³ and n are as defined above. The compound of formula (I-c) can be prepared from the compound of formula (I-d) and the compound of formula (I-e) by a method conventional to those skilled in the art, and a method for preparing the compound of formula (I-c) by the reaction of the compound of formula (I-d) and the compound of formula (I-e) is schematically provided as follows:
For example, the compound of formula (I-d) and the compound of formula (I-e) (LG is a leaving group) are dissolved in a suitable solvent (e.g., acetonitrile, tetrahydrofuran, or *N*,*N*-dimethylformamide, and preferably, *N*,*N*-dimethylformamide), an appropriate base (e.g., sodium carbonate, potassium carbonate, or cesium carbonate, and preferably, potassium carbonate) is added, the mixture is stirred at an appropriate temperature (e.g., 50 °C) for an appropriate time (e.g., 10 to 16 hours), after the reaction is completed, water and a suitable extractant are added, and the crude product is separated by a conventional purification method (e.g., silica gel column chromatography) to give the compound of formula (I-c).

For another example, the compound of formula (I-d) and the compound of formula (I-e) (LG is boric acid or boric acid ester) are dissolved in a suitable solvent (e.g., acetonitrile, dichloromethane, chloroform, pyridine, or *N*,*N*-dimethylformamide, and preferably, *N*,*N*-dimethylformamide), an appropriate base (e.g., triethylamine, or pyridine) and an appropriate catalyst (e.g., copper acetate, or copper chloride) are added, and the mixture is reacted in air or under oxygen atmosphere at an appropriate temperature (e.g., room temperature to 50 °C) for an appropriate time (e.g., 14 hours). After the reaction is completed, the crude product is obtained through conventional post-treatment (such as extraction and concentration), and then the product is obtained through conventional purification means (such as silica gel column chromatography).

As shown above, the compound of formula (I-f) can be prepared by the reaction of a compound of formula (I-h') and the compound of formula (I-g), 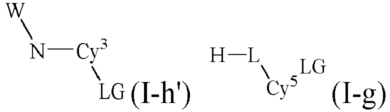 wherein Cy³, L, LG, W and Cy⁵ are as defined above. The compound of formula (I-f) can be prepared from the compound of formula (I-h') and the compound of formula (I-g) by a method conventional to those skilled in the art, and a method for preparing the compound of formula (I-f) from the compound of formula (I-h') and the compound of formula (I-g) is schematically provided as follows:
For example, the compound of formula (I-g) and the compound of formula (I-h') are added to an appropriate solvent (e.g., tetrahydrofuran, acetonitrile, *N*,*N*-dimethylformamide, or *N*,*N*-dimethylacetamide, and preferably *N*,*N*-dimethylformamide), an appropriate base (e.g., potassium carbonate, sodium carbonate, triethylamine, or *N*,*N*-diisopropylethylamine, and preferably potassium carbonate) is added, and the mixture is stirred at an appropriate temperature (e.g., room temperature to 80 °C) for an appropriate time (e.g., 14 hours). After the reaction is completed, the crude product is obtained by conventional post-treatment methods (such as filtration and extraction), and then separated by a conventional purification method (such as silica gel column chromatography) to give the product.

In the synthesis of the compounds of the present invention, the reaction solvents may be any solvent commonly used in the art, including, but not limited to, ethers, alkanes, halogenated alkanes, aromatic hydrocarbons, alcohols, etc. Specifically, the reaction solvents may be *N*,*N*-dimethylformamide, *N*,*N*-dimethylacetamide, dimethylsulfoxide, aromatic hydrocarbons (such as toluene, benzene, dimethylbenzene, and trimethylbenzene), saturated hydrocarbons (such as cyclohexane and hexane), halogenated hydrocarbons (such as dichloromethane, chloroform, and 1,2-dichloroethane), ethers (such as tetrahydrofuran, diethyl ether, dioxane, and 1,2-dimethoxyethane), esters (such as methyl acetate, and ethyl acetate), ketones (such as acetone, and methyl ethyl ketone), nitriles (such as acetonitrile), alcohols (such as methanol, ethanol, isopropanol, and *tert*-butanol), water, mixed solvents of the above, etc.

In the synthesis of the compounds of the present invention, the bases used herein may be bases commonly used in the art, including organic and inorganic bases. The organic bases may include, for example, methylamine, ethylamine, propylamine, *N*,*N*-diisopropylethylamine, trimethylamine, triethylamine, dicyclohexylamine, ethanolamine, diethanolamine, triethanolamine, meglumine, diethanolamine, ethylenediamine, pyridine, methylpyridine, quinoline, etc.; and the inorganic bases may include, for example, hydroxides, carbonates and bicarbonates of base metals (such as lithium, sodium, potassium and cesium); hydroxides, carbonates and bicarbonates of basene earth metals (magnesium, calcium, strontium and barium); sodium *tert*-butoxide, potassium *tert*-butoxide, sodium ethoxide, etc.

In the synthesis of the compounds of the present invention, the acids used herein may be acids commonly used in the art, including organic and inorganic acids. The organic acids may include, for example, formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, *p*-toluenesulfonic acid, methanesulfonic acid and ethylsulfonic acid; and the inorganic acids may include, for example, hydrochloric acid, sulfuric acid, nitric acid, carbonic acid, hydrobromic acid, phosphoric acid, hydroiodic acid, etc.

In the synthesis of the compounds of the present invention, the reducing agent used herein may be any reducing agent commonly used in the art, including, but not limited to, triphenylphosphine, tri-*n*-butylphosphine, sodium triacetoxyborohydride, sodium cyanoborohydride, sodium borohydride, iron powder, zinc powder, stannous chloride, sodium dithionite, hydrogen, etc.

In the synthesis of the compounds of the present invention, the oxidizing agent used herein may be any oxidizing agent commonly used in the art, including, but not limited to, copper chloride, manganese dioxide, permanganate, dichromate, peracetic acid, perbenzoic acid, etc.

In the synthesis of the compounds of the present invention, the catalyst used herein may be any catalyst commonly used in the art, including, but not limited to, copper acetate, copper chloride, palladium carbon, ferric chloride, palladium acetate, [1,1'-bis(diphenylphosphino)ferrocene]dichloropalladium, etc. Examples

The present invention can be better understood from the following examples and experimental examples. In addition, it is easily understood by those skilled in the art that the content described in the examples are only used to illustrate the present invention, and should not and will not limit the present invention described in detail in the claims.

If specific reaction conditions are not specified in the examples, conventional conditions or conditions recommended by the manufacturers shall be adopted. The adopted reagents or instruments, without manufacturers, are all commercially-available conventional products.

In the present invention, unless otherwise stated, (i) the temperature is expressed in Celsius (°C), and unless otherwise stated, the operation is performed at room temperature; (ii) the progress of the reaction is tracked by thin-layer chromatography (TLC) or LC-MS; and (iii) the final product has clear proton nuclear magnetic resonance spectroscopy (¹H-NMR) data and mass spectrometry (MS) data.

The abbreviations and English expressions used in the present invention have the following meanings:
DCM: dichloromethane
DMF: *N*,*N*-dimethylformamide
EA: ethyl acetate
MeOH: methanol
PE: petroleum ether
BINAP: (±)-2,2'-bis-(diphenylphosphino)-1,1'-binaphthyl
(NH₄)₂S: ammonium sulfide
Boc-: *tert*-butoxycarbonyl
DMSO: dimethyl sulfoxide
Pd₂(dba)₃: tris(dibenzylideneacetone)dipalladium(0)
Pd(OAc)₂: palladium acetate
EtOH: ethanol
-PMB: (4-methoxybenzyl)-
HATU: 2-(7-azabenzotriazol-1-yl)-*N*,*N*,*N'*,*N'*-tetramethyluronium hexafluorophosphate
XPhos: 2-dicyclohexylphosphino-2',4',6'-triisopropylbiphenyl
SPhos: 2-dicyclohexylphosphine-2',6'-dimethoxybiphenyl
Ruphos: 2-dicyclohexylphosphonium-2',6'-diisopropoxy-1,1'-biphenyl

### Example 1: Synthesis of 5-(4-fluorophenyl)-1-isopropyl-N-(5-((1-methyl-6-(1H-pyrazol-4-yl)-1H-indaz ol-5-yl)oxy)pyridin-2-yl)-4-oxo-1,4-dihydropyridazine-3-carboxamide

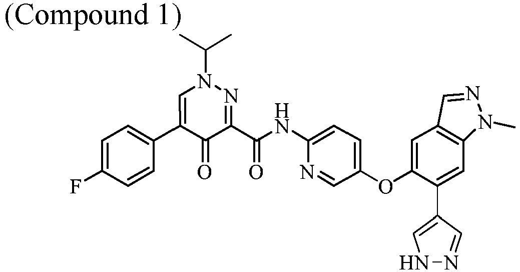

### Step 1: Synthesis of 2,4-dibromo-5-hydroxylbenzaldehyde

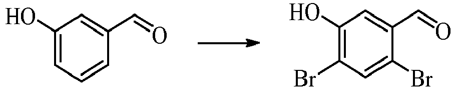

*m*-hydroxylbenzaldehyde (10.0 g, 0.0819 mol, 1.0 eq) was added to DCM (100 mL), bromine (27.5 g, 0.172 mol, 2.1 eq) was added dropwise and slowly, and the mixture was stirred at room temperature for 4 h; after the reaction was completed as monitored by TLC, an aqueous sodium thiosulfate solution (mass fraction 10%) was added dropwise until the red color of the reaction solution faded, a gray solid was precipitated and filtered under reduced pressure, and the filter cake was the product (8.0 g, yield: 35.1%).

### Step 2: Synthesis of 2,4-dibromo-5-((6-nitro-pyridin-3-yl)oxy)benzaldehyde

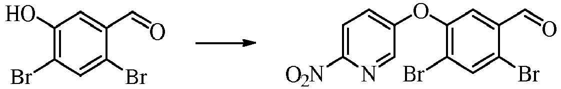

To a flask were added 2,4-dibromo-5-hydroxylbenzaldehyde (5.6 g, 20 mmol, 1.0 eq), 5-fluoro-2-nitropyridine (2.84 g, 20 mmol, 1.0 eq) and potassium carbonate (4.14 g, 30 mmol), and the mixture was then added with acetonitrile (15 mL) and *N*,*N*-dimethylacetamide (15 mL), heated to 60 °C and reacted for 16 h; after the reaction was completed as monitored by TLC, the reaction solution was concentrated under reduced pressure, and added with ethyl acetate (100 mL), the organic phase was washed with water (20 mL × 4), dried over anhydrous sodium sulfate and filtered, the filtrate was concentrated under reduced pressure, and the crude product was purified by silica gel column chromatography (PE:EA = 30:1-10:1) to give the product (3.5 g, yield: 43.7%).

### Step 3: Synthesis of 5-(2,4-dibromo-5-((2-methylhydrazono)methyl)phenoxy)-2-nitropyridine

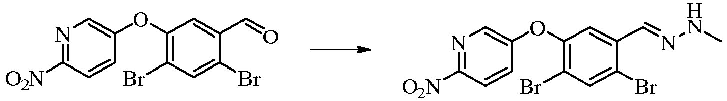

To a flask were added 2,4-dibromo-5-((6-nitro-pyridin-3-yl)oxy)benzaldehyde (3.12 g, 7.8 mmol, 1.0 eq) and methylhydrazine sulfate (2.25 g, 15.6 mmol), and the mixture was added with tetrahydrofuran (30 mL) and triethylamine (3.16 g, 31.2 mmol), heated to 80 °C and reacted for 16 h; after the reaction was completed as monitored by TLC, ethyl acetate (100 mL) was added, the organic phase was washed with water (20 mL × 4), dried over anhydrous sodium sulfate and filtered, and the filtrate was concentrated under reduced pressure to give the product (2 g, yield: 59.6%).

### Step 4: Synthesis of 6-bromo-1-methyl-5-((6-nitro-pyridin-3-yl)oxy)-1H-indazole

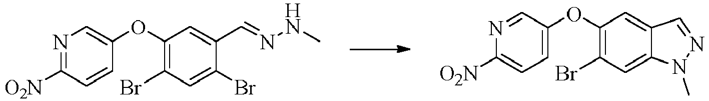

5-(2,4-dibromo-5-((2-methylhydrazono)methyl)phenoxy)-2-nitropyridine (2.0 g, 4.67 mmol, 1.0 eq), potassium carbonate (968.8 mg, 7.02 mL), cuprous chloride (46.28 mg, 0.468 mmol) and DMF (20 mL) were mixed and heated to 110 °C and reacted for 16 h; after the reaction was completed as monitored by TLC, the reaction solution was concentrated under reduced pressure and added with ethyl acetate (50 mL), the organic phase was washed with water (10 mL × 4), dried over anhydrous sodium sulfate and filtered, the filtrate was concentrated under reduced pressure, and the crude product was purified by silica gel column chromatography (PE:EA = 20:1-5:1) to give the product (500 mg, yield: 30.6%).

### Step 5: Synthesis of 5-((6-bromo-1-methyl-1H-indazol-5-yl)oxy)pyridin-2-amine

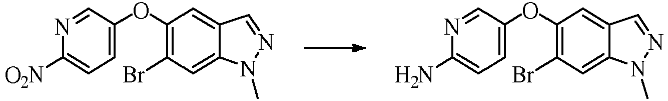

6-bromo-1-methyl-5-((6-nitro-pyridin-3-yl)oxy)-1*H*-indazole (500.0 mg, 1.43 mmol, 1.0 eq), iron powder (802 mg, 14.3 mmol) and a saturated ammonium chloride solution (2.5 mL) were added to ethanol (5 mL), and the mixture was heated to 80 °C and reacted for 8 h; after the reaction was completed as monitored by TLC, the reaction solution was concentrated under reduced pressure and added with ethyl acetate (50 mL) and water (10 mL), followed by liquid separation, the organic phase was dried over anhydrous sodium sulfate and filtered, and the filtrate was concentrated under reduced pressure to give the product (348.0 mg, yield: 76.2%).

### Step 6: Synthesis of N-(5-((6-bromo-1-methyl-1H-indazol-5-yl)oxy)pyridin-2-yl)-5-(4-fluoropheny 1)-1-isopropyl-4-oxo-1,4-dihydropyridazine-3-carboxamide

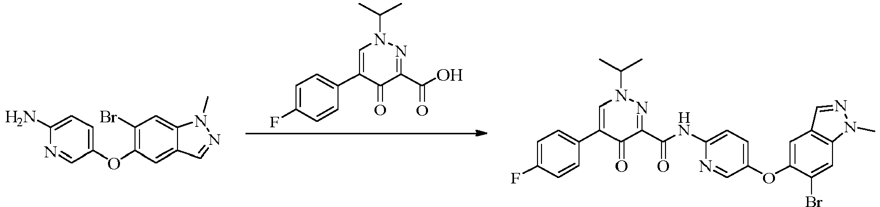

5-((6-bromo-1-methyl-1*H*-indazol-5-yl)oxy)pyridin-2-amine (348.0 mg, 1.086 mmol, 1.0 eq) and 5-(4-fluorophenyl)-1-isopropyl-4-oxo-1,4-dihydropyridazine-3-carboxylic acid (300 mg, 1.086 mmol) were added to pyridine (5 mL), and the mixture was added dropwise with phosphorus oxychloride (188.0 mg, 1.41 mmol) was added, and reacted at room temperature for 30 min; after the reaction was completed as monitored by TLC, water (30 mL) was added, and a solid was precipitated and filtered under reduced pressure to give the product as a filter cake (400.0 mg, yield: 63.9%).

### Step 7: Synthesis of tert-butyl 4-(5-((6-(5-(4-fluorophenyl)-1-isopropyl-4-oxo-1,4-dihydropyridazin-3-carbox amido)pyridin-3-yl)oxy)-1-methyl-1H-indazol-6-yl)-1H-pyrazole-1-carboxylat e

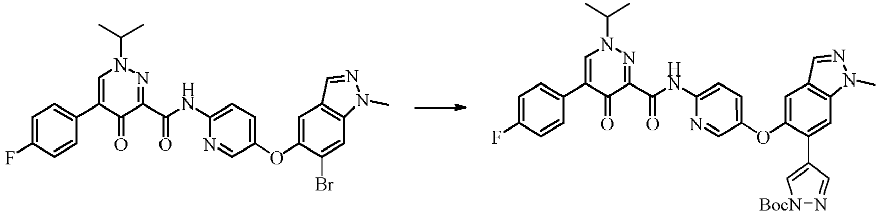

*N*-(5-((6-bromo-1-methyl-1*H*-indazol-5-yl)oxy)pyridin-2-yl)-5-(4-fluorop henyl)-1-isopropyl-4-oxo-1,4-dihydropyridazine-3-carboxamide (350.0 mg, 0.608 mmol), *tert-butyl* 4-(4,4,5,5-tetramethyl-1,3,2-dioxaborolan-2-yl)-1*H*-pyrazole-1-carboxylate (286.2 mg, 0.973 mmol), [1,1'-bis(di-*tert*-butylphosphino)ferrocene]palladium (II) dichloride (19.8 mg, 0.0304 mmol), potassium phosphate (516.2 mg, 2.432 mmol) and di-tert-butyl dicarbonate (106.3 mg, 0.486 mmol) were added to water (0.5 mL) and 1,4-dioxane (5 mL), and the mixture was reacted at 100 °C for 11 h; after the reaction was completed as monitored by TLC, water (20 mL) was added, a solid was precipitated and filtered under reduced pressure, the filter cake was dissolved in dichloromethane, dried over anhydrous sodium sulfate and filtered, the filtrate was concentrated under reduced pressure, and the crude product was purified by silica gel column chromatography (DCM:MeOH = 60:1-10:1) to give the product (350 mg, yield: 86.7%).

### Step 8: Synthesis of 5-(4-fluorophenyl)-1-isopropyl-N-(5-((1-methyl-6-(1H-pyrazol-4-yl)-1H-indaz ol-5-yl)oxy)pyridin-2-yl)-4-oxo-1,4-dihydropyridazine-3-carboxamide

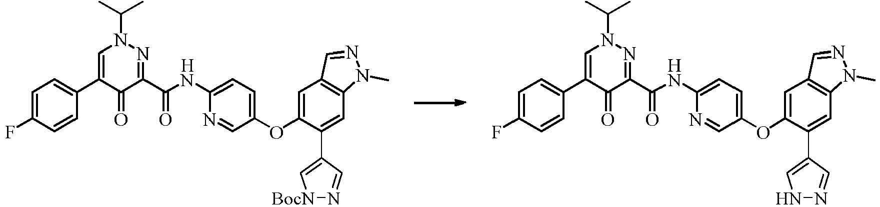

### Tert-butyl

4-(5-((6-(5-(4-fluorophenyl)-1-isopropyl-4-oxo-1,4-dihydropyridazin-3-carbox amido)pyridin-3-yl)oxy )-1-methyl-1*H*-indazol-6-yl)-1*H*-pyrazole-1-carboxylat e (350.0 mg, 0.527 mmol) was added to DCM (5 mL), and the mixture was added with trifluoroacetic acid (2.5 mL) and stirred at room temperature for 4 h; after the reaction was completed as monitored by TLC, DCM (20 mL) and water (10 mL) were added, the reaction system was added with sodium bicarbonate to adjust the pH value to 7-8, followed by liquid separation, the organic phase was dried over anhydrous sodium sulfate and filtered, the filtrate was concentrated under reduced pressure, and the crude product was purified by silica gel column chromatography (DCM:MeOH = 40:1-10:1) to give the product (218.0 mg, yield: 73.3%).

¹H-NMR (400 MHz, DMSO-*d₆*) δ(ppm): 13.03 (s, 1H), 8.39-8.36 (d, 1H), 8.15-8.14 (d, 1H), 8.10 (s, 1H), 8.04 (s, 1H), 7.91(s, 1H), 7.80-7.75 (m, 2H), 7.62 (s, 1H), 7.35 (s, 1H), 7.26 (s, 1H), 7.25 (s, 1H), 7.22-7.16 (t, 2H), 4.74-4.69 (t, 1H), 4.15 (s, 3H), 1.67-1.65 (d, 6H).

Molecular formula: C₃₀H₂₅FN₈O₃; molecular weight: 564.58; LC-MS (Pos, *m*/*z*)=565.23[M+H]⁺.

### Example 2: Synthesis of N-(5-((2-amino-3-chloropyridin-4-yl)oxy)pyridin-2-yl)-5-(4-fluorophenyl)-1-i sopropyl-4-oxo-1,4-dihydropyridazine-3-carboxamide (Compound 11)

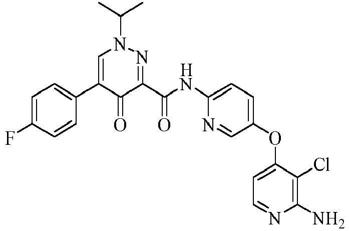

### Step 1: Synthesis of 2,3-dichloropyridin-4-ol

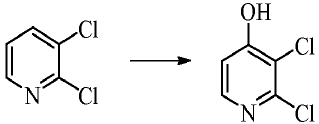

Diisopropylamine (16.1 g, 0.16 mol) was added to tetrahydrofuran (80 mL), the mixture was cooled to -10 °C in an ice salt bath, added dropwise and slowly with *n*-butyllithium (62.6 mL, 0.157 mmol), and reacted for 0.5 h, and then the reaction solution was added dropwise and slowly to a solution of 2,3-dichloropyridine (15.0 g, 0.101mol, 1.0 eq) and triisopropylborate (38.18 g, 0.203 mol) in tetrahydrofuran (150 mL) and reacted at 25 °C for 3 h; after the reaction was completed as monitored by TLC, the reaction solution was added with water (150 mL), added with sodium percarbonate (35.7 g) in two portions, stirred at 25 °C for 1 h, added with an aqueous citric acid solution to adjust the pH value to 2-3, then added with sodium bisulfite (12.75 g), and extracted with toluene (100 mL), the organic phases were combined, washed with water (10 mL × 2), and concentrated under reduced pressure, and a solid was precipitated and filtered under reduced pressure to give the product as a filter cake (10.0 g, yield: 60.5%).

### Step 2: Synthesis of 2,3-dichloro-4-((6-nitro-pyridin-3-yl)oxy)pyridine

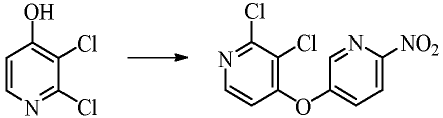

2,3-dichloropyridin-4-ol (4.68 g, 0.0285 mol, 1.0 eq), 5-fluoro-2-nitropyridine (4.06 g, 0.0285 mol, 1.0 eq) and lithium carbonate (3.17 g, 0.0428 mol) were added to DMSO (40 mL), and the mixture was heated to 120 °C and reacted for 16 h; after the reaction was completed as monitored by TLC, ethyl acetate (200 mL) was added, the organic phase was washed with water (20 mL × 4), dried over anhydrous sodium sulfate, filtered, and concentrated under reduced pressure, and the crude product was slurried with ethyl acetate (20 mL) to give the product (7 g, yield: 86.2%).

### Step 3: Synthesis of N-(3-chloro-4-((6-nitro-pyridin-3-yl)oxy)pyridin-2-yl)-1,1-diphenylmethanimi ne

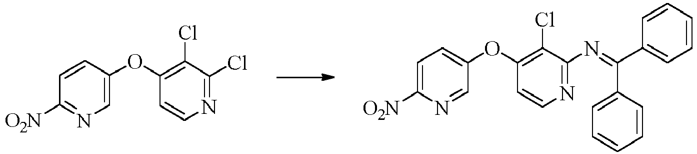

2,3-dichloro-4-((6-nitro-pyridin-3-yl)oxy)pyridine (7.0 g, 0.0246 mol, 1.0 eq), diphenylazomethine (4.46 g, 0.0246 mol), palladium acetate (139.2 mg, 0.62 mmol), BINAP (582.0 mg, 0.93 mmol), and cesium carbonate (16.0 g, 0.0492 mol) were added to 1,4-dioxane (50 mL), and the mixture was heated to 100 °C and reacted for 16 h; after the reaction was completed as monitored by TLC, ethyl acetate (200 mL) was added, the organic phase was washed with water (15 mL × 4), dried over anhydrous sodium sulfate and filtered, the filtrate was concentrated under reduced pressure, and the crude product was purified by silica gel column chromatography (DCM:MeOH = 100:1-10:1) to give the product (9 g, yield: 84.9%).

### Step 4: Synthesis of 5-((3-chloro-2-((diphenylmethylene)amino)pyridin-4-yl)oxy)pyridin-2-amine

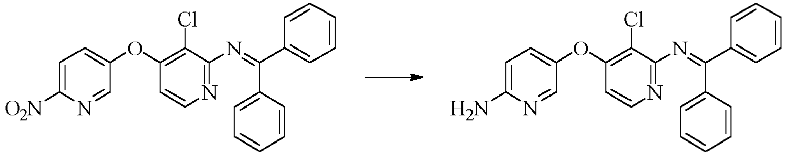

*N*-(3-chloro-4-((6-nitro-pyridin-3-yl)oxy)pyridin-2-yl)-1,1-diphenylmeth animine (2.0 g, 4.64 mmol, 1.0 eq) was added to a 20% aqueous (NH₄)₂S solution (15 mL,10.0 eq), and the mixture was added with isopropanol (100 mL), reacted at room temperature for 1 h, and heated to 70 °C and reacted for 2 h; after the reaction was completed, the reaction solution was cooled to room temperature, added with water (50 mL), and extracted with ethyl acetate (50 mL × 3), the organic phases were combined, dried over anhydrous sodium sulfate and filtered, the filtrate was concentrated under reduced pressure, and the crude product was purified by silica gel column chromatography (DCM:MeOH = 50:1-20:1) to give the product (300.0 mg, yield: 16.1%).

### Step 5: Synthesis of N-(5-((3-chloro-2-((diphenylmethylene)amino)pyridin-4-yl)oxy)pyridin-2-yl)-5-(4-fluorophenyl)-1-isopropyl-4-oxo-1,4-dihydropyridazine-3-carboxamide

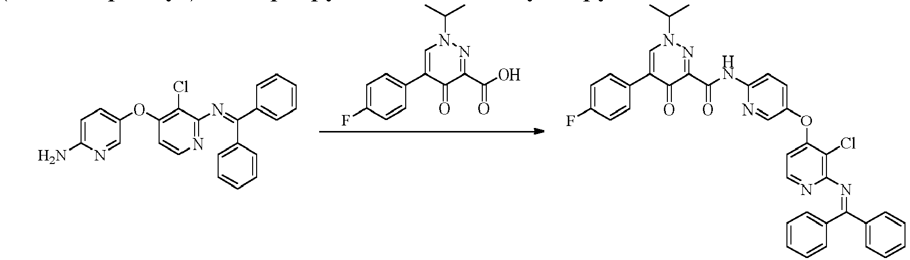

5-((3-chloro-2-((diphenylmethylene)amino)pyridin-4-yl)oxy)pyridin-2-a mine (275.0 mg, 0.686 mmol, 1.0 eq) and 5-(4-fluorophenyl)-1-isopropyl-4-oxo-1,4-dihydropyridazine-3-carboxylic acid (189.5 mg, 0.686 mmol, 1.0 eq) were dissolved in pyridine (5 mL), and the mixture was added dropwise with phosphorus oxychloride (157.8 mg, 1.029 mmol, 1.5 eq) and stirred at room temperature for 30 min; after the reaction was completed as monitored by TLC, the reaction was quenched with water (20 mL), the reaction solution was extracted with ethyl acetate (50 mL), the organic phase was dried over anhydrous sodium sulfate and filtered, and the filtrate was concentrated under reduced pressure to give the product (400 mg, yield: 88.5%).

### Step 6: Synthesis of N-(5-((2-amino-3-chloropyridin-4-yl)oxy)pyridin-2-yl)-5-(4-fluorophenyl)-1-i sopropyl-4-oxo-1,4-dihydropyridazine-3-carboxamide

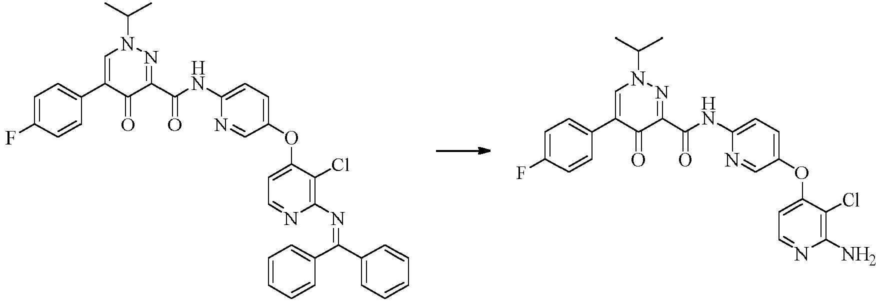

*N*-(5-((3-chloro-2-((diphenylmethylene)amino)pyridin-4-yl)oxy)pyridin-2 -yl)-5-(4-fluorophenyl)-1-isopropyl-4-oxo-1,4-dihydropyridazine-3-carboxami de (400.0 mg, 0.607 mmol, 1.0 eq) was added to isopropanol (4 mL), and the mixture was added with water (0.7 mL) and concentrated hydrochloric acid (0.2 mL), and stirred at room temperature for 30 min; after the reaction was completed as monitored by TLC, the reaction solution was cooled to room temperature, added with a saturated aqueous NaHCO₃ solution to adjust the pH value to 7-8, and extracted with DCM (20 mL × 3), the organic phases were combined, dried over anhydrous sodium sulfate and filtered, the filtrate was concentrated under reduced pressure, and the crude product was purified by silica gel column chromatography (DCM:MeOH = 30:1-10:1) to give the product (201.0 mg, yield: 59.2%).

¹H-NMR (300 MHz, CDCl₃) δ(ppm): 13.19 (s, 1H), 8.52 (d, 1H), 8.24 (s, 1H), 8.23 (s, 1H), 8.49 (d, 1H), 7.83-7.75 (m, 2H), 7.51 (t, 1H),7.18 (t, 2H), 6.12 (d, 1H), 5.09 (s, 2H), 4.79-4.66 (m, 1H), 1.68 (s, 3H), 1.66 (s,3H).

Molecular formula: C₂₄H₂₀ClFN₆O₃; molecular weight: 494.91; LC-MS (Pos, *m*/*z*)=494.05[M+H]⁺.

### Example 3: Synthesis of N-(5-((2-amino-3-chloropyridin-4-yl)oxy)pyrimidin-2-yl)-5-(4-fluorophenyl)-1-isopropyl-4-oxo-1,4-dihydropyridazine-3-carboxamide (Compound 12)

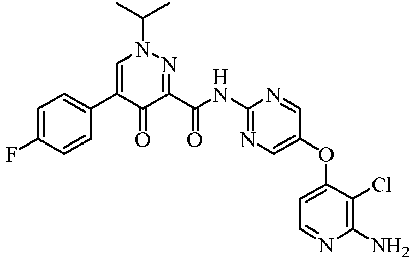

### Step 1: Synthesis of N-(5-((2,3-dichloropyridin-4-yl)oxy)pyrimidin-2-yl)-5-(4-fluorophenyl)-1-isop ropyl-4-oxo-1,4-dihydropyridazine-3-carboxamide

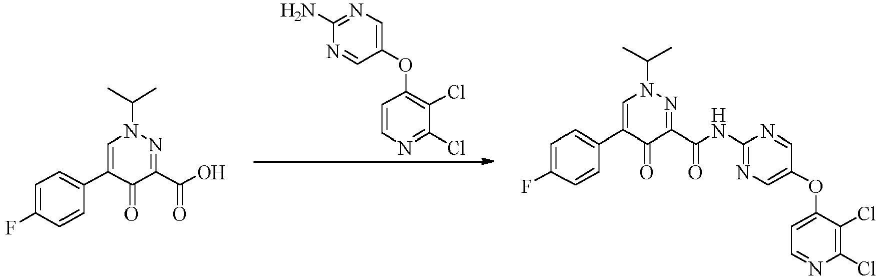

5-(4-fluorophenyl)-1-isopropyl-4-oxo-1,4-dihydropyridazine-3-carboxyli c acid (1.0 g, 3.61 mmol, 1.0 eq) was added to pyridine (10 mL), and the mixture was added with 5-((2,3-dichloropyridin-4-yl)oxy)pyrimidin-2-amine (930.5 mg, 3.61 mmol, 1.0 eq) and phosphorus oxychloride (0.5 mL) and stirred at room temperature for 15 min; after the reaction was completed as monitored by TLC, the reaction solution was added with ethyl acetate (200 mL) and water (150 mL), followed by liquid separation, the aqueous phase was extracted with ethyl acetate (100 mL), the organic phases were combined, dried over anhydrous sodium sulfate and filtered under vacuum, and the filtrate was purified by silica gel column chromatography (100-200 mesh silica gel, dichloromethane:methanol = 100:1-50:1) to give the product (1.56 g, yield: 83.8%).

### Step 2: Synthesis of N-(5-((3-chloro-2-((diphenylmethylene)amino)pyridin-4-yl)oxy)pyrimidin-2-y l)-5-(4-fluorophenyl)-1-isopropyl-4-oxo-1,4-dihydropyridazine-3-carboxamide

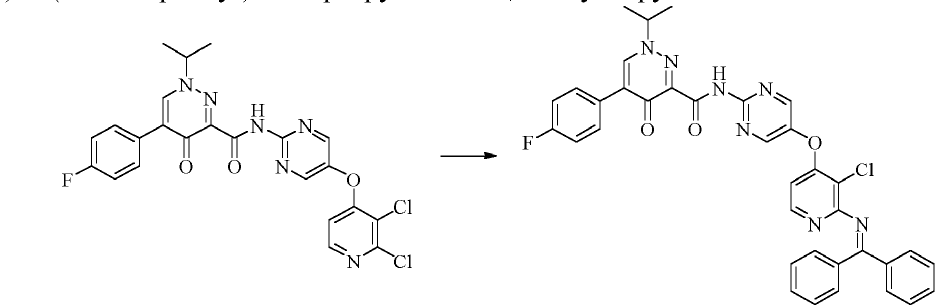

*N*-(5-((2,3-dichloropyridin-4-yl)oxy)pyrimidin-2-yl)-5-(4-fluorophenyl)-1-isopropyl-4-oxo-1,4-dihydropyridazine-3-carboxamide (1.54 g, 2.98 mmol, 1.0 eq) was added to 1,4-dioxane (16 mL), and the mixture was added with benzophenone imine (812.4 mg, 4.48 mmol, 1.5 eq), cesium carbonate (1.94 g, 5.97 mmol, 2.0 eq), Pd₂(dba)₃ (273.6 mg, 0.29 mmol, 0.1 eq) and 1,1'-binaphthyl-2,2'-bisdiphenylphosphine (278.9 mg, 0.44 mmol, 0.15 eq) and refluxed at 110 °C for 12 h; after the reaction was completed as monitored by TLC, the reaction solution was cooled to room temperature and concentrated, and the crude product was purified by silica gel column chromatography (100-200 mesh silica gel, dichloromethane:methanol = 100:1-50:1) to give the product (565.0 mg, yield: 28.6%).

### Step 3: Synthesis of N-(5-((2-amino-3-chloropyridin-4-yl)oxy)pyrimidin-2-yl)-5-(4-fluorophenyl)-1-isopropyl-4-oxo-1,4-dihydropyridazine-3-carboxamide

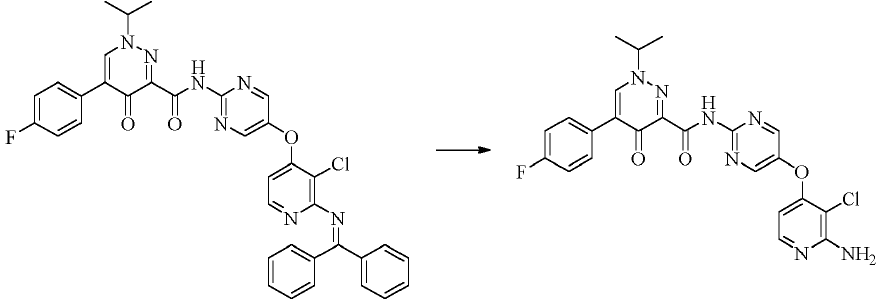

*N*-(5-((3-chloro-2-((diphenylmethylene)amino)pyridin-4-yl)oxy)pyrimidi n-2-yl)-5-(4-fluorophenyl)-1-isopropyl-4-oxo-1,4-dihydropyridazine-3-carbox amide (565.0 mg, 0.85 mmol, 1.0 eq) was dissolved in methanol (10 mL), and the mixture was added with concentrated hydrochloric acid (0.2 mL) and reacted at room temperature for 1 h; after the reaction was completed as monitored by TLC, the reaction solution was added with a saturated aqueous sodium carbonate solution to adjust the pH value to 8 and added with ethyl acetate (100 mL), followed by liquid separation, the aqueous phase was extracted with dichloromethane (100 mL), the organic phases were combined, dried over anhydrous sodium sulfate and filtered, the filtrate was concentrated, and the crude product was purified by silica gel column chromatography (100-200 mesh silica gel, dichloromethane:methanol = 80:1-40:1) to give the product (257.0 mg, yield: 60.6%).

¹HNMR (300 MHz, DMSO-*d₆*) δ(ppm): 12.85 (s, 1H), 8.87 (s, 1H), 8.66 (s, 2H), 8.19 (s, 2H), 7.90-7.94 (m, 2H), 7.77-7.78 (d, J=5.7 Hz, 1H), 7.28-7.34 (t, J=9 Hz, 2H), 6.48 (s, 2H), 6.11-6.13 (d, J=5.7 Hz, 1H), 4.69-4.73 (t, J=13.2 Hz, 1H), 1.47-1.49 (d, J=6.6 Hz, 6H).

Molecular formula: C₂₃H₁₉ ClFN₇O₃; molecular weight: 495.90; LC-MS(Pos, *m*/*z*)=496.02[M+H]⁺.

### Example 4: Synthesis of N-(5-((2-amino-3-chloropyridin-4-yl)oxy)pyridin-2-yl)-1-cyclopropyl-5-(4-flu orophenyl)-4-oxo-1,4-dihydropyridazine-3-carboxamide (Compound 17)

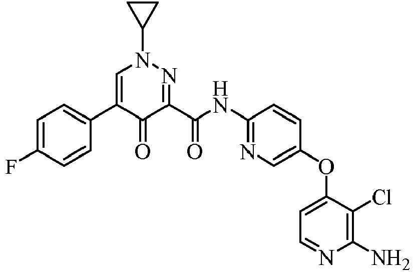

### Step 1: Synthesis of N-(5-((3-chloro-2-((diphenylmethylene)amino)pyridin-4-yl)oxy)pyridin-2-yl)-1-cyclopropyl-5-(4-fluorophenyl)-4-oxo-1,4-dihydropyridazine-3-carboxamid e

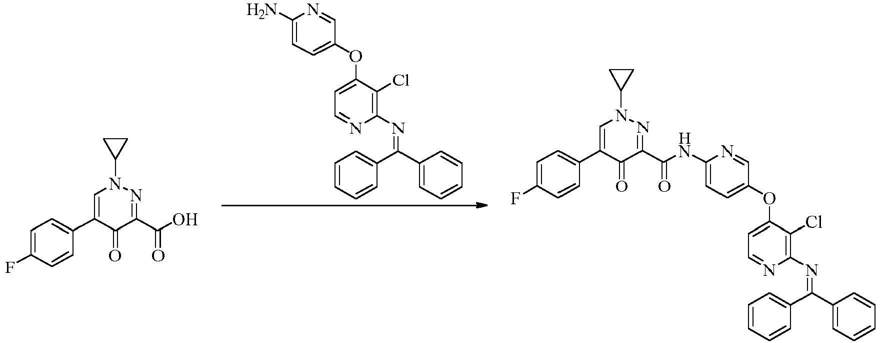

1-cyclopropyl-5-(4-fluorophenyl)-4-oxo-1,4-dihydropyridazine-3-carbox ylic acid (62.9 mg, 0.22 mmol, 1.0 eq) was added to pyridine (3 mL), and the mixture was added with 5-((3-chloro-2-(((diphenylmethylene)amino)pyridin-4-yl)oxy)pyridin-2-amine (92.0 mg, 0.22 mmol, 1.0 eq) and phosphorus oxychloride (0.2 mL) and stirred at room temperature for 0.5 h; after the reaction was completed as monitored by TLC, the reaction solution was added with ethyl acetate (50 mL) and water (100 mL), followed by liquid separation, the organic phase was dried over anhydrous sodium sulfate and filtered under vacuum, and the filtrate was concentrated to give the crude product (201.3 mg) which was used directly in the next step.

### Step 2: Synthesis of N-(5-((2-amino-3-chloropyridin-4-yl)oxy)pyridin-2-yl)-1-cyclopropyl-5-(4-flu orophenyl)-4-oxo-1,4-dihydropyridazine-3-carboxamide

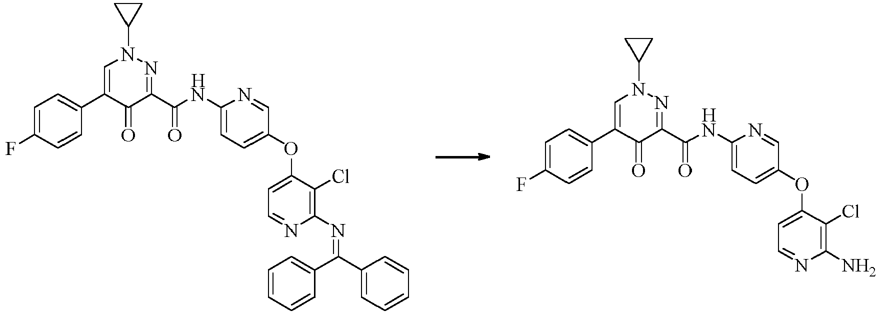

*N*-(5-((3-chloro-2-((diphenylmethylene)amino)pyridin-4-yl)oxy)pyridin-2-yl)-1-cyclopropyl-5-(4-fluorophenyl)-4-oxo-1,4-dihydropyridazine-3-carbox amide (201.3 mg crude, 0.22 mmol, 1.0 eq) was added to methanol (15 mL), and the mixture was added with water (5.0 mL) and concentrated hydrochloric acid (0.5 mL), and stirred at room temperature for 1 h; after the reaction was completed as monitored by TLC, the reaction solution was concentrated under reduced pressure and added with EA (50 mL) and water (50 mL), followed by liquid separation, the organic phase was dried over anhydrous sodium sulfate and filtered, the filtrate was concentrated under reduced pressure, and the crude product was purified by preparative thin-layer chromatography (DCM:MeOH = 20:1) to give the product (50.0 mg, two-step yield: 44.2%).

¹HNMR (400 MHz, DMSO-*d₆*) δ(ppm): 12.94 (s, 1H), 8.93 (s, 1H), 8.33-8.35 (d, *J*=9 Hz, 1H), 8.28-8.29 (d, *J*=2.8 Hz, 1H), 7.92-7.96 (m, 2H), 7.79-7.80 (d, *J*=5.64 Hz, 1H), 7.73-7.76 (dd, J=2.92, 2.96 Hz, 1H), 7.31-7.36 (t, J=8.92 Hz, 2H), 6.44 (s, 2H), 6.05-6.06 (d, *J*=5.64 Hz, 1H), 4.11-4.14 (q, *J*=3.72 Hz, 1H), 1.31-1.33 (t, *J*=3.04 Hz, 2H), 1.11-1.13 (q, *J*=1.84 Hz, 2H).

Molecular formula: C₂₄H₁₈ClFN₆O₃; molecular weight: 492.90; LC-MS(Pos, *m*/*z*)=493.07[M+H]⁺.

### Example 5: Synthesis of N-(5-((2-amino-3-chloropyridin-4-yl)oxy)pyrimidin-2-yl)-5-(4-fluorophenyl)-1-cyclopropyl-4-oxo-1,4-dihydropyridazine-3-carboxamide (Compound 18)

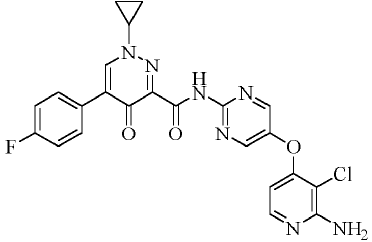

### Step 1: Synthesis of N-(5-((2,3-dichloropyridin-4-yl)oxy)pyrimidin-2-yl)-5-(4-fluorophenyl)-1-cycl opropyl-4-oxo-1,4-dihydropyridazine-3-carboxamide

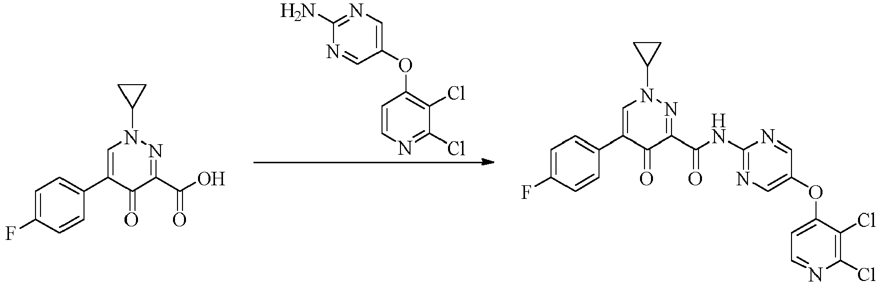

5-(4-fluorophenyl)-1-cyclopropyl-4-oxo-1,4-dihydropyridazine-3-carbox ylic acid (1.5 g, 5.46 mmol, 1.0 eq) was added to pyridine (15 mL), and the mixture was added with 5-((2,3-dichloropyridin-4-yl)oxy)pyrimidin-2-amine (1.4 g, 5.46 mmol, 1.0 eq) and phosphorus oxychloride (0.5 mL) and stirred at room temperature for 15 min; after the reaction was completed as monitored by TLC, the reaction solution was added with ethyl acetate (200 mL) and water (150 mL), followed by liquid separation, the aqueous phase was extracted with ethyl acetate (150 mL), the organic phases were combined, dried over anhydrous sodium sulfate and filtered under vacuum, the filtrate was concentrated, and the crude product was purified by silica gel column chromatography (100-200 mesh silica gel, dichloromethane:methanol = 100:1-50:1) to give the product (2.38 g, yield: 85.0%).

### Step 2: Synthesis of N-(5-((3-chloro-2-((diphenylmethylene)amino)pyridin-4-yl)oxy)pyrimidin-2-y l)-5-(4-fluorophenyl)-1-cyclopropyl-4-oxo-1,4-dihydropyridazine-3-carboxam ide

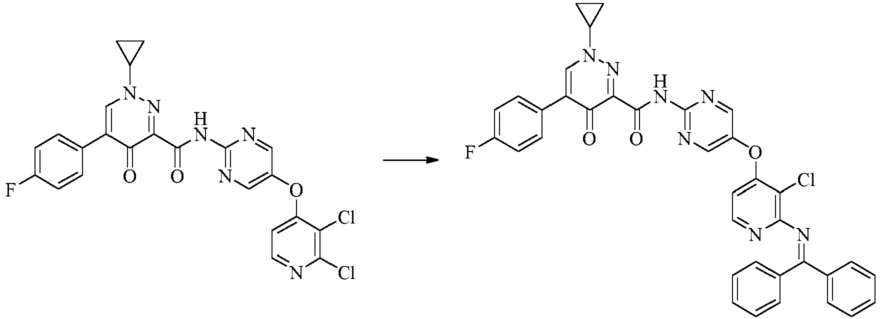

*N*-(5-((2,3-dichloropyridin-4-yl)oxy)pyrimidin-2-yl)-5-(4-fluorophenyl)-1-cyclopropyl-4-oxo-1,4-dihydropyridazine-3-carboxamide (960.0 mg, 1.87 mmol, 1.0 eq) was added to 1,4-dioxane (10 mL), and the mixture was added with benzophenone imine (508.4 mg, 2.80 mmol, 1.5 eq), cesium carbonate (1.22 g, 3.74 mmol, 2.0 eq), Pd₂(dba)₃ (171.2 mg, 0.18 mmol, 0.1 eq) and 1,1'-binaphthyl-2,2'-bisdiphenylphosphine (174.5 mg, 0.28 mmol, 0.15 eq) and stirred at 110 °C under reflux for 12 h; after the reaction was completed as monitored by TLC, the reaction solution was cooled to room temperature and concentrated, and the crude product was purified by silica gel column chromatography (100-200 mesh silica gel, dichloromethane:methanol = 100:1-40:1) to give the product (365.0 mg, yield: 29.7%).

### Step 3: Synthesis of N-(5-((2-amino-3-chloropyridin-4-yl)oxy)pyrimidin-2-yl)-5-(4-fluorophenyl)-1-cyclopropyl-4-oxo-1,4-dihydropyridazine-3 -carboxamide

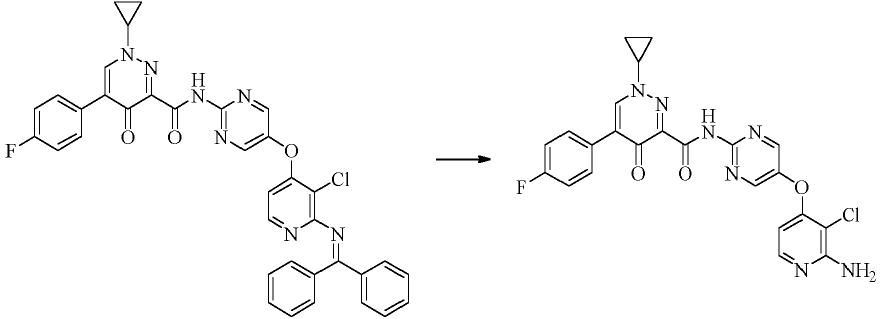

*N*-(5-((3-chloro-2-((diphenylmethylene)amino)pyridin-4-yl)oxy)pyrimidi n-2-yl)-5-(4-fluorophenyl)-1-cyclopropyl-4-oxo-1,4-dihydropyridazine-3-carb oxamide (360.0 mg, 0.54 mmol, 1.0 eq) was dissolved in methanol (6 mL), and the mixture was added with concentrated hydrochloric acid (0.15 mL) and reacted at room temperature for 1 h; after the reaction was completed as monitored by TLC, the reaction solution was added with a saturated aqueous sodium carbonate solution to adjust the pH value to 8 and added with ethyl acetate (100 mL), followed by liquid separation, the aqueous phase was extracted with dichloromethane (100 mL), the organic phases were combined, dried over anhydrous sodium sulfate and filtered, and the filtrate was purified by preparative thin-layer chromatography (dichloromethane:methanol = 20:1) to give the product (158.0 mg, yield: 58.5%).

¹HNMR (300 MHz, DMSO-*d₆*) δ(ppm): 12.79 (s, 1H), 8.88 (s, 1H), 8.66 (s, 2H), 7.88-8.19 (m, 2H), 7.72-7.78 (m, 1H), 7.28-7.34 (t, *J*=9 Hz, 2H), 6.49 (s, 2H), 6.10-6.12 (d, *J*=2 Hz, 1H), 4.04-4.11 (m, 1H), 1.22-1.29 (m, 2H), 1.10-1.12 (m, 2H).

Molecular formula: C₂₃H₁₇ClFN₇O₃; molecular weight: 493.88; LC-MS(Pos, *m*/*z*)=494.17[M+H]⁺.

### Example 6: Synthesis of N-(6-((2-amino-3-chloropyridin-4-yl)oxy)pyridin-3-yl)-1-cyclopropyl-5-(4-flu orophenyl)-4-oxo-1,4-dihydropyridazine-3-carboxamide (Compound 19)

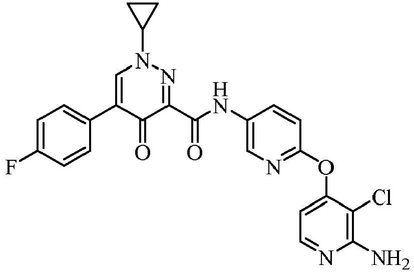

### Step 1: Synthesis of 2,3-dichloro-4-((5-nitropyridin-2-yl)oxy)pyridine

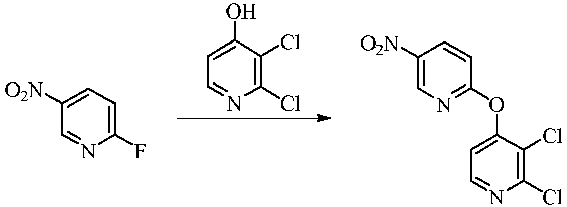

2-fluoro-5-nitropyridine (11.0 g, 77.38 mmol, 1.0 eq), 2,3-dichloro-4-hydroxylpyridine (12.69 g, 77.38 mmol, 1.0 eq) and lithium carbonate (8.58 g, 116.07 mmol, 1.5 eq) were added to DMSO (110 mL), and the mixture was stirred at 120 °C for 16 h; after the reaction was completed as monitored by TLC, the reaction solution was added with ethyl acetate (300 mL), washed with saturated brine (50 mL × 4), dried over anhydrous sodium sulfate, and filtered under vacuum, the filtrate was concentrated under reduced pressure, and the crude product was slurried with ethyl acetate (50 mL) and filtered under vacuum to give the product (20.55 g, yield: 92.8%).

### Step 2: Synthesis of N-(3-chloro-4-((5-nitropyridin-2-yl)oxy)pyridin-2-yl)-1,1-diphenylmethylamin e

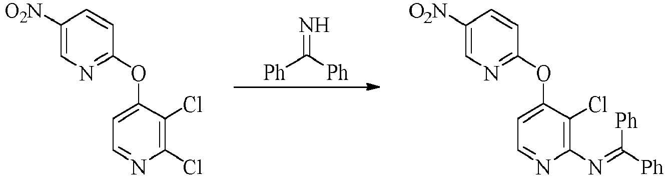

2,3-dichloro-4-((5-nitropyridin-2-yl)oxy)pyridine (20.80 g, 72.71 mmol, 1.0 eq), benzophenone imine (13.174 g, 72.69 mmol, 1.0 eq), palladium acetate (0.408 g, 1.82 mmol, 0.025 eq), BINAP (1.721 g, 2.76 mmol, 0.038 eq) and cesium carbonate (47.38 g, 145.42 mmol, 2.0 eq) were added to 1,4-dioxane (160 mL), and the mixture was stirred overnight at 100 °C in nitrogen atmosphere; after the reaction was completed as monitored by TLC, the reaction solution was concentrated under reduced pressure, added with ethyl acetate (200 mL), sequentially washed with water (50 mL × 3) and saturated brine (50 mL), dried over anhydrous sodium sulfate, and filtered under vacuum, the filtrate was concentrated under reduced pressure, and the crude product was purified by silica gel column chromatography (DCM:MeOH = 60:1) to give the product (11.3 g, yield: 36.1%).

### Step 3: Synthesis of 6-((3-chloro-2-((diphenylmethylene)amino)pyridin-4-yl)oxy)pyridin-3-amine

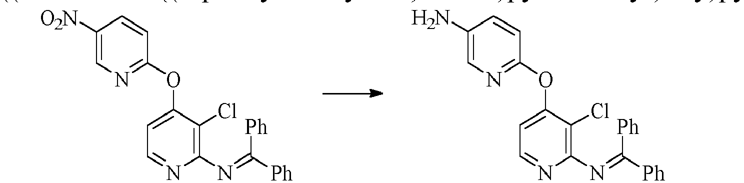

*N*-(3-chloro-4-((5-nitropyridin-2-yl)oxy)pyridin-2-yl)-1,1-diphenylmetha nimine (7.00 g, 16.25 mmol, 1.0 eq) was dissolved in isopropanol (70 mL), and the mixture was added with a 20% aqueous ammonium sulfide solution (55.37 g, 162.52 mmol, 10.0 eq), stirred and reacted at 20 °C for 1.0 h in nitrogen atmosphere, heated to 70 °C and then stirred and reacted for 2.5 h; after the reaction was completed as monitored by TLC, the reaction solution was concentrated under reduced pressure, added with ethyl acetate (80 mL), washed with saturated brine (40 mL × 2), dried over anhydrous sodium sulfate, and filtered under vacuum, the filtrate was concentrated under reduced pressure, and the crude product was purified by silica gel column chromatography (DCM:MeOH = 50:1) to give the product (2.73 g, yield: 41.9%).

### Step 4: Synthesis of N-(6-((3-chloro-2-((diphenylmethylene)amino)pyridin-4-yl)oxy)pyridin-3-yl)-1-cyclopropyl-5-(4-fluorophenyl)-4-oxo-1,4-dihydropyridazine-3-carboxamid e

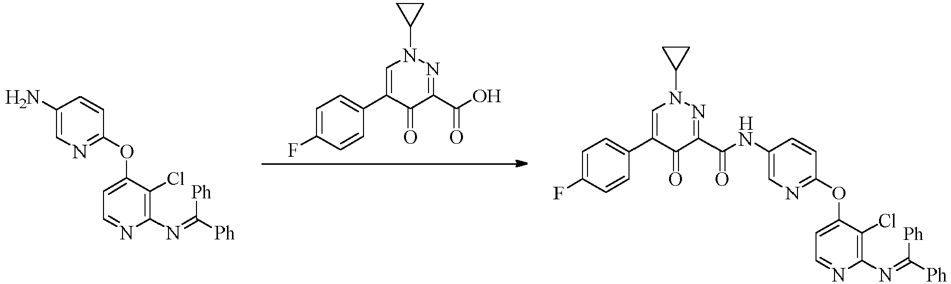

6-((3-chloro-2-((diphenylmethylene)amino)pyridin-4-yl)oxy)pyridin-3-a mine (200.0 mg, 0.50 mmol, 1.0 eq) and 1-cyclopropyl-5-(4-fluorophenyl)-4-oxo-1,4-dihydropyridazine-3-carboxylic acid (137.0 mg, 0.50 mmol, 1.0 eq) were dissolved in pyridine (5 mL), and the mixture was added with POCl₃ (115.0 mg, 0.75 mmol, 1.5 eq) and stirred at room temperature for 0.5 h; after the reaction was completed as monitored by TLC, the reaction solution was poured into water (15 mL), and extracted with ethyl acetate (15 mL × 2), the organic phases were combined, washed with saturated brine (15 mL), dried over anhydrous sodium sulfate and filtered under vacuum, the filtrate was concentrated under reduced pressure, and the crude product was purified by preparative thin-layer chromatography (DCM: MeOH = 20:1) to give the product (152.0 mg, yield: 46.4%).

### Step 5: Synthesis of N-(6-((2-amino-3-chloropyridin-4-yl)oxy)pyridin-3-yl)-1-cyclopropyl-5-(4-flu orophenyl)-4-oxo-1,4-dihydropyridazine-3-carboxamide

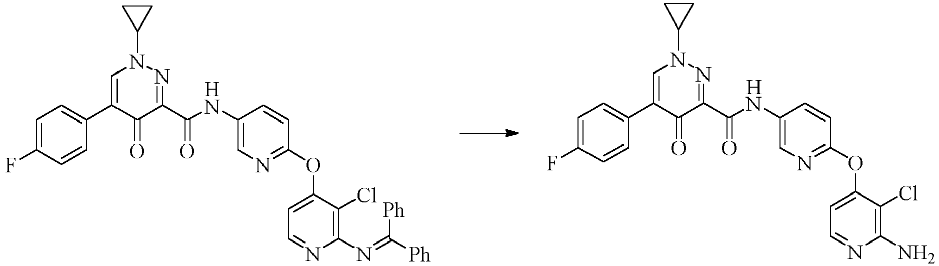

*N*-(6-((3-chloro-2-((diphenylmethylene)amino)pyridin-4-yl)oxy)pyridin-3-yl)-1-cyclopropyl-5-(4-fluorophenyl)-4-oxo-1,4-dihydropyridazine-3-carbox amide (152.0 mg, 0.23 mmol, 1.0 eq) was dissolved in methanol (5 mL), and the mixture was added dropwise with concentrated hydrochloric acid (0.1 mL) with stirring and reacted at room temperature for 0.5 h; after the reaction was completed as monitored by TLC, the reaction solution was poured into a saturated aqueous NaHCO₃ solution (10 mL), and extracted with dichloromethane (15 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM: MeOH = 15:1) to give the product (91.0 mg, yield: 79.8%).

¹H NMR (400 MHz, DMSO-*d₆*) δ(ppm): 12.21 (s, 1H), 8.91 (s, 1H), 8.51-8.50 (d, *J* = 2.6 Hz, 1H), 8.31-8.28 (dd, *J* = 2.7, 6.1 Hz, 1H), 7.95-7.91 (m, 2H), 7.87-7.85 (d, *J* = 5.5 Hz, 1H), 7.35-7.31 (m, 2H), 7.22-7.19 (d, *J* = 8.8 Hz, 1H), 6.42 (s, 2H), 6.36-6.35 (d, *J* = 5.5 Hz, 1H), 4.13-4.07 (m, 1H), 1.31-1.30 (m, 2H), 1.12-1.10 (m, 2H).

Molecular formula: C₂₄H₁₈ClFN₆O₃; molecular weight: 492.90; LC-MS(Pos, *m*/*z*)=493.10[M+H]⁺.

### Example 7: Synthesis of N-(5-((2-amino-3-chloropyridin-4-yl)oxy)pyridin-2-yl)-5-(4-fluorophenyl)-1-methyl-4-oxo-1,4-dihydropyridazine-3-carboxamide (Compound 33)

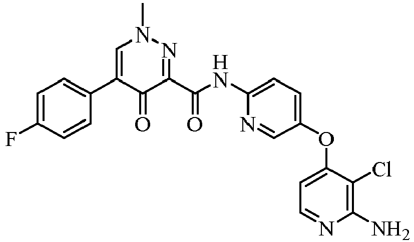

### Step 1: Synthesis of N-(5-((3-chloro-2-((diphenylmethylene)amino)pyridin-4-yl)oxy)pyridin-2-yl)-5-(4-fluorophenyl)-1-methyl-4-oxo-1,4-dihydropyridazine-3-carboxamide

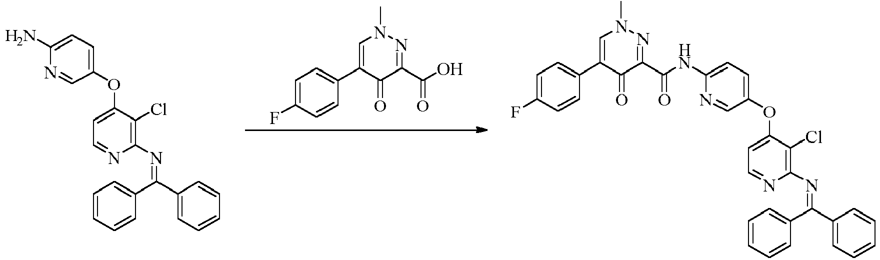

5-(4-fluorophenyl)-1-methyl-4-oxo-1,4-dihydropyridazine-3-carboxylic acid (124.03 mg, 0.5 mmol, 1.0 eq) and 5-((3-chloro-2-(((diphenylmethylene)amino)pyridin-4-yl)oxy)pyridin-2-amine (200.0 mg, 0.5 mmol, 1.0 eq) were dissolved in pyridine (5 mL), and the mixture was added dropwise with phosphorus oxychloride (329.0 mg, 2.15 mmol, 4.3 eq) and stirred at room temperature for 1 h; after the reaction was completed as monitored by TLC, the reaction was quenched with water (20 mL), the reaction solution was extracted with DCM (50 mL × 3), the organic phases were combined, washed with water, washed with saturated brine, dried over anhydrous sodium sulfate and filtered, and the filtrate was concentrated under reduced pressure to give the product (210.0 mg, yield: 66.6%).

### Step 2: Synthesis of N-(5-((2-amino-3-chloropyridin-4-yl)oxy)pyridin-2-yl)-5-(4-fluorophenyl)-1-methyl-4-oxo-1,4-dihydropyridazine-3-carboxamide

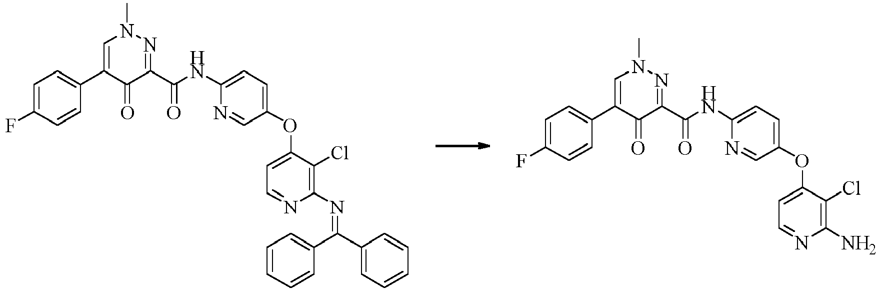

*N*-(5-((3-chloro-2-((diphenylmethylene)amino)pyridin-4-yl)oxy)pyridin-2 -yl)-5-(4-fluorophenyl)-1-methyl-4-oxo-1,4-dihydropyridazine-3-carboxamide (210.0 mg, 0.33 mmol, 1.0 eq) was added to methanol (2 mL), and the mixture was added with hydrochloric acid (1 mol/L, 1 mL) and stirred at room temperature for 5 h; after the reaction was completed as monitored by TLC, the reaction solution was added with a saturated aqueous NaHCO₃ solution to adjust the pH value to 7-8, and extracted with EA, the organic phases were combined, washed with water, washed with saturated brine, dried over anhydrous sodium sulfate and filtered, the filtrate was concentrated under reduced pressure, and the crude product was separated by preparative thin-layer chromatography (DCM:MeOH = 10:1) to give the product (53.0 mg, yield: 34.14%).

¹HNMR (300 MHz, CDCl₃) δ(ppm): 13.10 (s, 1H), 8.91 (s, 1H), 8.37-8.34 (d, 1H), 8.30-8.29 (d, 1H), 7.97-7.91 (m, 2H), 7.80-7.79 (d, 1H), 7.78-7.74 (m, 1H), 7.38-7.32 (t, 2H), 6.46 (s, 2H), 6.07-6.05 (d, 1H), 4.14 (s, 3H).

Molecular formula: C₂₂H₆ClFN₆O₃; molecular weight: 466.86; LC-MS(Pos,*m*/*z*)=467.04[M+H]⁺.

### Example 8: Synthesis of N-(5-((2-aminopyridin-4-yl)oxy)pyridin-2-yl)-5-(4-fluorophenyl)-1-isopropyl-4-oxo-1,4-dihydropyridazine-3-carboxamide (Compound 34)

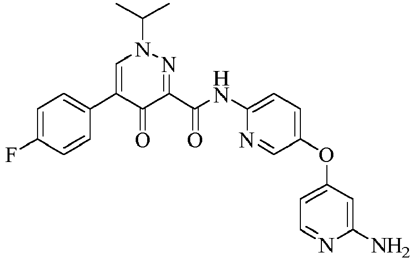

### Step 1: Synthesis of 2-chloro-4-((6-nitropyridin-3-yl)oxy)pyridine

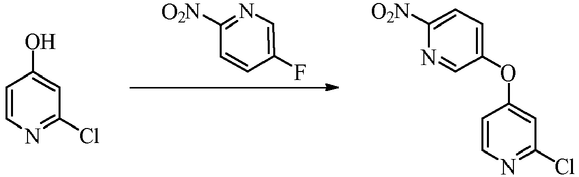

2-chloro-4-hydroxylpyridine (500 mg, 3.86 mmol, 1.0 eq), 5-fluoro-2-nitropyridine (550 mg, 3.86 mmol, 1.0 eq) and K₂CO₃ (1.07 g, 7.72 mmol, 2.0 eq) were added to DMF (5 mL), and the mixture was heated to 88 °C and reacted for 16 h; after the reaction was completed as monitored by TLC, the reaction was quenched with H₂O (30 mL), the reaction solution was extracted with EA (20 mL × 2), the organic phase was concentrated, and the crude product was separated by silica gel column chromatography (DCM:MeOH = 20:1) to give the product (800 mg, yield: 82.4%).

### Step 2: Synthesis of N-(4-((6-nitropyridin-3-yl)oxy)pyridin-2-yl)-1,1-diphenylazomethine

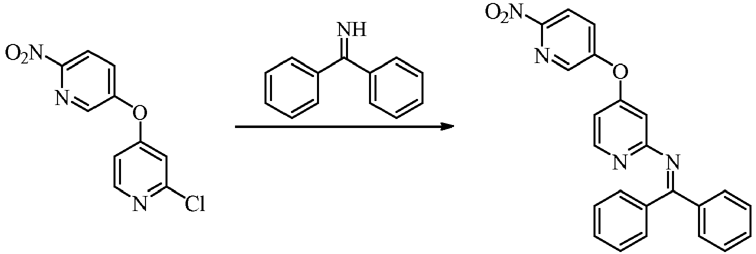

2-chloro-4-((6-nitropyridin-3-yl)oxy)pyridine (720 mg, 2.86 mmol, 1.0 eq), benzophenone imine (519 mg, 2.86 mmol, 1.0 eq), Pd(OAc)₂ (16.1 mg, 0.072 mmol, 0.025 eq), BINAP (67.7 mg, 0.11 mmol) and Cs₂CO₃ (1.86 g, 5.72 mmol) were added to 1,4-dioxane (8 mL), and the mixture was heated to 100 °C in nitrogen atmosphere for 24 h; the reaction was quenched with H₂O (30 mL), the reaction solution was extracted with EA (20 mL × 2), the organic phase was concentrated, and the crude product was separated by silica gel column chromatography (PE:EA = 5:1-2:1) to give the product (476 mg, yield: 42.0%).

### Step 3: Synthesis of 5((2-((diphenylmethylene)amino)pyridin-4-yl)oxy)pyridin-2-amine

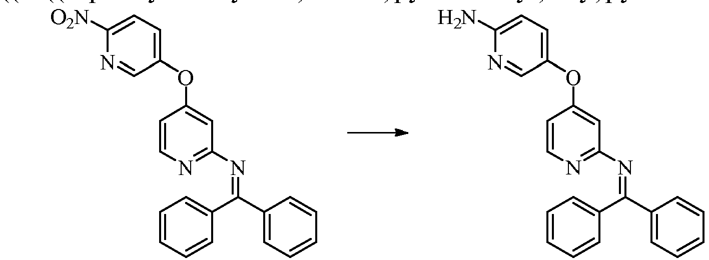

*N-*(4-((6-nitropyridin-3-yl)oxy)pyridin-2-yl)-1,1-diphenylazomethine (470 mg, 1.19 mmol, 1.0 eq), FeSO₄·7H₂O (6.59 g, 23.7 mmol, 20.0 eq) and NH₃·H₂O (2.49 g, 71.1 mmol, 60.0 eq) were dissolved in EtOH (10 mL), and the mixture was heated to 80 °C for 2 h; after the starting materials reacted completely as detected by TLC, the reaction solution was filtered under vacuum, the filtrate was added with H₂O (20 mL), added with NH₃·H₂O (1 mL), and extracted with EA (20 mL × 2), the organic phase was concentrated, and the crude product was separated by silica gel column chromatography (DCM:MeOH = 50:1-30:1) to give the product (290 mg, yield: 66.8%).

### Step 4: Synthesis of N-(5-((2-((diphenylmethylene)amino)pyridin-4-yl)oxy)pyridin-2-yl)-5-(4-fluor ohenl)-1-isorol-4-oxo-1,4-dihydropyridazine-3-carboxamide

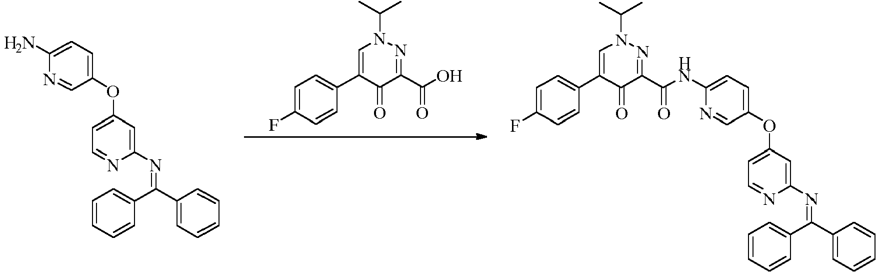

5((2-((diphenylmethylene)amino)pyridin-4-yl)oxy)pyridin-2-amine (290 mg, 0.79 mmol, 1.0 eq) and 5-(4-fluorophenyl)-1-isopropyl-4-oxo-1,4-dihydropyridazine-3-carboxylic acid (219 mg, 0.79 mmol, 1.0 eq) were dissolved in pyridine (5 mL), and the mixture was added dropwise with phosphorus oxychloride (182 mg, 1.19 mmol, 1.5 eq) and stirred at room temperature for 20 min; after the reaction was completed as monitored by TLC, the reaction was quenched with water (30 mL), the reaction solution was extracted with EA (50 mL), the organic phase was dried over anhydrous sodium sulfate and filtered, the filtrate was concentrated under reduced pressure, and the crude product was separated by silica gel column chromatography (DCM:MeOH = 100:1-50:1) to give the product (350 mg, yield: 70.8%).

### Step 5: Synthesis of N-(5-((2-aminopyridin-4-yl)oxy)pyridin-2-yl)-5-(4-fluorophenyl)-1-isopropyl-4-oxo-1,4-dihydropyridazine-3-carboxamide

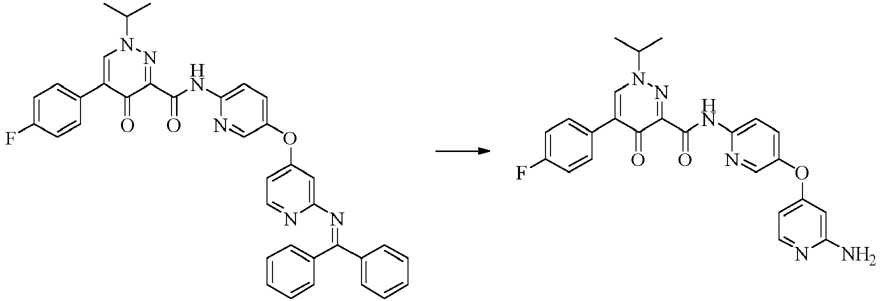

*N*-(5-((2-((diphenylmethylene)amino)pyridin-4-yl)oxy)pyridin-2-yl)-5-(4-fluorophenyl)-1-isopropyl-4-oxo-1,4-dihydropyridazine-3-carboxamide (350 mg, 0.560 mmol, 1.0 eq) was added to methanol (4 mL), and the mixture was added with water (0.7 mL) and concentrated hydrochloric acid (0.2 mL), and stirred at room temperature for 30 min; after the reaction was completed as monitored by TLC, the reaction solution was added with a saturated aqueous NaHCO₃ solution to adjust the pH value to 7-8, and extracted with EA (20 mL), the organic phase was dried over anhydrous sodium sulfate and filtered, the filtrate was concentrated under reduced pressure, and the crude product was separated by silica gel column chromatography (DCM:MeOH = 60:1-30:1) to give the product (170 mg, yield: 65.9%).

¹HNMR (300 MHz, DMSO) δ(ppm): 13.03 (s, 1H), 8.93 (s, 1H), 8.39-8.36 (d, *J* = 9.0 Hz, 1H), 8.28-8.27 (d, *J* = 3.0 Hz, 1H), 7.98-7.93 (m,

2H), 7.85-7.83 (d, *J* = 5.7 Hz, 1H), 7.78-7.74 (dd, *J* = 9.0, 3.0 Hz, 1H), 7.37-7.31 (t, *J* = 8.9 Hz, 2H), 6.24-6.21 (dd, *J=* 6.0, 2.4 Hz, 1H), 6.06 (s, 2H), 5.88-5.87 (d, *J* = 2.1 Hz, 1H), 4.81-4.72 (m, 1H), 1.54 (s, 3H), 1.52 (s, 3H).

Molecular formula: C₂₄H₂₁FN₆O₃; molecular weight: 460.47; LC-MS(Pos, *m*/*z*)=461.18[M+H]⁺.

### Example 9: Synthesis of 1-allyl-N-(5-((2-amino-3-chloropyridin-4-yl)oxy)pyridin-2-yl)-5-(4-fluorophe nyl)-4-oxo-1,4-dihydropyridazine-3-carboxamide (Compound 35)

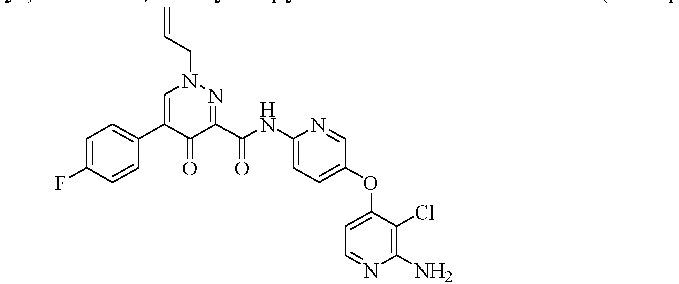

### Step 1: Synthesis of ethyl 1-allyl-5-(4-fluorophenyl)-4-oxo-1,4-dihydropyridazine-3-carboxylate

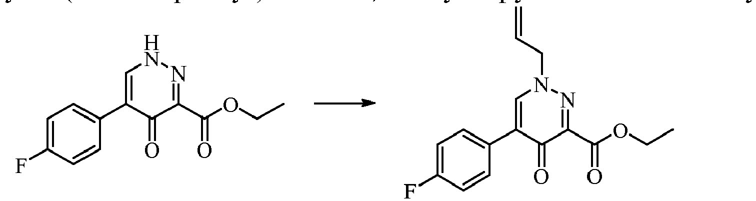

Ethyl 5-(4-fluorophenyl)-4-oxo-1,4-dihydropyridazine-3-carboxylate (475.0 mg, 1.81 mmol, 1.0 eq), bromopropene (442.0 mg, 3.65 mmol, 2.0 eq) and potassium carbonate (1.38 g, 9.96 mmol, 5.5 eq) were added to DMF (15 mL), and the mixture was stirred and reacted at 50 °C for 3 h; after the reaction was completed as monitored by TLC, the reaction solution was poured into water (15 mL), and extracted with ethyl acetate (15 mL × 2), the organic phases were combined, washed with saturated brine (15 mL), dried over anhydrous sodium sulfate and filtered under vacuum, the filtrate was concentrated under reduced pressure, and the crude product was purified by silica gel column chromatography (DCM:MeOH = 20:1) to give the product (463.0 mg, yield: 84.6%).

### Step 2: Synthesis of 1-allyl-5-(4-fluorophenyl)-4-oxo-1,4-dihydropyridazine-3-carboxylic acid

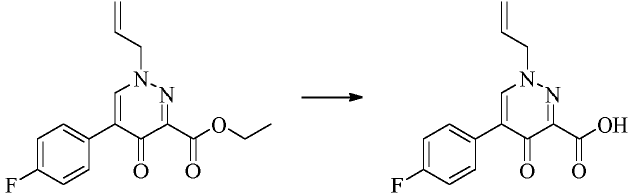

### Ethyl

1-allyl-5-(4-fluorophenyl)-4-oxo-1,4-dihydropyridazine-3-carboxylate (463.0 mg, 1.53 mmol, 1.0 eq) and lithium hydroxide monohydrate (531.0 mg, 12.67 mmol, 8.25 eq) were added to a mixed solution of methanol (30 mL) and water (10 mL), and the mixture was stirred and reacted at room temperature for 2 h; after the reaction was completed as monitored by TLC, the reaction solution was concentrated under reduced pressure, added with water (20 mL), and extracted with ethyl acetate (10mL), the aqueous phase was added dropwise with concentrated hydrochloric acid to adjust the pH value to 3, a large amount of precipitate was precipitated, and filtered under vacuum, the filter cake was dissolved in dichloromethane (30 mL), dried over anhydrous sodium sulfate, and filtered under vacuum, and the filtrate was concentrated under reduced pressure to give the product (418.0 mg, yield: 99.5%).

### Step 3: Synthesis of 1-allyl-N-(5-((3-chloro-2-((diphenylmethylene)amino)pyridin-4-yl)oxy)pyridi n-2-yl)-5-(4-fluorophenyl)-4-oxo-1,4-dihydropyridazine-3-carboxamide

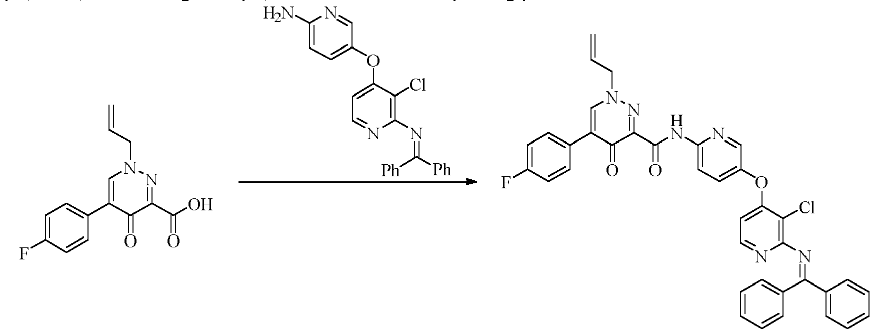

1-allyl-5-(4-fluorophenyl)-4-oxo-1,4-dihydropyridazine-3-carboxylic acid (274.0 mg, 1.00 mmol, 1.0 eq) and 5-((3-chloro-2-((diphenylmethylene)amino)pyridin-4-yl)oxy)pyridin-2-amine (400.0 mg, 1.00 mmol, 1.0 eq) were dissolved in pyridine (5 mL), and the mixture was added with POCl₃ (230.0 mg, 1.50 mmol, 1.5 eq), and stirred and reacted at room temperature for 0.5 h; after the reaction was completed as monitored by TLC, the reaction solution was poured into water (15 mL), and extracted with ethyl acetate (15 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 20:1) to give the product (417.0 mg, yield: 63.5%).

### Step 4: Synthesis of 1-allyl-N-(5-((2-amino-3-chloropyridin-4-yl)oxy)pyridin-2-yl)-5-(4-fluorophe nyl-4-oxo-14-dihydropyridazine-3-carboxamide

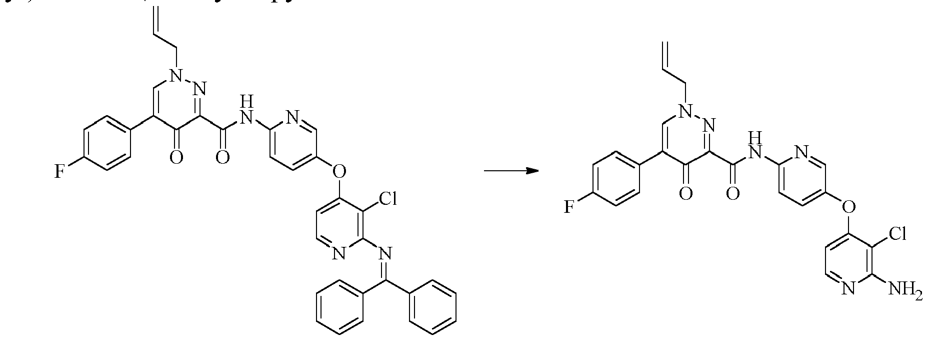

1-allyl-*N-*(5-((3-chloro-2-((diphenylmethylene)amino)pyridin-4-yl)oxy)p yridin-2-yl)-5-(4-fluorophenyl)-4-oxo-1,4-dihydropyridazine-3-carboxamide (417.0 mg, 0.63 mmol, 1.0 eq) was dissolved in methanol (5 mL), and the mixture was added dropwise with concentrated hydrochloric acid (0.1 mL) with stirring and reacted at room temperature for 0.5 h; after the reaction was completed as monitored by TLC, the reaction solution was poured into a saturated aqueous NaHCO₃ solution (10 mL), and extracted with dichloromethane (15 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 15:1) to give the product (230.0 mg, yield: 73.5%).

¹H NMR (400 MHz, DMSO-*d₆*) δ(ppm): 12.96 (s, 1H), 8.91 (s, 1H), 8.36-8.34 (d, *J=* 9.0 Hz, 1H), 8.29-8.28 (d, *J* = 2.8 Hz, 1H), 7.95-7.91 (dd, *J* = 5.7, 3.0 Hz, 2H), 7.80-7.74 (m, 2H), 7.37-7.33 (t, *J* = 8.9 Hz, 2H), 6.45 (s, 2H), 6.20-6.11(m, 1H), 6.06-6.05 (d, *J* = 5.6 Hz, 1H), 5.39-5.35 (m, 2H), 5.00-4.98 (d, *J* = 5.7 Hz, 2H).

Molecular formula: C₂₄H₁₈ClFN₆O₃; molecular weight: 492.90; LC-MS(Pos, *m*/*z*)=493.12[M+H]⁺.

### Example 10: Synthesis of N-(5-((2-amino-3-chloropyridin-4-yl)oxy)pyridin-2-yl)-2-cyclopropyl-6-(4-flu orophenyl)-5-oxo-2,5-dihydropyridazine-4-carboxamide (Compound 36) hydrochloride

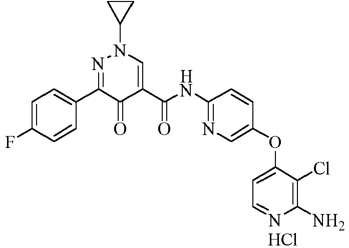

### Step 1: Synthesis of N-(5-((3-chloro-2-((diphenylmethylene)amino)pyridin-4-yl)oxy)pyridin-2-yl)-2-cyclopropyl-6-(4-fluorophenyl)-5-oxo-2,5-dihydropyridazine-4-carboxamid e

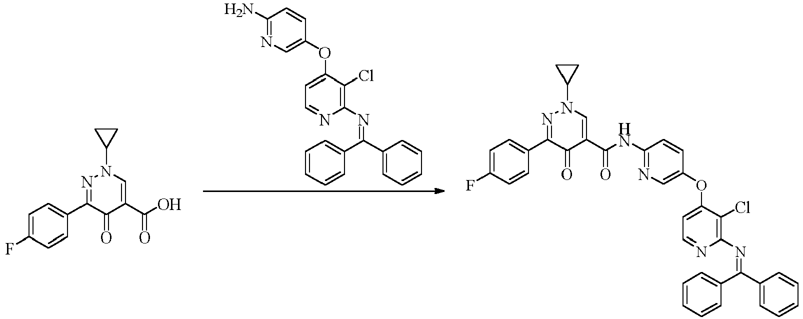

2-cyclopropyl-6-(4-fluorophenyl)-5-oxo-2,5-dihydropyridazine-4-carbox ylic acid (109.1 mg, 0.39 mmol, 1.05 eq) was added to pyridine (5 mL), and the mixture was added with 5-((3-chloro-2-((diphenylmethylene)amino)pyridin-4-yl)oxy)pyridin-2-amine (152.0 mg, 0.37 mmol, 1.0 eq) and phosphorus oxychloride (0.1 mL) and stirred at room temperature for 10 min; after the reaction was completed as monitored by TLC, the reaction solution was added with ethyl acetate (50 mL) and water (50 mL), followed by liquid separation, the organic phase was dried over anhydrous sodium sulfate and filtered, and the filtrate was concentrated under reduced pressure and purified by preparative thin-layer chromatography to give *N*-(5-((3-chloro-2-((diphenylmethylene)amino)pyridin-4-yl)oxy)pyridin-2-yl)-2-cyclopropyl-6-(4-fluorophenyl)-5-oxo-2,5-dihydropyridazine-4-carboxamid e (175.0 mg, yield: 66.8%).

### Step 2: Synthesis of N-(5-((2-amino-3-chloropyridin-4-yl)oxy)pyridin-2-yl)-2-cyclopropyl-6-(4-flu orophenyl)-5-oxo-2,5-dihydropyridazine-4-carboxamide hydrochloride

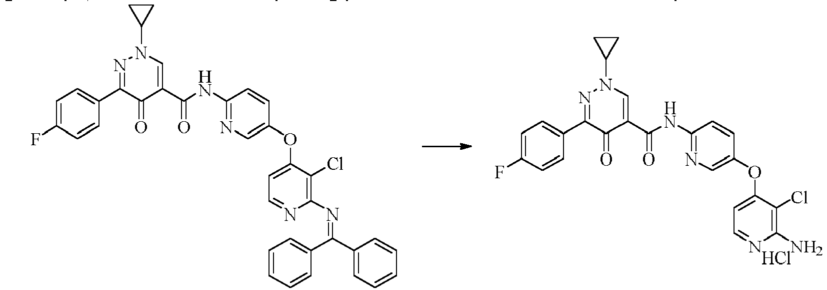

*N*-(5-((3-chloro-2-((benzhydryl)amino)pyridin-4-yl)oxy)pyridin-2-yl)-2-c yclopropy1-6-(4-fluorophenyl)-5-oxo-2,5-dihydropyridazine-4-carboxamide (175.0 mg, 0.26 mmol, 1.0 eq) was added to methanol (6 mL), and the mixture was added with concentrated hydrochloric acid (0.5 mL) and stirred at room temperature for 0.5 h; after the reaction was completed as monitored by TLC, the system was filtered, and the filter cake was dried to give the product *N-*(5-((2-amino-3-chloropyridin-4-yl)oxy)pyridin-2-yl)-2-cyclopropyl-6-(4-flu orophenyl)-5-oxo-2,5-dihydropyridazine-4-carboxamide hydrochloride (100.0 mg, yield: 70.9%).

¹HNMR (400 MHz, DMSO-*d₆*) δ(ppm): 12.66 (s, 1H), 9.24 (s, 1H), 8.36-8.41 (m, 2H), 8.12-8.15 (m, 2H), 7.90-7.92 (m, 1H), 7.83-7.86 (m, 1H), 7.73-7.75 (m, 1H), 7.32-7.36 (t, J=6.84 Hz, 2H), 6.34-6.36 (m, 1H), 4.25-4.27 (m, 1H), 1.30-1.33 (m, 2H), 1.09-1.11 (m, 2H).

Molecular formula: C₂₄H₁₉ Cl₂FN₆O₃; molecular weight: 529.35; LC-MS(Pos, *m*/*z*)=493.00[M+H]⁺.

### Example 11: Synthesis of 1-allyl-N-(5-(((2-amino-3-chloropyridin-4-yl)oxy)pyrimidin-2-yl)-5-(4-fluorop henyl)-4-oxo-1,4-dihydropyridazine-3-carboxamide (Compound 37)

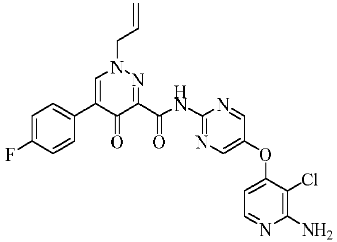

### Step 1: Synthesis of ethyl 1-allyl-5-(4-fluorophenyl)-4-oxo-1,4-dihydropyridazine-3-carboxylate

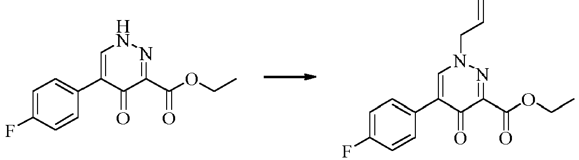

Ethyl 5-(4-fluorophenyl)-4-oxo-1,4-dihydropyridazine-3 -carboxylate (320.0 mg, 1.22 mmol, 1.0 eq) was dissolved in DMF (10 mL), and the mixture was added with potassium carbonate (506.0 mg, 3.66 mmol, 3.0 eq) and allyl bromide (162.0 mg, 1.34 mmol, 1.1 eq) and reacted at 50 °C for 2 h; after the reaction was completed as monitored by TLC, the reaction solution was cooled to room temperature, diluted with ethyl acetate (30 mL), washed with saturated brine (30 mL × 4), dried over anhydrous sodium sulfate, and filtered under vacuum, the filtrate was concentrated, and the crude product was purified by preparative thin-layer chromatography (dichloromethane:methanol = 20:1) to give the product (343.0 mg, yield: 93.2%).

### Step 2: Synthesis of 1-allyl-5-(4-fluorophenyl)-4-oxo-1,4-dihydropyridazine-3-carboxylic acid

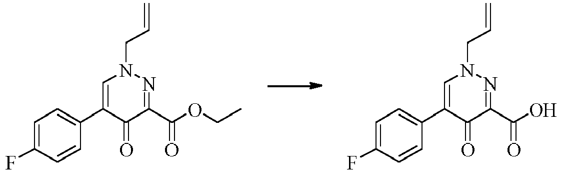

### Ethyl

1-allyl-5-(4-fluorophenyl)-4-oxo-1,4-dihydropyridazine-3-carboxylate (343.0 mg, 1.13 mmol, 1.0 eq) was added to methanol (6 mL), and the mixture was added with an aqueous solution (3 mL) of lithium hydroxide monohydrate (142.7 mg, 3.40 mmol, 3.0 eq) and reacted at room temperature for 1 h; after the reaction was completed as monitored by TLC, the reaction system was concentrated, and added with 6 mol/L hydrochloric acid to adjust the pH value to 3-4, a solid was precipitated, stirred for 1 h, and filtered under vacuum, and the filter cake was dried to give the product (310.0 mg, yield: 100%).

### Step 3: Synthesis of 5-((3-chloro-2-((4-methoxybenzyl)amino)pyridin-4-yl)oxy)pyrimidin-2-amine

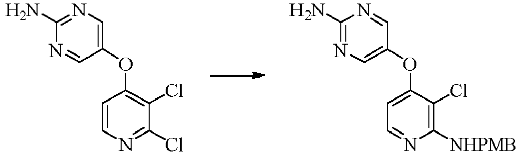

5-((2,3-dichloropyridin-4-yl)oxy)pyrimidin-2-amine (1.0 g, 3.88 mmol, 1.0 eq) was added to (4-methoxyphenyl)methylamine (4.8 g, 34.92 mmol, 9.0 eq), and the mixture was reacted at 150 °C for 2 h under microwave; after the reaction was completed as monitored by TLC, the reaction solution was added with ethyl acetate (200 mL) and water (150 mL), followed by liquid separation, the aqueous phase was extracted with DCM (200 mL), the organic phases were combined, dried over anhydrous sodium sulfate, and filtered under vacuum, the filtrate was concentrated, and the crude product was purified by silica gel column chromatography (100-200 mesh silica gel, dichloromethane:methanol = 100:1-80:1) to give the product (480.0 mg, yield: 34.5%).

### Step 4: Synthesis of 1-allyl-N-(5-((3-chloro-2-((4-methoxybenzyl)amino)pyridin-4-yl)oxy)pyrimidi n-2-yl)-5-(4-fluorophenyl)-4-oxo-1,4-dihydropyridazine-3-carboxamide

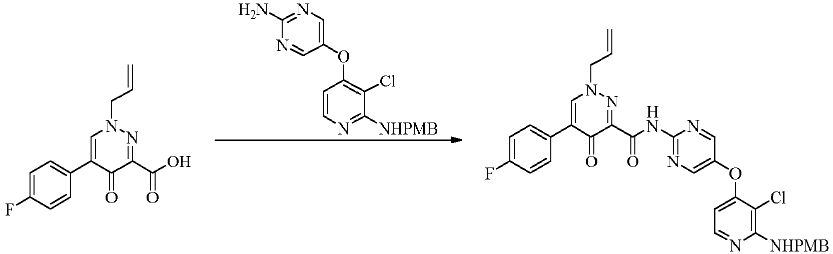

1-allyl-5-(4-fluorophenyl)-4-oxo-1,4-dihydropyridazine-3-carboxylic acid (276.4 mg, 1.00 mmol, 1.0 eq) was added to pyridine (6 mL), and the mixture was added with 5-((3-chloro-2-((4-methoxybenzyl)amino)pyridin-4-yl)oxy)pyrimidin-2-amine (360.6 mg, 1.00 mmol, 1.0 eq) and phosphorus oxychloride (0.1 mL) and stirred at room temperature for 15 min; after the reaction was completed as monitored by TLC, the reaction solution was added with ethyl acetate (200 mL) and water (150 mL), followed by liquid separation, the aqueous phase was extracted with ethyl acetate (100 mL), and washed with a saturated aqueous ammonium chloride solution, the organic phases were combined, dried over anhydrous sodium sulfate and filtered under vacuum, the filtrate was concentrated, and the crude product was purified by silica gel column chromatography (100-200 mesh silica gel, dichloromethane:methanol = 100:1-60:1) to give the product (300.0 mg, yield: 48.4%).

### Step 5: Synthesis of 1-allyl-N-(5-(((2-amino-3-chloropyridyl-4-yl)oxy)pyrimidin-2-yl)-5-(4-fluorop henyl)-4-oxo-1,4-dihydropyridazine-3-carboxamide

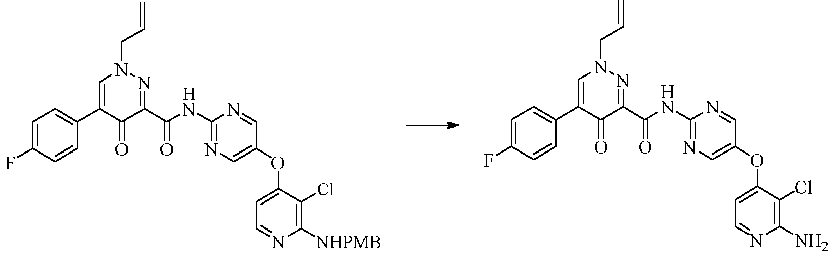

1-allyl-*N*-(5-((3-chloro-2-((4-methoxybenzyl)amino)pyridin-4-yl)oxy)pyr imidin-2-yl)-5-(4-fluorophenyl)-4-oxo-1,4-dihydropyridazine-3-carboxamide (263.0 mg, 0.42 mmol, 1.0 eq) was added to DCM (2 mL), and the mixture was added with trifluoroacetic acid (4 mL) and reacted at 60 °C for 3 h; after the reaction was completed as monitored by TLC, the reaction system was cooled to room temperature, added with a saturated aqueous sodium carbonate solution to adjust the pH value to 8, and added with DCM (200 mL), followed by liquid separation, the organic phase was dried over anhydrous sodium sulfate and filtered, the filtrate was concentrated, and the crude product was purified by preparative thin-layer chromatography (dichloromethane:methanol = 20:1) to give the product (187.0 mg, yield: 88.6%).

¹HNMR (300 MHz, DMSO-*d₆*) δ(ppm): 12.79-12.81 (s, 1H), 8.87 (s, 1H), 8.67 (s, 2H), 7.88-7.92 (t, *J*=12 Hz, 2H), 7.77-7.79 (d, *J*=6 Hz, 1H), 7.30-7.36 (t, *J*=9 Hz, 2H), 6.50 (s, 2H), 6.08-6.13 (t, *J*=6 Hz, 2H), 5.30-5.35 (t, *J*=9 Hz, 2H), 4.94-4.95 (d, *J*=3 Hz, 2H).

Molecular formula: C₂₃H₁₇ClFN₇O₃; molecular weight: 493.88; LC-MS(Pos, *m*/*z*)=494.11M+H]⁺.

### Example 12: Synthesis of N-(6-((2-amino-3-chloropyridin-4-yl)oxy)pyridin-3-yl)-5-(4-fluorophenyl)-1-i sopropyl-4-oxo-1,4-dihydropyridazine-3-carboxamide (Compound 13)

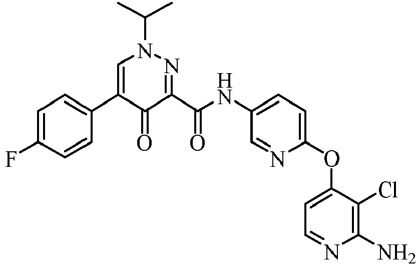

### Step 1: Synthesis of N-(6-((3-chloro-2-((diphenylmethylene)amino)pyridin-4-yl)oxy)pyridin-3-yl)-5-(4-fluorohenl)-1-isorol-4-oxo-1,4-dihdroridazine-3-carboxamide

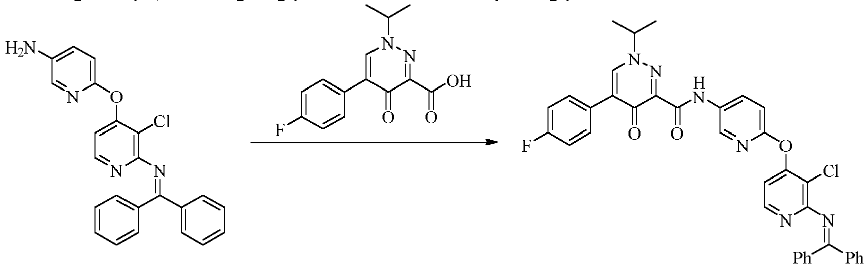

6-((3-chloro-2-(diphenylmethylene)aminopyridin-4-yl)oxy)pyridin-3-ami ne (435 mg, 1.09 mmol, 1.0 eq) and 5-(4-fluorophenyl)-1-isopropyl-4-oxo-1,4-dihydropyridazine-3-carboxylic acid (300 mg, 1.09 mmol, 1.0 eq) were added to pyridine (5 mL), and the mixture was dissolved with stirring, then added dropwise with phosphorus oxychloride (251 mg, 1.64 mmol, 1.5 eq), and stirred at room temperature for 30 min; after the reaction was completed as monitored by TLC, the reaction was quenched with water (20 mL), the reaction solution was extracted with EA (50 mL), the organic phase was concentrated under reduced pressure, and the crude product was purified by silica gel column chromatography (DCM:MeOH = 100:1-50:1) to give the product (230 mg, 32.0% yield).

### Step 2: Synthesis of N-(6-((2-amino-3-chloropyridin-4-yl)oxy)pyridin-3-yl)-5-(4-fluorophenyl)-1-i sopropyl-4-oxo-1,4-dihydropyridazine-3-carboxamide

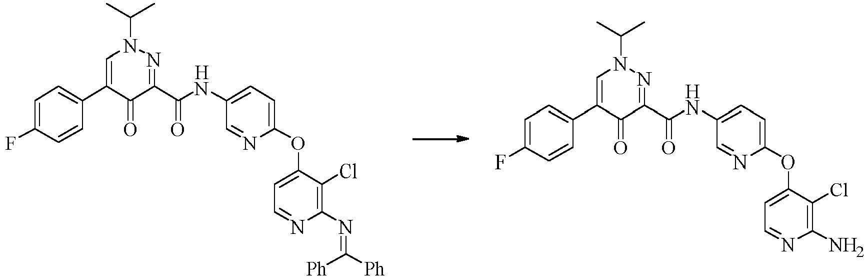

*N*-(6-((3-chloro-2-((diphenylmethylene)amino)pyridin-4-yl)oxy)pyridin-3 -yl)-5-(4-fluorophenyl)-1-isopropyl-4-oxo-1,4-dihydropyridazine-3-carboxami de (230 mg, 0.349 mmol, 1.0 eq) was added to methanol (4 mL) and water (0.8 mL), and the mixture was added with concentrated hydrochloric acid (0.2 mL) and stirred at room temperature for 30 min; after the reaction was completed as monitored by TLC, the system was cooled to room temperature, added with a saturated aqueous NaHCO₃ solution to adjust the pH value to 7-8, and extracted with EA (20 mL × 2), the organic phase was concentrated under reduced pressure, and the crude product was purified by silica gel column chromatography (DCM:MeOH = 60:1-30:1) to give the product (92 mg, yield: 53.3%).

¹H NMR (300 MHz, CDCl₃) δ(ppm): 12.87 (s, 1H), 8.56-8.55 (d, *J* = 2.4 Hz, 1H), 8.43-8.39 (m, 1H), 8.11 (s, 1H), 7.93-7.91 (d, *J* = 5.7 Hz, 1H), 7.71-7.67 (m, 2H), 7.23-7.17 (t, *J=* 9.0 Hz, 2H), 7.08-7.05 (d, *J* = 8.7 Hz, 1H), 6.45-6.43 (d, *J* = 5.7 Hz, 1H), 5.01 (s, 2H), 4.75-4.68 (m, 1H), 1.67 (s, 3H), 1.65 (s, 3H).

Molecular formula: C₂₄H₂₀ClFN₆O₃; molecular weight: 494.91; LC-MS(Pos, *m*/*z*)=495.15[M+H]⁺.

### Example 13: Synthesis of N-(2-((2-amino-3-chloropyridin-4-yl)oxy)pyrimidin-5-yl)-5-(4-fluorophenyl)-1-isopropyl-4-oxo-1,4-dihydropyridazine-3-carboxamide (Compound 16)

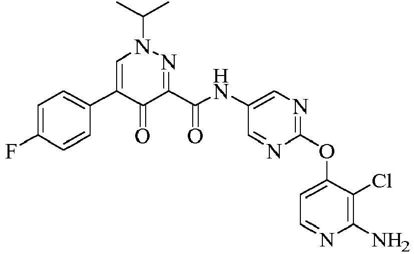

### Step 1: Synthesis of N-(2-((3-chloro-2-((4-methoxybenzyl)amino)pyridin-4-yl)oxy)pyrimidin-5-yl) -5-4-fluorohenl-1-isorol-4-oxo-1,4-dihdroridazine-3-carboxamide

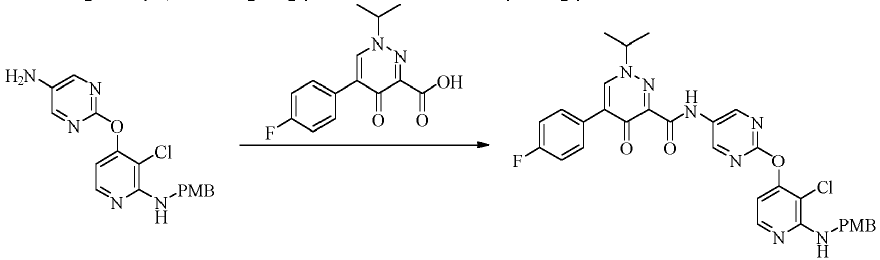

5-(4-fluorophenyl)-1-isopropyl-4-oxo-1,4-dihydropyridazine-3-carboxyli c acid (309.5 mg, 1.12 mmol, 1.0 eq) and 2-((3-chloro-2-((4-methoxybenzyl)amino)pyridin-4-yl)oxy)pyrimidin-5-amine (380.7 mg, 1.06 mmol, 0.95 eq) were added to pyridine (6 mL), and the mixture was dissolved with stirring, then added dropwise with 5 drops of phosphorus oxychloride and stirred at room temperature for 30 min; after the reaction was completed as monitored by TLC, the reaction was quenched with water (10 mL), the reaction solution was extracted with EA (20 mL), the organic phase was washed with saturated brine, dried over anhydrous sodium sulfate and filtered, the filtrate was concentrated under reduced pressure, and the crude product was purified by silica gel column chromatography (DCM:MeOH = 100:1-90:1) to give the product (155.0 mg, 22.4% yield).

### Step 2: Synthesis of N-(2-((2-amino-3-chloropyridin-4-yl)oxy)pyrimidin-5-yl)-5-(4-fluorophenyl)-1-isopropyl-4-oxo-1,4-dihydropyridazine-3-carboxamide

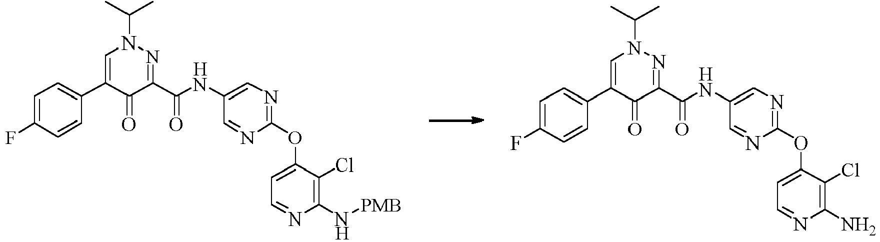

*N*-(2-((3-chloro-2-((4-methoxybenzyl)amino)pyridin-4-yl)oxy)pyrimidin-5-yl)-5-(4-fluorophenyl)-1-isopropyl-4-oxo-1,4-dihydropyridazine-3-carboxa mide (155.0 mg, 0.25 mmol, 1.0 eq) was dissolved in a mixed solution of trifluoroacetic acid (4 mL) and dichloromethane (1 mL), and the mixture was stirred and reacted at 60 °C for 4 h; after the reaction was completed as monitored by TLC, the system was cooled to room temperature, added with a saturated aqueous Na₂CO₃ solution to adjust the pH value to 7-8, and extracted with DCM (10 mL), the organic phase was dried over anhydrous sodium sulfate, filtered and concentrated, and the crude product was purified by preparative thin-layer chromatography (DCM:MeOH = 15:1) to give the product (100.0 mg, yield: 80.1%).

¹H NMR (300 MHz, DMSO-*d₆*) δ(ppm): 12.48 (s, 1H), 9.04 (s, 2H), 8.93 (s, 1H), 7.98-7.92 (m, 3H), 7.37-7.31 (t, *J* = 9.0 Hz, 2H), 6.59-6.57 (d, *J* = 5.4 Hz, 1H), 6.50 (s, 2H), 4.79-4.70 (m, 1H), 1.52-1.50 (s, 6H).

Molecular formula: C₂₃H₁₉ClFN₇O₃; molecular weight: 495.90; LC-MS(Pos, *m*/*z*)=496.15[M+H]⁺.

### Example 14: Synthesis of N-(2-((2-amino-3-chloropyridin-4-yl)oxy)pyrimidin-5-yl)-1-cyclopropyl-5-(4-fluorophenyl)-4-oxo-1,4-dihydropyridazine-3-carboxamide (Compound 20)

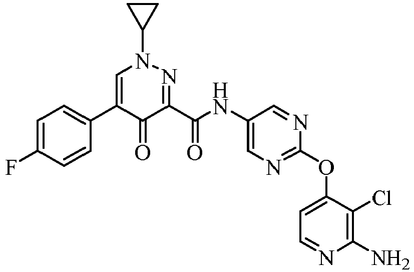

### Step 1: Synthesis of 2-((2,3-dichloropyridin-4-yl)oxy)-5-nitropyrimidine

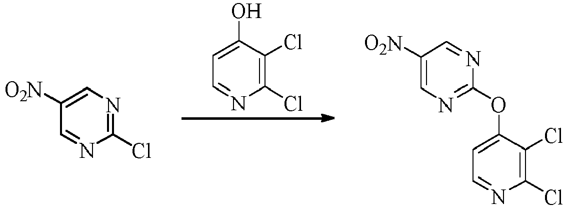

2-chloro-5-nitropyrimidine (11.000 g, 67.08 mmol, 1.0 eq), 2,3-dichloro-4-hydroxylpyridine (10.703 g, 67.09 mmol, 1.0 eq) and triethylamine (8.140 g, 80.44 mmol, 1.2 eq) were added to DMSO (60 mL) in an ice bath, and the mixture was heated to room temperature naturally and stirred overnight; after the reaction was completed as monitored by TLC, the reaction solution was added with ethyl acetate (300 mL), and washed with saturated brine (50 mL × 4), the organic phase was dried over anhydrous sodium sulfate, and filtered under vacuum, the filtrate was concentrated under reduced pressure, and the crude product was slurried with ethyl acetate (50 mL) and filtered under vacuum to give the product (17.85 g, yield: 92.7%).

### Step 2: Synthesis of 2-((2,3-dichloropyridin-4-yl)oxy)pyrimidin-5-amine

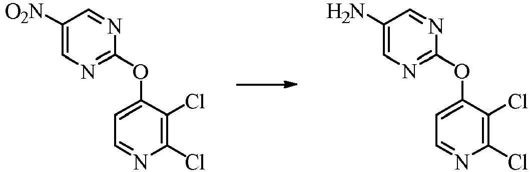

2-((2,3-dichloropyridin-4-yl)oxy)-5-nitropyrimidine (11.050 g, 38.49 mmol, 1.0 eq), reduced iron powder (12.930 g, 230.96 mmol, 6.0 eq) and ammonium chloride (24.750 g, 461.92 mmol, 12.0 eq) were added to a mixed solution of ethanol (100 mL) and water (50 mL), and the mixture was stirred and reacted at 80 °C for 2 h; after the reaction was completed as monitored by TLC, the reaction solution was filtered under vacuum through celite, the filtrate was concentrated under reduced pressure, and the concentrated solution was added with a saturated aqueous NaHCO₃ solution until no bubbles were generated; ethyl acetate (80 mL) was added, the organic phase was washed with saturated brine (40 mL), dried over anhydrous sodium sulfate, and filtered under vacuum, the filtrate was concentrated under reduced pressure, and the crude product was purified by silica gel column chromatography (DCM:MeOH = 50:1) to give the product (5.068 g, yield: 51.2%).

### Step 3: Synthesis of 2-((3-chloro-2-((4-methoxybenzyl)amino)pyridin-4-yl)oxy)pyrimidin-5-amine

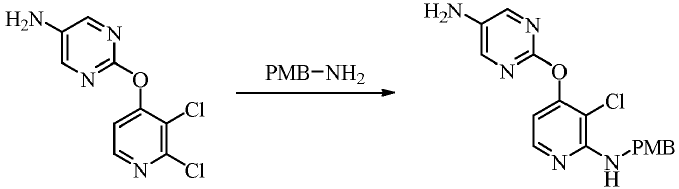

2-((2,3-dichloropyridin-4-yl)oxy)pyrimidin-5-amine (2.800 g, 10.89 mmol, 1.0 eq) was dissolved in *p*-methoxybenzylamine (15 mL), and the mixture was reacted at 150 °C for 1.5 h under microwave; after the reaction was completed as monitored by TLC, the reaction solution was added with ethyl acetate (50 mL), and sequentially washed with 0.1 mol/L dilute hydrochloric acid (50 mL × 4) and saturated brine (50 mL × 2), the organic phase was dried over anhydrous sodium sulfate, and filtered under vacuum, the filtrate was concentrated under reduced pressure, and the crude product was purified by silica gel column chromatography (PE:EA = 1:4) to give the product (1.8 g, yield: 46.2%).

### Step 4: Synthesis of N-(2-((3-chloro-2-((4-methoxybenzyl)amino)pyridin-4-yl)oxy)pyrimidin-5-yl) -1-cyclopropyl-5-(4-fluorophenyl)-4-oxo-1,4-dihydropyridazine-3-carboxamid e

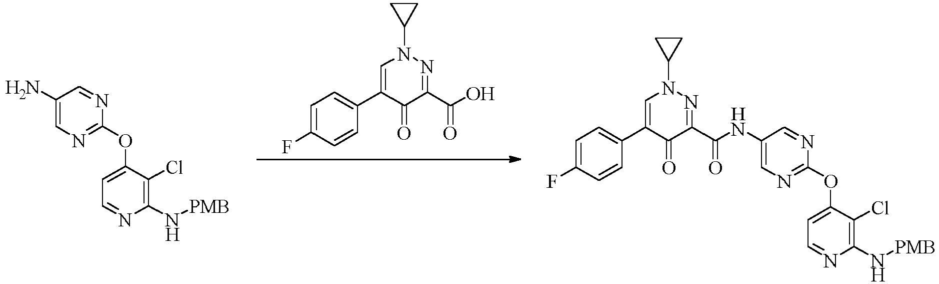

2-((3-chloro-2-((4-methoxybenzyl)amino)pyridin-4-yl)oxy)pyrimidin-5-a mine (357.8 mg, 1.00 mmol, 1.0 eq) and 1-cyclopropyl-5-(4-fluorophenyl)-4-oxo-1,4-dihydropyridazine-3-carboxylic acid (274.3 mg, 1.00 mmol, 1.0 eq) were dissolved in pyridine (10 mL), and the mixture was added with POCl₃ (230.0 mg, 1.50 mmol, 1.5 eq) and stirred and reacted at room temperature for 0.5 h, after the reaction was completed as monitored by TLC, the reaction solution was poured into water (30 mL), and extracted with ethyl acetate (30 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 20:1) to give the product (223.0 mg, yield: 36.3%).

### Step 5: Synthesis of N-(2-((2-amino-3-chloropyridin-4-yl)oxy)pyrimidin-5-yl)-1-cyclopropyl-5-(4-fluorophenyl)-4-oxo-1,4-dihydropyridazine-3-carboxamide

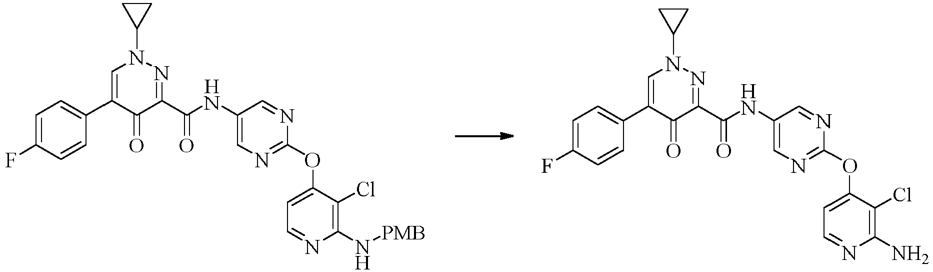

*N*-(2-((3-chloro-2-((4-methoxybenzyl)amino)pyridin-4-yl)oxy)pyrimidin-5-yl)-1-cyclopropyl-5-(4-fluorophenyl)-4-oxo-1,4-dihydropyridazine-3-carbox amide (223.0 mg, 0.36 mmol, 1.0 eq) was dissolved in a mixed solution of trifluoroacetic acid (20 mL) and dichloromethane (5 mL), and the mixture was stirred and reacted at 60 °C for 4.5 h; after the reaction was completed as monitored by TLC, the reaction solution was poured into a saturated aqueous NaHCO₃ solution (30 mL), and extracted with dichloromethane (30 mL × 2), the organic phases were combined, washed with saturated brine (30 mL), dried over anhydrous sodium sulfate, and filtered under vacuum, the filtrate was concentrated under reduced pressure, and the crude product was purified by preparative thin-layer chromatography (DCM: MeOH = 15:1) to give the product (94.0 mg, yield: 52.4%).

¹H NMR (400 MHz, DMSO-*d₆*) δ(ppm): 12.36 (s, 1H), 9.03 (s, 2H), 8.94 (s, 1H), 7.96-7.92 (m, 3H), 7.36-7.31 (t, *J* = 8.9 Hz, 2H), 6.58-6.57 (d, *J* = 5.5 Hz, 1H), 6.50 (s, 2H), 4.14-4.09 (m, 1H), 1.33-1.29 (m, 2H), 1.14-1.10 (m, 2H).

Molecular formula: C₂₃H₁₇ClFN₇O₃; molecular weight: 493.88; LC-MS(Pos, *m*/*z*)=494.13[M+H]⁺.

### Example 15: Synthesis of N-(6-((2-amino-3-chloropyridin-4-yl)oxy)pyridin-3-yl)-6-(4-fluorophenyl)-2-i sopropyl-5-oxo-2,5-dihydropyridazine-4-carboxamide (Compound 23)

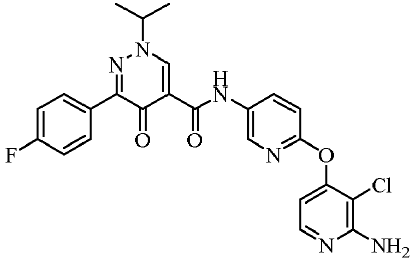

### Step 1: Synthesis of N-(6-((3-chloro-2-((diphenylmethylene)amino)pyridin-4-yl)oxy)pyridin-3-yl-6 -(4-fluorophenyl)-2-isopropyl-5-oxo-2,5-dihydropyridazine-4-carboxamide

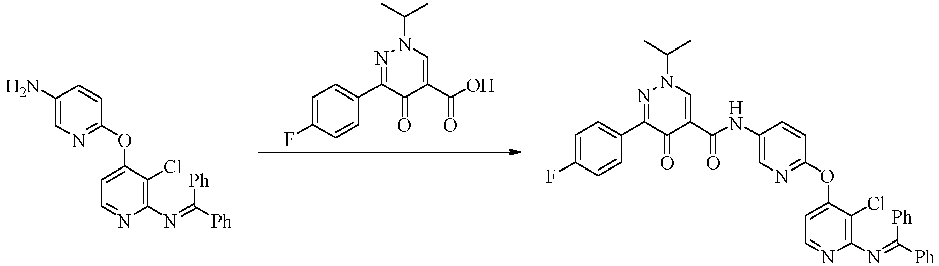

6-((3-chloro-2-((diphenylmethylene)amino)pyridin-4-yl)oxy)pyridin-3-a mine (200.0 mg, 0.50 mmol, 1.0 eq) and 6-(4-fluorophenyl)-2-isopropyl-5-oxo-2,5-dihydropyridazine-4-carboxylic acid (138.0 mg, 0.50 mmol, 1.0 eq) were dissolved in pyridine (5 mL), and the mixture was added with POCl₃ (115.0 mg, 0.75 mmol, 1.5 eq) and stirred and reacted at room temperature for 0.5 h; after the reaction was completed as monitored by TLC, the reaction solution was poured into water (15 mL), and extracted with ethyl acetate (15 mL × 2), the organic phases were combined, washed with saturated brine (15 mL), dried over anhydrous sodium sulfate and filtered under vacuum, the filtrate was concentrated under reduced pressure, and the crude product was purified by preparative thin-layer chromatography (DCM: MeOH = 20:1) to give the product (148.0 mg, yield: 45.0%).

### Step 2: Synthesis of N-(6-((2-amino-3-chloropyridin-4-yl)oxy)pyridin-3-yl)-6-(4-fluorophenyl)-2-i sopropyl-5-oxo-2,5-dihydropyridazine-4-carboxamide

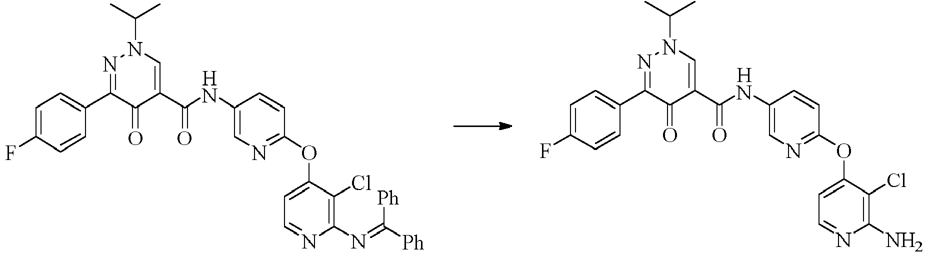

*N*-(6-((3-chloro-2-((diphenylmethylene)amino)pyridin-4-yl)oxy)pyridin-3-yl)-6-(4-fluorophenyl)-2-isopropyl-5-oxo-2,5-dihydropyridazine-4-carboxa mide (148.0 mg, 0.22 mmol, 1.0 eq) was dissolved in methanol (5 mL), and the mixture was added dropwise with concentrated hydrochloric acid (0.1 mL) with stirring and stirred and reacted at room temperature for 0.5 h; after the reaction was completed as monitored by TLC, the reaction solution was poured into a saturated aqueous NaHCO₃ solution (10 mL), and extracted with dichloromethane (15 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 10:1) to give the product (89.0 mg, yield: 80.0%).

¹H NMR (400 MHz, DMSO-*d₆*) δ(ppm): 12.26 (s, 1H), 9.21 (s, 1H), 8.55-8.54 (d, *J* = 2.6 Hz, 1H), 8.33-8.30 (m, 1H), 8.21-8.17 (m, 2H), 7.87-7.86 (d, *J* = 5.5 Hz, 1H), 7.38-7.33 (m, 2H), 7.22-7.20 (d, *J* = 8.8 Hz, 1H), 6.42 (s, 2H), 6.37-6.35 (d, *J* = 5.5 Hz, 1H), 4.90-4.83 (m, 1H), 1.51-1.50 (d, *J* = 6.5 Hz, 6H).

Molecular formula: C₂₄H₂₀ClFN₆O₃; molecular weight: 494.91; LC-MS(Pos, *m*/*z*)=495.15[M+H]⁺.

### Example 16: Synthesis of N-(6-((2-amino-3-chloropyridin-4-yl)oxy)pyridin-3-yl)-2-cyclopropyl-6-(4-flu orophenyl)-5-oxo-2,5-dihydropyridazine-4-carboxamide (Compound 38)

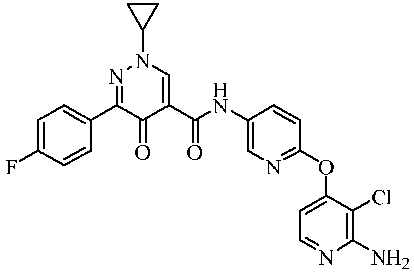

### Step 1: Synthesis of N-(6-((3-chloro-2-((diphenylmethylene)amino)pyridin-4-yl)oxy)pyridin-3-yl)-2-cyclopropyl-6-(4-fluorophenyl)-5-oxo-2,5-dihydropyridazine-4-carboxamid e

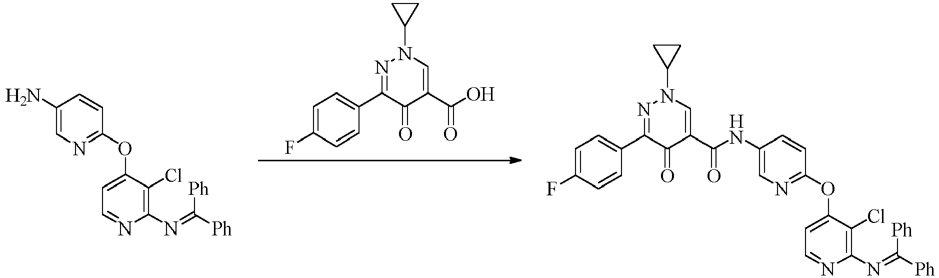

6-((3-chloro-2-((diphenylmethylene)amino)pyridin-4-yl)oxy)pyridin-3-a mine (200.0 mg, 0.50 mmol, 1.0 eq) and 2-cyclopropyl-6-(4-fluorophenyl)-5-oxo-2,5-dihydropyridazine-4-carboxylic acid (137.0 mg, 0.50 mmol, 1.0 eq) were dissolved in pyridine (5 mL), and the mixture was added with POCl₃ (115.0 mg, 0.75 mmol, 1.5 eq) and stirred at room temperature for 0.5 h; after the reaction was completed as monitored by TLC, the reaction solution was poured into water (15 mL), and extracted with ethyl acetate (15 mL × 2), the organic phases were combined, washed with saturated brine (15 mL), dried over anhydrous sodium sulfate and filtered under vacuum, the filtrate was concentrated under reduced pressure, and the crude product was purified by preparative thin-layer chromatography (DCM: MeOH = 20:1) to give the product (80.0 mg, yield: 24.4%).

### Step 2: Synthesis of N-(6-((2-amino-3-chloropyridin-4-yl)oxy)pyridin-3-yl)-2-cyclopropyl-6-(4-flu orophenyl)-5-oxo-2,5-dihydropyridazine-4-carboxamide

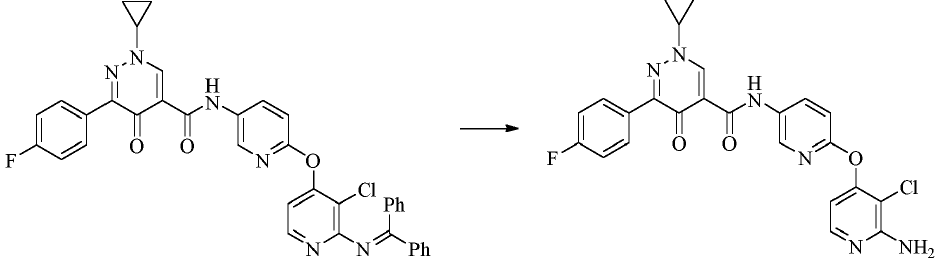

*N*-(6-((3-chloro-2-((diphenylmethylene)amino)pyridin-4-yl)oxy)pyridin-3-yl)-2-cyclopropyl-6-(4-fluorophenyl)-5-oxo-2,5-dihydropyridazine-4-carbox amide (80.0 mg, 0.12 mmol, 1.0 eq) was dissolved in methanol (5 mL), and the mixture was added dropwise with concentrated hydrochloric acid (0.1 mL) with stirring, and stirred and reacted at room temperature for 0.5 h; after the reaction was completed as monitored by TLC, the reaction solution was poured into a saturated aqueous NaHCO₃ solution (10 mL), and extracted with dichloromethane (15 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 10:1) to give the product (48.0 mg, yield: 80.0%).

¹H NMR (400 MHz, DMSO-*d₆*) δ(ppm): 12.18 (s, 1H), 9.21 (s, 1H), 8.54-8.53 (d, *J* = 2.6 Hz, 1H), 8.33-8.30 (m, 1H), 8.15-8.11 (m, 2H), 7.87-7.86 (d, *J* = 5.5 Hz, 1H), 7.37-7.32 (m, 2H), 7.22-7.20 (d, *J* = 8.8 Hz, 1H), 6.42 (s, 2H), 6.37-6.35 (d, *J* = 5.5 Hz, 1H), 4.27-4.21(m, 1H), 1.32-1.30 (m, 2H), 1.11-1.08 (m, 2H).

Molecular formula: C₂₄H₁₈ClFN₆O₃; molecular weight: 492.90; LC-MS(Pos, *m*/*z*)=493.15[M+H]⁺.

### Example 17: Synthesis of 1-allyl-N-(6-((2-amino-3-chloropyridin-4-yl)oxy)pyridin-3-yl)-5-(4-fluorophe nyl)-4-oxo-1,4-dihydropyridazine-3-carboxamide (Compound 39)

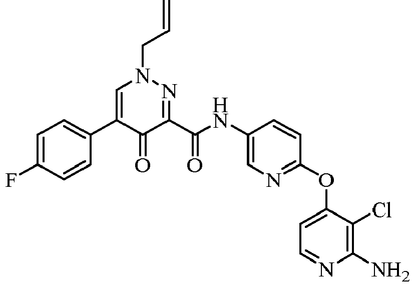

### Step 1: Synthesis of 1-allyl-N-(6-((2-amino-3-chloropyridin-4-yl)oxy)pyridin-3-yl)-5-(4-fluorophe nyl)-4-oxo-1,4-dihydropyridazine-3-carboxamide

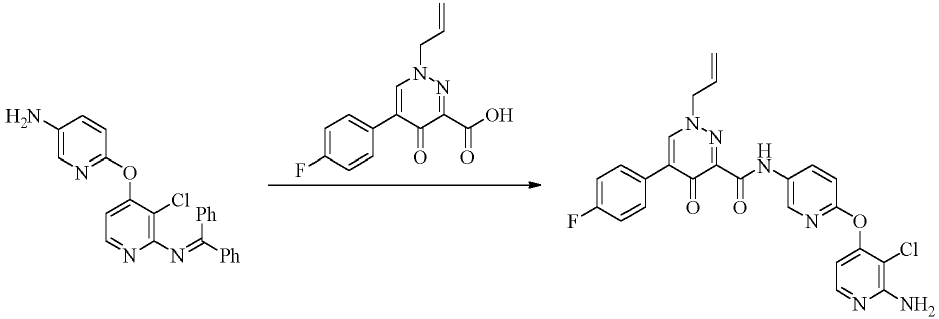

6-((3-chloro-2-((diphenylmethylene)amino)pyridin-4-yl)oxy)pyridin-3-a mine (400.8 mg, 1.00 mmol, 1.0 eq) and 1-allyl-5-(4-fluorophenyl)-4-oxo-1,4-dihydropyridazine-3-carboxylic acid (274.3 mg, 1.00 mmol, 1.0 eq) were dissolved in pyridine (10 mL), and the mixture was added with POCl₃ (230.0 mg, 1.50 mmol, 1.5 eq) and stirred and reacted at room temperature for 0.5 h; after the reaction was completed as monitored by TLC, the reaction solution was poured into 0.1 mol/L dilute hydrochloric acid (30 mL), and extracted with ethyl acetate (30 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 15:1) to give the product (132.0 mg, yield: 26.8%).

¹H NMR (400 MHz, DMSO-*d₆*) δ(ppm): 12.21 (s, 1H), 8.90 (s, 1H), 8.52-8.51 (d, *J* = 2.6 Hz, 1H), 8.31-8.28 (dd, *J* = 2.7, 6.1 Hz, 1H), 7.94-7.91 (dd, *J* = 5.6, 3.3 Hz, 2H), 7.87-7.86 (d, *J* = 5.5 Hz, 1H), 7.36-7.32 (t, *J* = 8.9 Hz, 2H), 7.22-7.20 (d, *J* = 8.8 Hz, 1H), 6.44 (s, 2H), 6.37-6.36 (d, *J* = 5.5 Hz, 1H), 6.18-6.11 (m, 1H), 5.38-5.37 (m, 1H), 5.34-5.33 (t, 1H), 4.97-4.95 (d, *J* = 5.9 Hz, 2H).

Molecular formula: C₂₄H₁₈ClFN₆O₃; molecular weight: 492.90; LC-MS(Pos, *m*/*z*)=493.32[M+H]⁺.

### Example 18: Synthesis of 1-allyl-N-(2-((2-amino-3-chloropyridin-4-yl)oxy)pyrimidin-5-yl)-5-(4-fluorop henyl)-4-oxo-1,4-dihydropyridazine-3-carboxamide (Compound 40

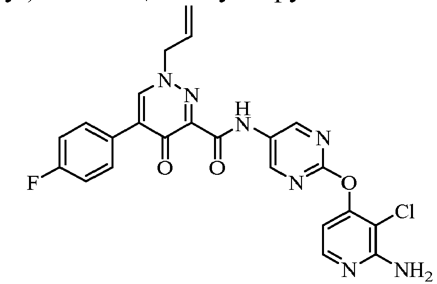

### Step 1: Synthesis of 1-allyl-N-(2-((3-chloro-2-((4-methoxybenzyl)amino)pyridin-4-yl)oxy)pyrimidi n-5-yl)-5-(4-fluorophenyl)-4-oxo-1,4-dihydropyridazine-3-carboxamide

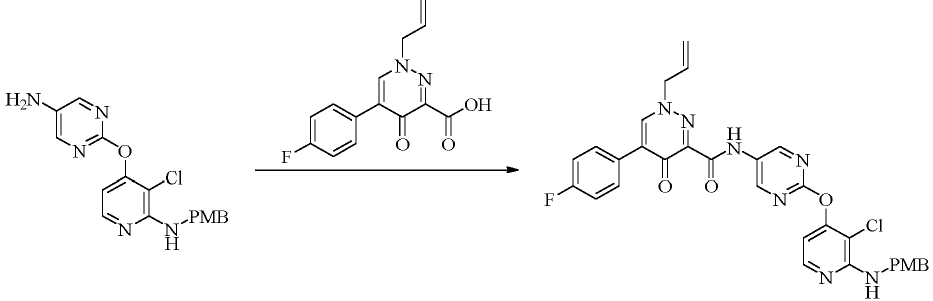

2-((3-chloro-2-((4-methoxybenzyl)amino)pyridin-4-yl)oxy)pyrimidin-5-a mine (357.8 mg, 1.00 mmol, 1.0 eq) and 1-allyl-5-(4-fluorophenyl)-4-oxo-1,4-dihydropyridazine-3-carboxylic acid (274.3 mg, 1.00 mmol, 1.0 eq) were dissolved in pyridine (10 mL), and the mixture was added with POCl₃ (230.0 mg, 1.50 mmol, 1.5 eq) and stirred and reacted at room temperature for 0.5 h; after the reaction was completed as monitored by TLC, the reaction solution was poured into water (30 mL), and extracted with ethyl acetate (30 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 20:1) to give the product (208.0 mg, yield: 33.9%).

### Step 2: Synthesis of 1-allyl-N-(2-((2-amino-3-chloropyridin-4-yl)oxy)pyrimidin-5-yl)-5-(4-fluorop henyl)-4-oxo-1,4-dihydropyridazine-3-carboxamide

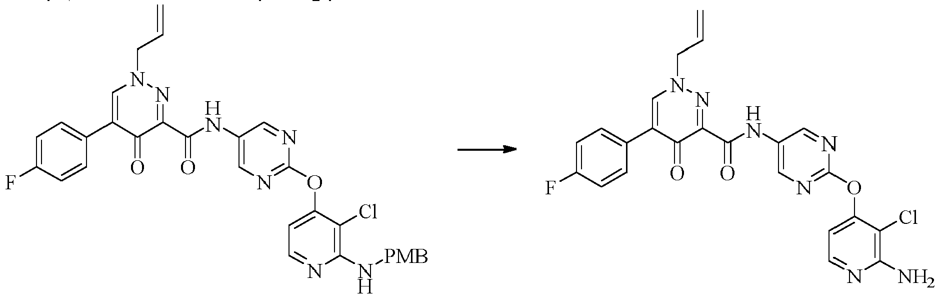

1-allyl-*N*-(2-((3-chloro-2-((4-methoxybenzyl)amino)pyridin-4-yl)oxy)pyr imidin-5-yl)-5-(4-fluorophenyl)-4-oxo-1,4-dihydropyridazine-3-carboxamide (208.0 mg, 0.34 mmol, 1.0 eq) was dissolved in a mixed solution of trifluoroacetic acid (20 mL) and dichloromethane (5 mL), and the mixture was stirred and reacted at 60 °C for 4.5 h; after the reaction was completed as monitored by TLC, the reaction solution was poured into a saturated aqueous NaHCO₃ solution (30 mL), added dropwise with a saturated aqueous NaHCO₃ solution until no bubbles were generated, and extracted with dichloromethane (30 mL × 2), the organic phases were combined, washed with saturated brine (30 mL), dried over anhydrous sodium sulfate, and filtered under vacuum, the filtrate was concentrated under reduced pressure, and the crude product was purified by preparative thin-layer chromatography (DCM:MeOH = 15:1) to give the product (110.0 mg, yield: 65.8%).

¹H NMR (400 MHz, DMSO-*d₆*) δ(ppm): 12.35 (s, 1H), 9.03 (s, 2H), 8.92 (s, 1H), 7.95-7.91 (m, 3H), 7.37-7.32 (t, *J* = 8.9 Hz, 2H), 6.59-6.57 (d, *J* = 5.5 Hz, 1H), 6.50 (s, 2H), 6.19-6.10 (m, 1H), 5.39-5.37 (m, 1H), 5.35-5.34 (t, *J* = 1.4 Hz, 1H), 4.98-4.96 (d, *J* = 5.9 Hz, 2H).

Molecular formula: C₂₃H₁₇ClFN₇O₃; molecular weight: 493.88; LC-MS(Pos, *m*/*z*)=494.28[M+H]⁺.

### Example 19: Synthesis of N-(2-((2-amino-3-chloropyridin-4-yl)oxy)pyrimidin-5-yl)-2-cyclopropyl-6-(4-fluorophenyl)-5-oxo-2,5-dihydropyridazine-4-carboxamide (Compound 57)

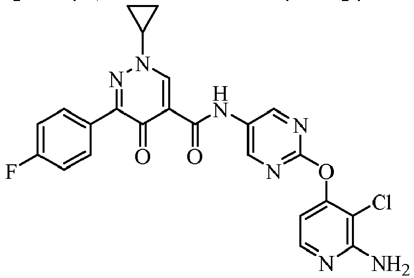

### Step 1: Synthesis of N-(2-((3-chloro-2-((4-methoxybenzyl)amino)pyridin-4-yl)oxy)pyrimidin-5-yl) -2-cyclopropyl-6-(4-fluorophenyl)-5-oxo-2,5-dihydropyridazine-4-carboxamid e

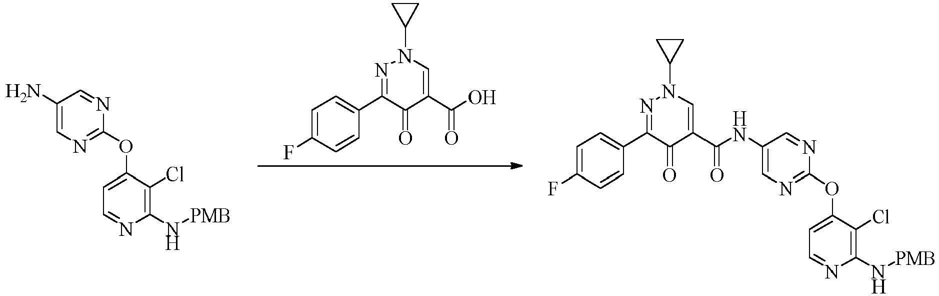

2-((3-chloro-2-((4-methoxybenzyl)amino)pyridin-4-yl)oxy)pyrimidin-5-a mine (267.5 mg, 0.75 mmol, 1.0 eq) and 2-cyclopropyl-6-(4-fluorophenyl)-5-oxo-2,5-dihydropyridazine-4-carboxylic acid (205.0 mg, 0.75 mmol, 1.0 eq) were dissolved in pyridine (10 mL), and the mixture was added with POCl₃ (171.9 mg, 1.12 mmol, 1.5 eq) and stirred and reacted at room temperature for 0.5 h; after the reaction was completed as monitored by TLC, the reaction solution was poured into water (30 mL), and extracted with ethyl acetate (30 mL × 2), the organic phases were combined, washed with saturated brine (15 mL), dried over anhydrous sodium sulfate, and filtered under vacuum, the filtrate was concentrated under reduced pressure, and the crude product was purified by preparative thin-layer chromatography (DCM:MeOH = 20:1) to give the product (119.0 mg, yield: 25.9%).

### Step 2: Synthesis of N-(2-((2-amino-3-chloropyridin-4-yl)oxy)pyrimidin-5-yl)-2-cyclopropyl-6-(4-fluorophenyl)-5-oxo-2,5-dihydropyridazine-3 -carboxamide

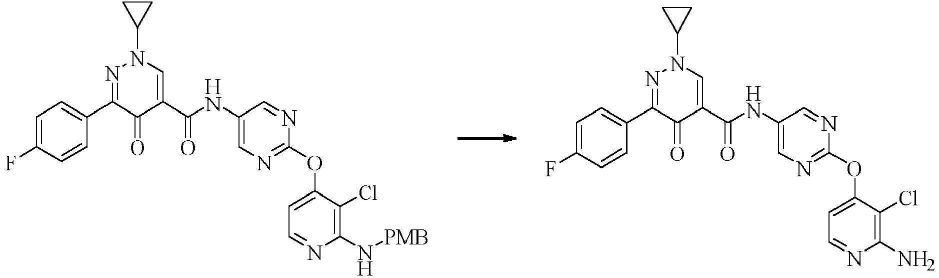

*N*-(2-((3-chloro-2-((4-methoxybenzyl)amino)pyridin-4-yl)oxy)pyrimidin-5-yl)-2-cyclopropyl-6-(4-fluorophenyl)-5-oxo-2,5-dihydropyridazine-4-carbox amide (119.0 mg, 0.19 mmol, 1.0 eq) was dissolved in a mixed solution of trifluoroacetic acid (20 mL) and dichloromethane (5 mL), and the mixture was stirred and reacted at 60 °C for 4.5 h; after the reaction was completed as monitored by TLC, the reaction solution was poured into a saturated aqueous NaHCO₃ solution (30 mL), added dropwise with a saturated aqueous NaHCO₃ solution until no bubbles were generated, and extracted with dichloromethane (30 mL × 2), the organic phases were combined, washed with saturated brine (30 mL), dried over anhydrous sodium sulfate, and filtered under vacuum, the filtrate was concentrated under reduced pressure, and the crude product was purified by preparative thin-layer chromatography (DCM:MeOH = 10:1) to give the product (63.0 mg, yield: 65.8%).

¹H NMR (400 MHz, DMSO-*d₆*) δ(ppm): 12.16 (s, 1H), 9.22 (s, 1H), 9.05 (s, 2H), 8.15-8.12 (dd, *J* = 5.7, 3.2 Hz, 2H), 7.94-7.92 (d, *J* = 5.4 Hz, 1H), 7.37-7.32 (t, *J* = 8.9 Hz, 2H), 6.58-6.56 (d, *J* = 5.4 Hz, 1H), 6.49 (s, 2H), 4.27-4.21 (m, 1H), 1.34-1.30 (m, 2H), 1.12-1.09 (m, 2H).

Molecular formula: C₂₃H₁₇ClFN₇O₃; molecular weight: 493.88; LC-MS(Pos, *m*/*z*)=494.24[M+H]⁺.

### Experimental Example 1: Assay on Inhibitory Activity of Compounds of the Present Invention on Cell pAxl

H1299 is a non-small cell lung cancer cell.

Test samples: the compounds of the present invention, having structures shown above.

Test instruments: protein electrophoresis apparatus (made by Bio Rad), membrane transfer apparatus (made by Bio Rad), exposure apparatus (made by Tanon), and CO₂ cell incubator (made by Thermo).

Test method:
H1299 cells were inoculated into a 6-well plate (containing 10% FBS 1640 medium, 5 × 10⁵ cells per well) and adherently cultivated at 37 °C and 5% CO₂ for 18 h. The cells were starved overnight, and compounds with different concentrations were added. The compounds had final concentrations of 1.1 nM, 3.3 nM, 10 nM and 30 nM, with DMSO having a final concentration of 1 ‰. There was a medium containing 1‰ DMSO in the negative control well. The cells were incubated at 37 °C and 5% CO₂ for 60 min, and then hGAS6 (R&D, final concentration: 200 ng/mL) was added to each well. The cells were further incubated for 60 min, and then the total protein of the cells was extracted for Western Blot experiment. IC₅₀ values were calculated with GraphPad 5.0 software, and the inhibitory activities of the compounds on cell pAxl were detected.

The test results are shown in Table 1 below.

**Table 1. Inhibitory Activity of Compounds of the Present Invention on Cell pAxl - IC₅₀ (nM)**

| Compound | IC₅₀(nM) |
|---|---|
| Compound 1 | 1.1 |
| Compound 11 | <1.1 |
| Compound 12 | 3.3 |
| Compound 13 | 1.1-3.3 |
| Compound 16 | 10 |
| Compound 17 | <1.1 |
| Compound 18 | 3.3 |
| Compound 19 | 3.3-10 |
| Compound 20 | 10-30 |
| Compound 33 | 3.3-10 |
| Compound 34 | 10 |
| Compound 35 | <1.1 |
| Compound 36 hydrochloride | 10 |
| Compound 37 | 1.1 |
| Compound 39 | 1.1-3.3 |
| Compound 40 | 10 |

It can be seen from the experimental results in Table 1 that the compounds of the present invention have significant inhibitory effects on H1299 cell pAxl. It demonstrates that the compounds of the present invention can effectively inhibit the activity of Axl at the cellular level and are prominent Axl inhibitors.

### Experimental Example 2: Assay on Inhibitory Activity of Compounds of the Present Invention on Cell pMer

H1299 is a non-small cell lung cancer cell.

Test samples: the compounds of the present invention, having structures shown above.

Test instruments: protein electrophoresis apparatus (made by Bio Rad), membrane transfer apparatus (made by Bio Rad), exposure apparatus (made by Tanon), and CO₂ cell incubator (made by Thermo).

Test method:
H1299 cells were inoculated into a 6-well plate (containing 10% FBS 1640 medium, 5 × 10⁵ cells per well) and adherently cultivated at 37 °C and 5% CO₂ for 18 h. The cells were starved overnight, and compounds with different concentrations were added. The compounds had final concentrations of 0.37 nM, 1.1 nM, 3.3 nM and 10 nM, with DMSO having a final concentration of 1 ‰. There was a medium containing 1‰ DMSO in the negative control well. The cells were incubated at 37 °C and 5% CO₂ for 60 min, and then Human MerMab (R&D, final concentration: 200 ng/mL) was added to each well. The cells were further incubated for 60 min, and then the total protein of the cells was extracted for Western Blot experiment. Gray analysis was performed on the results with Image J software, and IC₅₀ values were calculated with GraphPad 5.0 software. The inhibitory activities of the compounds on cell pMer were detected.

The test results are shown in Table 2 below.

**Table 2. Inhibitory Activity of Compounds of the Present Invention on Cell pMer - IC₅₀ (nM)**

| Compound | IC₅₀(nM) |
|---|---|
| Compound 11 | 1.1 |
| Compound 12 | 1.1 |
| Compound 13 | 3.3-10 |
| Compound 17 | <1.1 |
| Compound 18 | 1.1 |
| Compound 19 | 10 |
| Compound 33 | 3.3 |
| Compound 35 | 0.37 |
| Compound 37 | 3.3 |
| Compound 39 | 3.3-10 |

It can be seen from the experimental results in Table 2 that the compounds of the present invention have significant inhibitory effects on H1299 cell pMer. It demonstrates that the compounds of the present invention can effectively inhibit the activity of Mer at the cellular level and are prominent Mer inhibitors.

### Experimental Example 3: Assay on Enzymatic Activity of Compounds of the Present Invention

Test samples: the compounds of the present invention, having structures and preparation methods shown above.

Test method:
(1) Preparation of compound stock solutions
   Compounds were each dissolved in 100% DMSO to prepare stock solutions with a maximum concentration of 500 µM.
(2) Preparation of compound working solutions
   The compound stock solutions were diluted to final concentrations of 500 µM, 150 µM, 50 µM, 15 µM, 5 µM, 1.5 µM, 0.5 µM, 0.15 µM and 0.05 µM to prepare compound working solutions (50 ×).
(3) Preparation of different enzymatic reaction solutions
   Ron (h)
   Ron (h) enzyme was dissolved in a mixture of 8 mM MOPS (pH 7.0), 0.2 mM EDTA and 250 µM GGMEDIYFEFMGGKKK to prepare an enzyme solution with a final concentration of 40.4 nM. An Mg/ATP mixture of 10 mM magnesium acetate and 10 µM [γ-³³P]-ATP was used to activate the enzymatic reaction.
(4) Enzymatic reaction

The compound working solutions were added to a 384-well plate, with final concentrations of 10000 nM, 3000 nM, 1000 nM, 300 nM, 100 nM, 30 nM, 10 nM, 3 nM and 1 nM, respectively, and then different enzymatic reaction solutions prepared according to the above conditions were added. The reaction solutions were incubated at room temperature for 40 min, and then 0.5% phosphoric acid solutions were added to terminate the reaction. 10 µL of the reaction solution was added dropwise to P30 filter paper which was washed 4 times with a 0.425% phosphoric acid solution and once with methanol and then placed in a scintillation counter for detection. Gray analysis was performed on the results with Image J software, and IC₅₀ values were calculated with GraphPad 5.0 software.

The test results are shown in Table 3 below.

**Table 3. Inhibitory Activity of Compounds of the Present Invention on Ron (h) kinase - IC₅₀ (nM)**

| Test samples | Ron (h) |
|---|---|
| Compound 11 | 2 |
| Compound 12 | 19 |
| Compound 13 | 16 |
| Compound 17 | 7 |
| Compound 18 | 14 |
| Compound 19 | 64 |
| Compound 23 | 92 |
| Compound 33 | 17 |
| Compound 35 | 5 |
| Compound 37 | 12 |
| Compound 39 | 12 |

It can be seen from the experimental results in Table 3 that the compounds of the present invention have good inhibitory activities on Ron (h). Therefore, the compounds of the present invention can be used for the treatment and/or prevention of Ron (h)-mediated diseases.

### Experimental Example 4: Assay on Enzymatic Activity of Compounds of the Present Invention

Test samples: the compounds of the present invention, having structures and preparation methods shown above.

Test method:
(1) Preparation of compound stock solutions
   Compounds were each dissolved in 100% DMSO to prepare stock solutions with a maximum concentration of 500 µM.
(2) Preparation of compound working solutions
   The compound stock solutions were diluted to final concentrations of 500 µM, 150 µM, 50 µM, 15 µM, 5 µM, 1.5 µM, 0.5 µM, 0.15 µM and 0.05 µM to prepare compound working solutions (50 ×).
(3) Preparation of different enzymatic reaction solutions
   a) Axl (h)
      Axl (h) enzyme was dissolved in a mixture of 8 mM MOPS (pH 7.0), 0.2 mM EDTA and 250 µM KKSRGDYMTMQIG to prepare an enzyme solution with a final concentration of 1.7 nM. An Mg/ATP mixture of 10 mM magnesium acetate and 10 µM [γ-³³P]-ATP was used to activate the enzymatic reaction.
   b) Mer (h)
      Mer (h) enzyme was dissolved in a mixture of 8 mM MOPS (pH 7.0), 0.2 mM EDTA, 30 mM NaCl and 250 µM GGMEDIYFEFMGGKKK to prepare an enzyme solution with a final concentration of 3.1 nM. An Mg/ATP mixture of 10 mM magnesium acetate and 10 µM [γ-³³P]-ATP was used to activate the enzymatic reaction.
   c) Tyro-3 (h)
      Tyro3 (h) enzyme was dissolved in a mixture of 8 mM MOPS (pH 7.0), 0.2 mM EDTA, 1 mM MnCl₂ and 250 µM KVEKIGEGTYGVVYK to prepare an enzyme solution with a final concentration of 38 nM. An Mg/ATP mixture of 10 mM magnesium acetate and 10 µM [γ-³³P]-ATP was used to activate the enzymatic reaction.
(4) Enzymatic reaction

The compound working solutions were added to a 384-well plate, with final concentrations of 10000 nM, 3000 nM, 1000 nM, 300 nM, 100 nM, 30 nM, 10 nM, 3 nM and 1 nM, respectively, and then different enzymatic reaction solutions prepared according to the above conditions were added. The reaction solutions were incubated at room temperature for 40 min, and then 0.5% phosphoric acid solutions were added to terminate the reaction. 10 µL of the reaction solution was added dropwise to P30 filter paper which was washed 4 times with a 0.425% phosphoric acid solution and once with methanol and then placed in a scintillation counter for detection. Gray analysis was performed on the results with Image J software, and IC₅₀ values were calculated with GraphPad 5.0 software.

The test results are shown in Table 4 below.

**Table 4. Inhibitory Activity of Compounds of the Present Invention on Different Kinases - IC₅₀ (nM)**

| Compound | Axl (h) | Mer (h) | Tyro-3 (h) |
|---|---|---|---|
| Compound 11 | 6 | 9 | 54 |
| Compound 12 | 23 | 8 | 23 |
| Compound 13 | 9 | 8 | 39 |
| Compound 16 | 16 | - | - |
| Compound 17 | 9 | 2 | 31 |
| Compound 18 | 8 | 6 | 49 |
| Compound 19 | 12 | 18 | 73 |
| Compound 20 | 17 | - | - |
| Compound 23 | 30 | - | - |
| Compound 33 | 12 | 3 | 23 |
| Compound 35 | 4 | 4 | 24 |
| Compound 37 | 9 | 7 | 22 |
| Compound 39 | 12 | 3 | 28 |
| Compound 40 | 13 - | - | - |

| | | | |
|---|---|---|---|
| - means that it is not measured. | | | |

It can be seen from the experimental results in Table 4 that the compounds of the present invention have good inhibitory activities on Axl (h), Mer (h) and Tyro3 (h). Therefore, the compounds of the present invention can be used for the prevention and/or treatment of diseases mediated by Axl (h), Mer (h) and Tyro-3 (h).

### Experimental Example 5: Assay on Enzymatic Activity of Compounds of the Present Invention

Test samples: the compounds of the present invention, having structures and preparation methods shown above.

Test method:
Preparation of compound stock solutions:
   Compounds were each dissolved in 100% DMSO to prepare stock solutions with a maximum concentration of 500 µM.
Preparation of compound working solutions:
   The compound stock solutions were diluted to final concentrations of 500 µM, 150 µM, 50 µM, 15 µM, 5 µM, 1.5 µM, 0.5 µM, 0.15 µM and 0.05 µM to prepare compound working solutions (50 ×).
Preparation of different enzymatic reaction solutions:
   LCK (h)

LCK (h) enzyme was dissolved in a mixture of 50 mM Tris (pH 7.5), 0.1 mM EGTA, 0.1 mM Na₃VO₄ and 250 µM KVEKIGEGTYGVVYK to prepare an enzyme solution with a final concentration of 45 nM. An Mg/ATP mixture of 10 mM magnesium acetate and 10 µM [γ-³³P]-ATP was used to activate the enzymatic reaction.

Enzymatic reaction:
The compound working solutions were added to a 384-well plate, with final concentrations of 10000 nM, 3000 nM, 1000 nM, 300 nM, 100 nM, 30 nM, 10 nM, 3 nM and InM, respectively, and then different enzymatic reaction solutions prepared according to the above conditions were added. The reaction solutions were incubated at room temperature for 40 min, and then 0.5% phosphoric acid solutions were added to terminate the reaction. 10 µL of the reaction solution was added dropwise to P30 filter paper which was washed 4 times with a 0.425% phosphoric acid solution and once with methanol and then placed in a scintillation counter for detection. Gray analysis was performed on the results with Image J software, and IC₅₀ values were calculated with GraphPad 5.0 software.

The test results are shown in Table 5 below.

**Table 5. Inhibitory Activity of Compounds of the Present Invention on LCK (h) kinase - IC₅₀ (nM)**

| Test samples | LCK (h) |
|---|---|
| Compound 11 | 20 |
| Compound 12 | 53 |
| Compound 13 | 132 |
| Compound 16 | 579 |
| Compound 17 | 24 |
| Compound 19 | 104 |
| Compound 20 | 2248 |
| Compound 23 | >10,000 |
| Compound 33 | 26 |
| Compound 35 | 20 |
| Compound 39 | 95 |
| Compound 40 | 1067 |

It can be seen from the experimental results in Table 5 that the compounds of the present invention have low inhibitory activities on LCK (h). It can demonstrate that the compounds of the present invention have a low risk of immunosuppression on LCK (h).

### Experimental Example 6: Beagle PK Evaluation Experiment of Compounds of the Present Invention

Test samples: the compounds of the present invention, prepared according to exemplary methods.

Administration and sample collection:
Experimental compounds 11, 12 and 35 were each dissolved in a mixture of 5% DMSO + 20% (30% Solutol) + 75% saline to prepare solutions. The compound solutions were administrated via intragastric gavage to beagles at a dose of 1.0 mg/kg, respectively. The blood was collected at time points of 0 min, 15 min, 30 min, 1 h, 2 h, 4 h, 6 h, 8 h, 10 h and 24 h.

Experimental compounds 11, 12 and 35 were each dissolved in a mixture of 5% DMSO + 20% (30% Solutol) + 75% saline to prepare solutions. The compound solutions were intravenously injected into beagles at a dose of 1.0 mg/kg, respectively. The blood was collected at time points of 0 min, 5 min, 15 min, 30 min, 1 h, 2 h, 4 h, 6 h, 8 h, 10 h and 24 h.

Experimental compound 17 and compound 37 were each dissolved in a mixture of 5% DMSO + 20% PEG400 + 75% (20% Captisol in Saline) to prepare solutions. The compound solutions were administered via intragastric gavage to beagles at a dose of 5.0 mg/kg, respectively. The blood was collected at time points of 0 min, 15 min, 30 min, 1 h, 2 h, 4 h, 6 h, 8 h, 10 h and 24 h.

Experimental compound 17 and compound 37 were each dissolved in a mixture of 5% DMSO + 20% PEG400 + 75% (20% Captisol in Water) to prepare solutions. The compound solutions were intravenously injected into beagles at a dose of 1.0 mg/kg, respectively. The blood was collected at time points of 0 min, 5 min, 15 min, 30 min, 1 h, 2 h, 4 h, 6 h, 8 h, 10 h and 24 h.

Experimental compound 18 was dissolved in a mixture of 5% DMA + 30% PEG400 + 65% (20% Captisol in Saline) to prepare a solution. The compound solution was administered via intragastric gavage to a beagle at a dose of 5.0 mg/kg. The blood was collected at time points of 0 min, 15 min, 30 min, 1 h, 2 h, 4 h, 6 h, 8 h, 10 h and 24 h.

Experimental compound 18 was dissolved in a mixture of 5% DMA + 30% PEG400 + 65% (20% Captisol in Saline) to prepare a solution. The compound solution was intravenously injected into a beagle at a dose of 1.0 mg/kg. The blood was collected at time points of 0 min, 5 min, 15 min, 30 min, 1 h, 2 h, 4 h, 6 h, 8 h, 10 h and 24 h.

About 1 mL of blood was collected via the upper extremity, and the collected blood was placed in an anticoagulation tube containing EDTA-K2. The blood samples were centrifuged at 1800 g for 5 min at 4 °C to give plasma samples which were prepared within 30 min after blood collection. The plasma samples were stored in a refrigerator at -80 °C before test.

Sample analysis:
The samples to be tested were taken out from the refrigerator at -80 °C, and vortexed for 5 min after naturally thawed at room temperature. 20 µL of plasma samples were each pipetted into a 1.5 mL centrifuge tube, and 200 µL of internal standard working solution (a solution of tolbutamide in methanol) at 100 ng/mL was added. The resulting mixture was mixed well, vortexed for 5 min, and then centrifuged at 12,000 rpm for 5 min. 50 µL of the supernatant was pipetted into a 96-well plate with 150 µL of water per well, and the plate was vortexed for 5 min and mixed well before LC-MS/MS analysis.

Data processing:
The concentrations of test samples were calculated by Analyst 1.6.3 from AB Sciex. Parameters such as mean, standard deviation and coefficient of variation (excluding those output directly by Analyst 1.6.3) were calculated by Microsoft Excel. PK parameters were calculated by Pharsight Phoenix 6.1 software NCA (Tₘₐₓ was in median).

The experimental results are shown in Table 6.

Table 6 shows PK parameters of compounds in beagles (IV: 1 mg/kg, PO: 1 mg/kg, n = 3).

**Table 6. PK Experimental Results of Compounds of the Present Invention**

| Compound | Dose iv/po mg/kg | t_{z1/2} iv/po (h) | V_{z_obs}iv (L/kg) | Cl_{_obs} iv (L/h/kg) | Tmax po (h) | AUCₗₐₛₜ iv/ po (h^{∗}ng/mL) | F% |
|---|---|---|---|---|---|---|---|
| Compound 11 | 1/1 | 15.0/5.97 | 5.36 | 0.26 | 1.00 | 3322/1365 | 37.3 |
| Compound 12 | 1/1 | 1.10/1.24 | 1.04 | 0.65 | 0.50 | 1533/1190 | 78.0 |
| Compound 17 | 1/1 | 7.46/13.6 | 3.16 | 0.30 | 1.00 | 3362/3666 | 109 |
| Compound 18 | 1/1 | 1.54/2.00 | 0.65 | 0.29 | 1.00 | 3733/1089 | 29.7 |
| Compound 35 | 1/1 | 9.32/4.01 | 3.16 | 0.23 | 1.00 | 4119/2195 | 50.7 |
| Compound 37 | 1/1 | 1.79/1.88 | 1.07 | 0.41 | 0.50 | 2604/1826 | 70.6 |

| | | | | | | | |
|---|---|---|---|---|---|---|---|
| Note: iv: intravenous injection; po: gavage administration; t_{z1/2}: terminal half-life; Cl_{_obs}: clearance rate; V_{z_obs}: apparent volume of distribution; Tₘₐₓ: time to maximum plasma concentration; AUCₗₐₛₜ: area under concentration-time curve for 0-24 h; F%: absolute bioavailability. | | | | | | | |

It can be seen from the experimental results in Table 6 that the compounds of the present invention have good pharmacokinetic characteristics and good druggability.

### Industrial applicability

The present invention provides a novel TAM family kinase inhibitor compound, which has a good kinase inhibitory activity and can be used for the treatment and/or prevention of diseases mediated by abnormal expression of TAM family kinase receptors and/or ligands thereof. In addition, the compound of the present invention can also target Ron kinase, and thus can be used for the treatment and/or prevention of diseases mediated by abnormal expression of TAM family kinase and/or Ron kinase receptors and/or ligands thereof. Furthermore, the compound of the present invention can inhibit the growth, migration and/or drug resistance of a tumor caused by TAM family kinase and/or Ron kinase.

## Claims

1. A compound of general formula (I), or a pharmaceutically acceptable salt, an ester, a stereoisomer or a tautomer thereof: 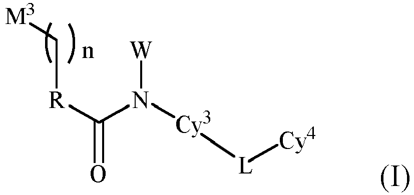 wherein W is selected from hydrogen and optionally substituted C₁₋₆ alkyl;
R represents a group shown as general formula (b): 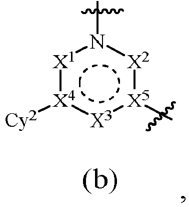 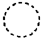 represents an optional double bond moiety in the ring structure, and the 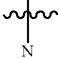 moiety is linked to the M³ group via a linking group;
X¹, X² and X³ are each independently selected from CR^{a}, C=O, NR^{b} and O, and at least one of the above is C=O;
X⁴ and X⁵ are each independently selected from C;
M³ is selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl;
Cy² is selected from 3-12 membered cycloalkyl, 3-12 membered cycloalkenyl, 3-14 membered heterocyclyl, 6-14 membered aryl and 5-10 membered heteroaryl optionally substituted with one or more R², and R² is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl;
Cy³ is selected from 5-10 membered heteroaryl optionally substituted with one or more R³, and R³ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl;
Cy⁴ is selected from 5-9 membered heterocyclyl and 5-9 membered heteroaryl optionally substituted with one or more R⁴, and R⁴ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-14 membered heterocyclyl, 6-14 membered aryl and 5-10 membered heteroaryl, or, two R⁴, together with the atoms attached thereto, can form a 5-6 membered cyclic group;
L is selected from -NR^{b}-, -O-, -S-, and -(CR^{a}R^{a})ₘ-, and m is selected from integers between 1 and 3;
R^{a} is present or absent, and when present, is each independently selected from hydrogen, cyano, hydroxyl, halogen, carboxyl, nitro, -NR^{e}R^{f}, -C(O)R^{g}, -C(O)NR^{e}R^{f}, -OC(O)NR^{e}R^{f}, -NR^{e}C(O)OR^{g}, -NR^{e}C(O)R^{g}, -SO₂-NR^{e}R^{f}, -SO₂R^{g}, and -NR^{e}SO₂R^{g}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{e}C(O)-R', 3-12 membered cycloalkyl, 3-12 membered cycloalkenyl, 3-14 membered heterocyclyl, 6-14 membered aryl and 5-10 membered heteroaryl;
R^{b} and R^{c} are present or absent, and when present, are each independently selected from hydrogen, hydroxyl, -C(O)R^{g}, -C(O)NR^{e}R^{f}, -SO₂-NR^{e}R^{f}, and -SO₂R^{g}; and optionally substituted C₁₋₆ alkyl, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R^{d} is present or absent, and when present, is each independently selected from hydrogen, -NR^{e}R^{f}, -NR^{e}C(O)OR^{g}, -NR^{e}C(O)R^{g}, and -NR^{e}SO₂R^{g}; and optionally substituted C₁₋₆ alkyl, C₁₋₆ alkoxy, -C₁₋₆ alkyl-R', -C₁₋₆ alkoxy-R', -O-R', -NR^{e}C(O)-R', 3-12 membered cycloalkyl, 3-12 membered cycloalkenyl, 3-14 membered heterocyclyl, 6-14 membered aryl and 5-10 membered heteroaryl;
R^{e} and R^{f} are present or absent, and when present, are each independently selected from hydrogen, hydroxyl, carboxyl, cyano, nitro and halogen; and optionally substituted C₁₋₆ alkyl, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R^{g} is present or absent, and when present, is each independently selected from hydrogen; and optionally substituted C₁₋₆ alkyl, C₁₋₆ alkoxy, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R' is 3-12 membered cycloalkyl, 3-12 membered cycloalkenyl, 3-14 membered heterocyclyl, 6-14 membered aryl, or 5-10 membered heteroaryl;
n is an integer between 0 and 4;
the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino,
halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo.

2. The compound, or the pharmaceutically acceptable salt, the ester, the stereoisomer or the tautomer thereof according to claim 1,
wherein W is selected from hydrogen and optionally substituted C₁₋₆ alkyl;
R represents a group shown as general formula (b): 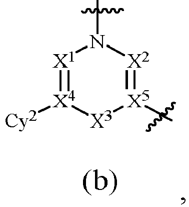
the 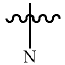 moiety is linked to the M³ group through a linking group;
X¹, X² and X³ are each independently selected from CR^{a}, C=O and NR^{b}, and at least one of the above is C=O;
X⁴ and X⁵ are each selected from C;
M³ is selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, 3-8 membered cycloalkyl and 3-14 membered heterocyclyl;
Cy² is selected from 6-14 membered aryl and 5-10 membered heteroaryl optionally substituted with one or more R², and R² is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-8 membered cycloalkyl, 3-8 membered cycloalkenyl, 3-14 membered heterocyclyl, 6-14 membered aryl and 5-10 membered heteroaryl;
Cy³ is selected from 5-10 membered heteroaryl optionally substituted with one or more R³, and R³ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-8 membered cycloalkyl, 3-8 membered cycloalkenyl, 3-14 membered heterocyclyl, 6-14 membered aryl and 5-10 membered heteroaryl;
Cy⁴ is selected from 5-9 membered heteroaryl optionally substituted with one or more R⁴, and R⁴ is each independently selected from hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, and -NR^{b}SO₂R^{d}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-8 membered cycloalkyl, 3-8 membered cycloalkenyl, 3-14 membered heterocyclyl, 6-14 membered aryl and 5-10 membered heteroaryl, or, two R⁴, together with the atoms attached thereto, can form a 5-6 membered cyclic group;
L is selected from -NR^{b}-, -O- and -S-;
R^{a} is present or absent, and when present, is each independently selected from hydrogen, cyano, hydroxyl, halogen, carboxyl, nitro, -NR^{e}R^{f}, -C(O)R^{g}, -C(O)NR^{e}R^{f}, -OC(O)NR^{e}R^{f}, -NR^{e}C(O)OR^{g}, -NR^{e}C(O)R^{g}, -SO2-NR^{e}R^{f}, -SO₂R^{g}, and -NR^{e}SO₂R^{g}; and optionally substituted C₁₋₆ alkyl, C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{e}C(O)-R', 3-8 membered cycloalkyl, 3-8 membered cycloalkenyl, 3-14 membered heterocyclyl, 6-14 membered aryl and 5-10 membered heteroaryl;
R^{b} and R^{c} are present or absent, and when present, are each independently selected from hydrogen, hydroxyl, -C(O)R^{g}, -C(O)NR^{e}R^{f}, -SO₂-NR^{e}R^{f}, and -SO₂R^{g}; and optionally substituted C₁₋₆ alkyl, -C₁₋₆ alkyl-R', -C(O)-R', -SO₂-R', 3-8 membered cycloalkyl, 3-8 membered cycloalkenyl, 3-14 membered heterocyclyl, 6-14 membered aryl and 5-10 membered heteroaryl;
R^{d} is present or absent, and when present, is each independently selected from hydrogen, -NR^{e}R^{f}, -NR^{e}C(O)OR^{g}, -NR^{e}C(O)R^{g}, and -NR^{e}SO₂R^{g}; and optionally substituted C₁₋₆ alkyl, C₁₋₆ alkoxy, -C₁₋₆ alkyl-R', -C₁₋₆ alkoxy-R', -O-R', -NR^{e}C(O)-R', 3-8 membered cycloalkyl, 3-8 membered cycloalkenyl, 3-14 membered heterocyclyl, 6-14 membered aryl and 5-10 membered heteroaryl;
R^{e} and R^{f} are present or absent, and when present, are independently selected from hydrogen, hydroxyl, carboxyl, cyano, nitro and halogen; and optionally substituted C₁₋₆ alkyl, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R^{g} is present or absent, and when present, is independently selected from hydrogen, and optionally substituted C₁₋₆ alkyl, C₁₋₆ alkoxy, -C₁₋₆ alkyl-R', -C(O)-R', -SO₂-R', 3-12 membered cycloalkyl, 3-12 membered cycloalkenyl, 3-14 membered heterocyclyl, 6-14 membered aryl and 5-10 membered heteroaryl;
R' is 3-8 membered cycloalkyl, 3-8 membered cycloalkenyl, 3-14 membered heterocyclyl, 6-14 membered aryl, or 5-10 membered heteroaryl;
n is an integer between 0 and 3;
the substituents involved in the phrase "optionally substituted" are each independently selected from hydroxyl, sulfydryl, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂ amino, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-8 membered cycloalkyl, 6-14 membered aryl, 3-8 membered heterocyclyl, 5-10 membered heteroaryl and oxo;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O.

3. The compound, or the pharmaceutically acceptable salt, the ester, the stereoisomer or the tautomer thereof according to claim 2,
wherein, X¹, X² and X³ are each independently selected from CR^{a}, C=O and NR^{b}, and at least one of the above is C=O;
X⁴ is selected from C, and X⁵ is selected from C;
M³ is selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl, C₂₋₈ alkenyl, C₂₋₈ alkynyl, 3-8 membered cycloalkyl and 3-8 membered heterocyclyl;
Cy² is selected from phenyl and 5-6 membered heteroaryl optionally substituted with one or more R², and R² is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
Cy³ is selected from 5-6 membered heteroaryl optionally substituted with one or more R³, and R³ is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
Cy⁴ is selected from 5-6 membered heteroaryl and 9-membered ortho-fused heteroaryl optionally substituted with one or more R⁴, and R⁴ is each independently selected from hydrogen, hydroxyl, halogen, nitro, -NR^{b}R^{c}, -C(O)R^{d}, -C(O)NR^{b}R^{c}, and -NR^{b}C(O)R^{d}; and optionally substituted C₁₋₆ alkyl, C₁₋₆ alkoxy and 5-10 membered heteroaryl, or, two R⁴, together with the atoms attached thereto, can form a 5-6 membered oxygen-containing cyclic group;
L is selected from -O-;
R^{a} is present or absent, and when present, is each independently selected from hydrogen and optionally substituted C₁₋₆ alkyl;
R^{b} and R^{c} are present or absent, and when present, are each independently selected from hydrogen and optionally substituted C₁₋₆ alkyl;
R^{d} is present or absent, and when present, is each independently selected from hydrogen and optionally substituted C₁₋₆ alkyl;
n is an integer between 0 and 2;
the substituents involved in the phrase "optionally substituted" are each independently selected from hydroxyl, amino, carboxyl, cyano, nitro, halogen, C₁₋₆ alkyl and C₁₋₆ alkoxy;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O
preferably, Cy³ is 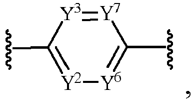 Y², Y³, Y⁶ and Y⁷ are each independently selected from CH and N, and at least one of the above is N.

4. The compound, or the pharmaceutically acceptable salt, the ester, the stereoisomer or the tautomer thereof according to claim 3,
wherein, X¹, X² and X³ are each independently selected from CR^{a}, C=O and NR^{b}, and at least one of the above is C=O;
X⁴ is selected from C, and X⁵ is selected from C;
M³ is selected from hydrogen; and optionally substituted C₁₋₆ alkyl, C₂₋₈ alkenyl, C₂₋₈ alkynyl, 3-8 membered cycloalkyl and 5-6 membered heterocyclyl;
Cy² is selected from phenyl and 5-6 membered heteroaryl optionally substituted with one or more R²;
Cy³ represents 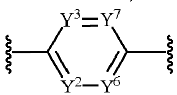 optionally substituted with one or more R³;
Y², Y³, Y⁶ and Y⁷ are each independently selected from CH and N, and at least one of the above is N;
Cy⁴ is selected from 6-membered heteroaryl and 9-membered ortho-fused heteroaryl optionally substituted with one or more R⁴;
R² is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R³ is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R⁴ is each independently selected from hydrogen, hydroxyl, halogen, nitro, -NR^{b}R^{c}, -C(O)R^{d}, -C(O)NR^{b}R^{c}, -NR^{b}C(O)R^{d} and 5-10 membered heteroaryl; and optionally substituted C₁₋₆ alkyl and C₁₋₆ alkoxy;
L is selected from -O-;
R^{a} is present or absent, and when present, is each independently selected from hydrogen, C₁₋₆ alkyl and halogenated C₁₋₆ alkyl;
R^{b} and R^{c} are present or absent, and when present, are each independently selected from hydrogen and C₁₋₆ alkyl;
R^{d} is present or absent, and when present, is each independently selected from hydrogen and C₁₋₆ alkyl;
n is an integer between 0 and 2;
the substituents involved in the phrase "optionally substituted" are each independently selected from hydroxyl, sulfydryl, amino, carboxyl, cyano, nitro, halogen, C₁₋₆ alkyl and C₁₋₆ alkoxy;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O;
preferably, Cy⁴ is selected from 6-membered N-containing heteroaryl and 9-membered N-containing ortho-fused heteroaryl optionally substituted with one or more R⁴.

5. The compound, or the pharmaceutically acceptable salt, the ester, the stereoisomer or the tautomer thereof according to claim 4,
wherein X¹ and X² are each independently selected from CR^{a} and NR^{b}, and X³ is C=O;
M³ is selected from hydrogen, C₁₋₆ alkyl, C₂₋₈ alkenyl, C₂₋₈ alkynyl, 3-6 membered cycloalkyl and 5-6 membered heterocyclyl;
Cy² is selected from phenyl optionally substituted with one or more R²;
Cy³ represents 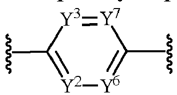 optionally substituted with one or more R³;
Y², Y³, Y⁶ and Y⁷ are independently selected from CH and N, and at least one of the above is N;
Cy⁴ is selected from 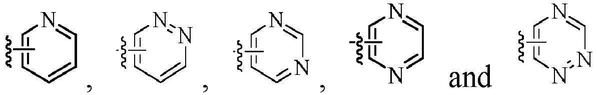 optionally substituted with one or more R⁴;
R² is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R³ is each independently selected from hydrogen, hydroxyl, halogen, carboxyl, nitro, -NR^{b}R^{c}, -C(O)R^{d}, -C(O)NR^{b}R^{c}, and -NR^{b}C(O)R^{d}; and optionally substituted C₁₋₆ alkyl and C₁₋₆ alkoxy;
R⁴ is each independently selected from hydrogen, hydroxyl, halogen, nitro, -NR^{b}R^{c}, -C(O)R^{d}, -C(O)NR^{b}R^{c}, and -NR^{b}C(O)R^{d}; and optionally substituted C₁₋₆ alkyl, C₁₋₆ alkoxy and 5-6 membered heteroaryl;
L is selected from -O-;
R^{a} is present or absent, and when present, is each independently selected from hydrogen, C₁₋₆ alkyl and halogenated C₁₋₆ alkyl;
R^{b} and R^{c} are present or absent, and when present, are each independently selected from hydrogen and C₁₋₆ alkyl;
R^{d} is present or absent, and when present, is each independently selected from hydrogen and C₁₋₆ alkyl;
n is an integer between 0 and 1;
the substituents involved in the phrase "optionally substituted" are each independently selected from hydroxyl, sulfydryl, amino, carboxyl, cyano, nitro and halogen.

6. The compound, or the pharmaceutically acceptable salt, the ester, the stereoisomer or the tautomer thereof according to claim 1,
wherein W is selected from hydrogen;
R represents a group shown as general formula (b): 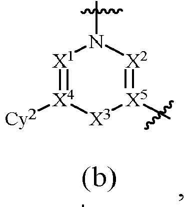
the 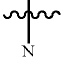 moiety is linked to the M³ group through a linking group;
X¹ and X² are each independently selected from CR^{a} and NR^{b}, and X³ is C=O;
X⁴ is selected from C, and X⁵ is selected from C;
M³ is selected from hydrogen, hydroxyl, halogen, nitro, C₁₋₄ alkyl, C₂₋₆ alkenyl, C₂₋₆ alkynyl, 3-6 membered cycloalkyl and 5-6 membered heterocyclyl;
Cy² is selected from 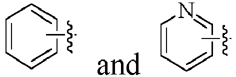 optionally substituted with one or more R², and R² is each independently selected from hydrogen, hydroxyl, fluoro, chloro, bromo and C₁₋₄ alkyl;
Cy³ is selected from 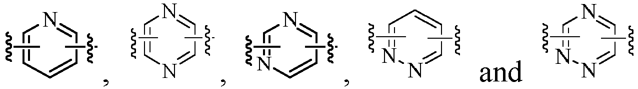 optionally substituted with one or more R³, and R³ is each independently selected from hydrogen, hydroxyl, fluoro, chloro, bromo and C₁₋₄ alkyl;
Cy⁴ is selected from 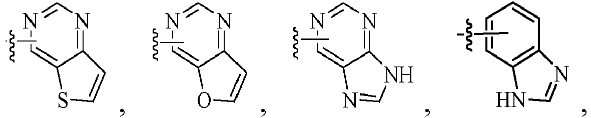 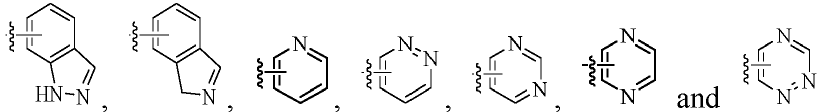 optionally substituted with one or more R⁴, and
R⁴ is each independently selected from hydrogen, hydroxyl, halogen, amino, (C₁₋₄ alkyl)₁₋₂ amino-, C₁₋₄ alkyl, halogenated C₁₋₄ alkyl, C₁₋₄ alkoxy, halogenated C₁₋₄ alkoxy, 3-14 membered heterocyclyl, and 5-6 membered heteroaryl;
L is -O-;
R^{a} is present or absent, and when present, is each independently selected from hydrogen, C₁₋₆ alkyl and halogenated C₁₋₆ alkyl;
R^{b} is present or absent, and when present, is each independently selected from hydrogen and C₁₋₆ alkyl;
n is an integer between 0 and 1;
preferably, X¹ is N, X² is CR^{a}, and X³ is C=O;
preferably, X¹ is CR^{a}, X² is N, and X³ is C=O;
preferably, X¹ is CR^{a}, X² is CR^{a}, and X³ is C=O.

7. The compound, or the pharmaceutically acceptable salt, the ester, the stereoisomer or the tautomer thereof according to claim 1, being a compound selected from the following structures:

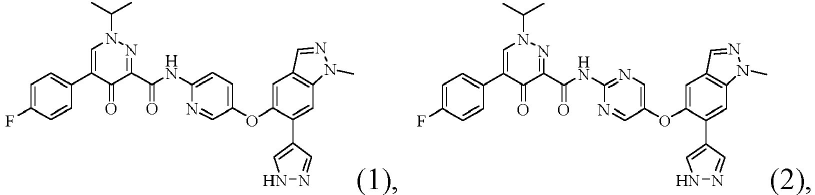

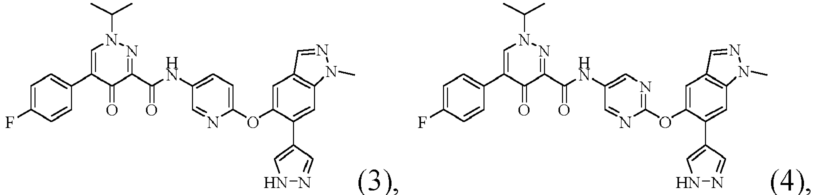

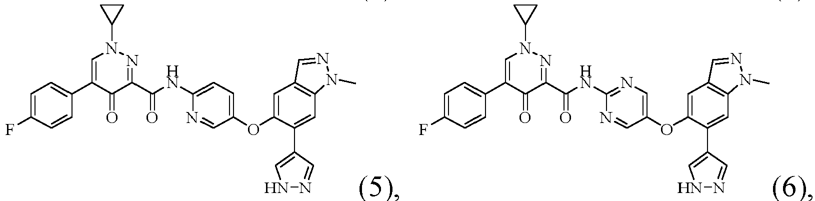

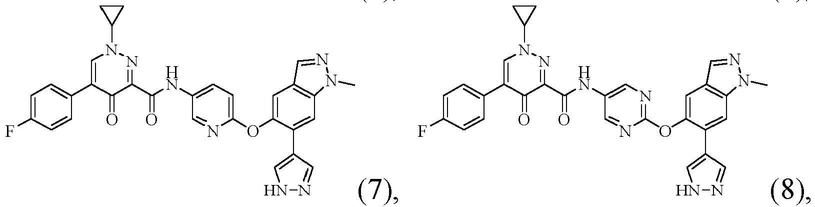

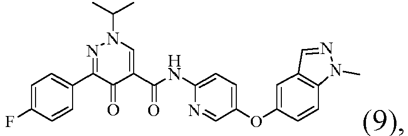

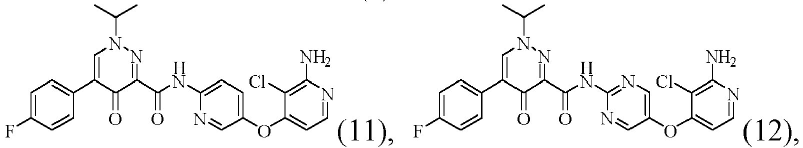

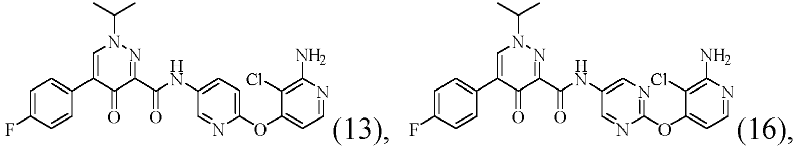

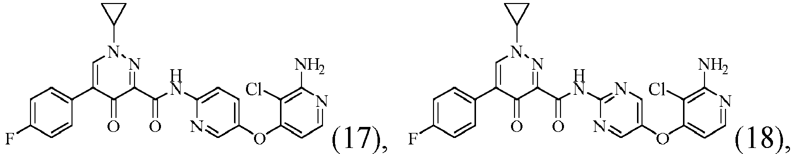

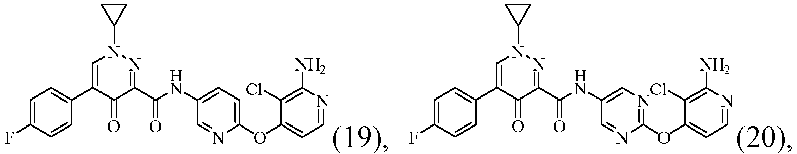

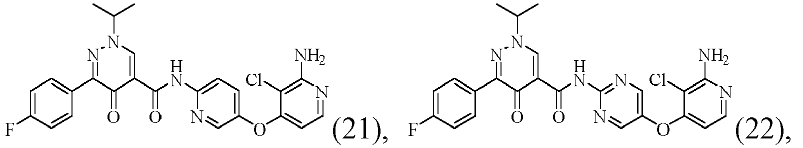

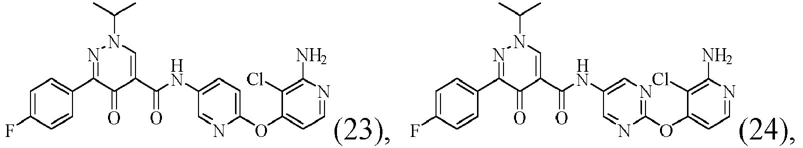

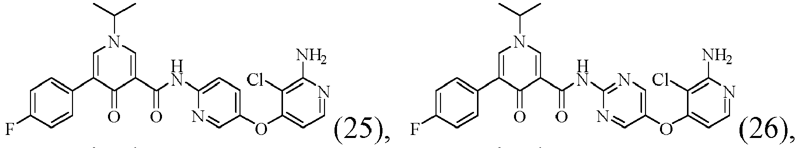

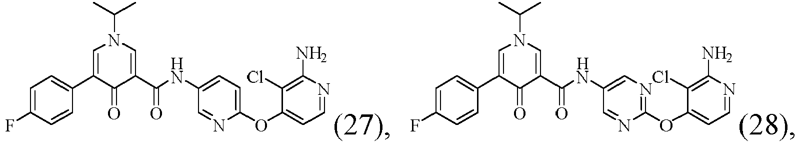

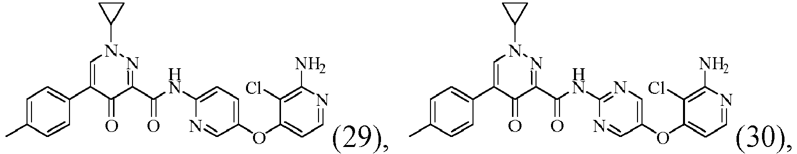

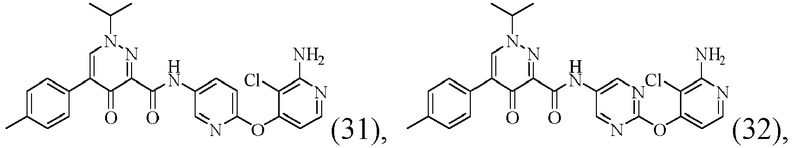

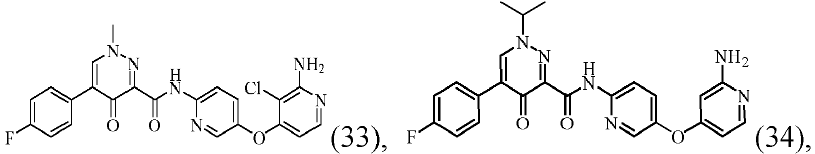

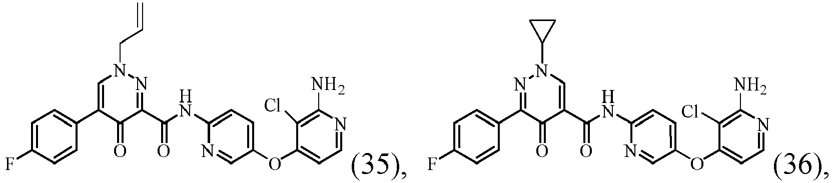

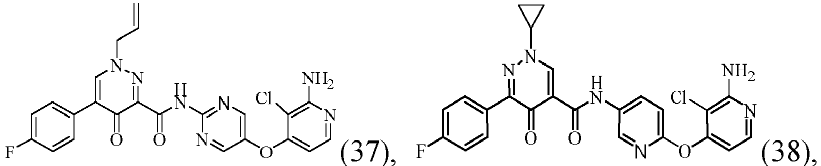

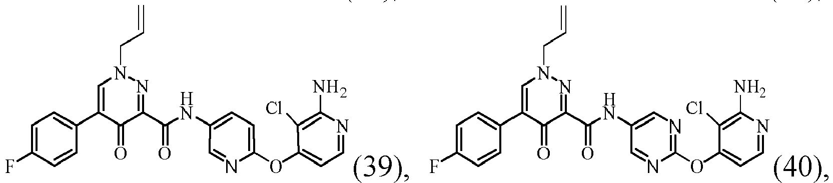

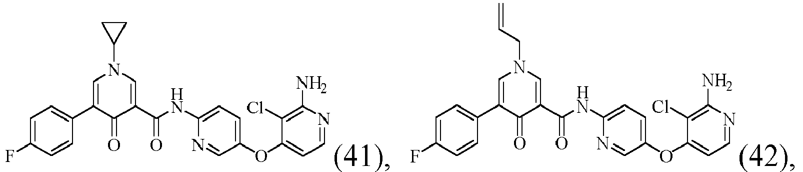

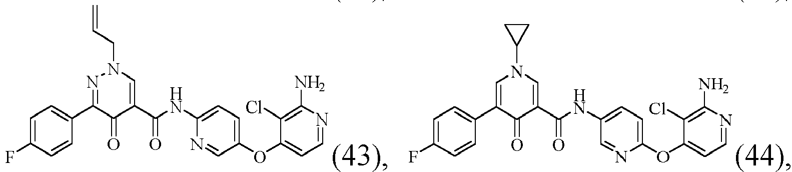

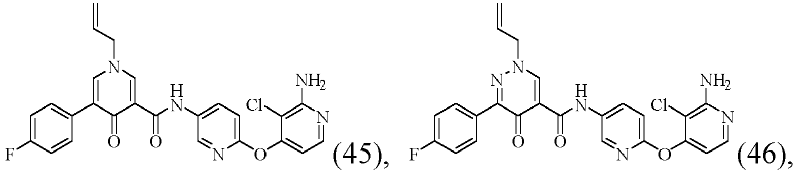

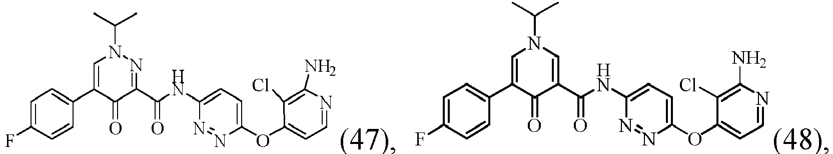

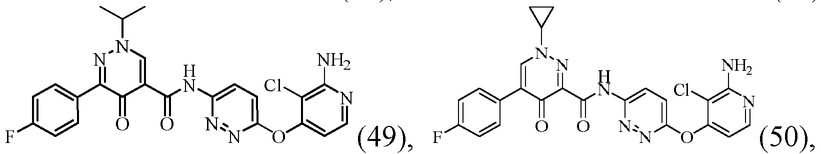

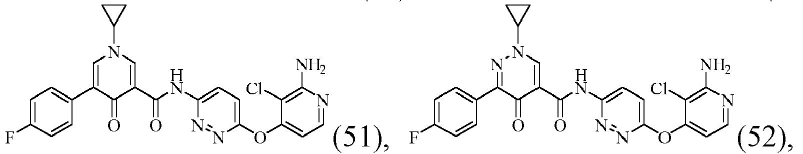

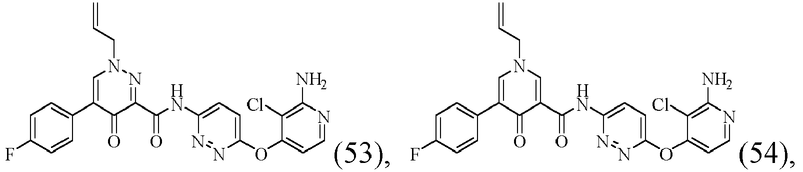

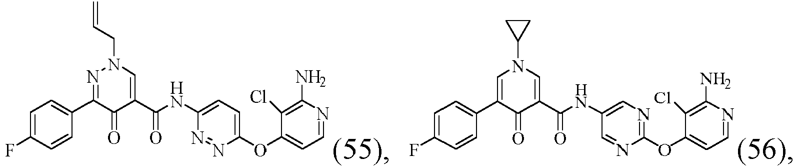

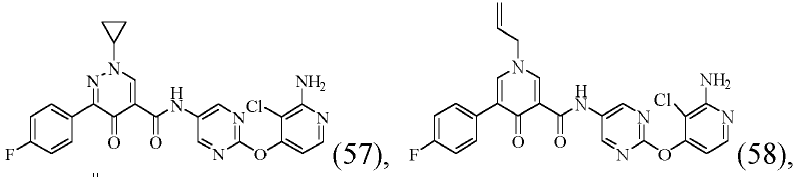

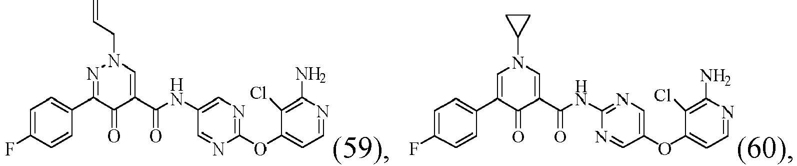

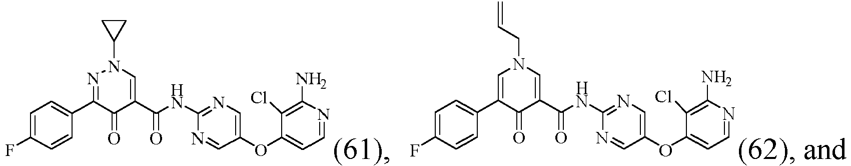

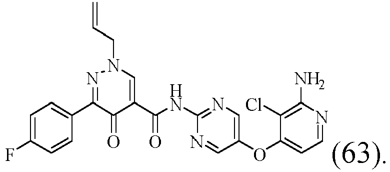

8. A pharmaceutical composition comprising the compound, or the pharmaceutically acceptable salt, the ester, the stereoisomer or the tautomer thereof according to any one of claims 1-7, optionally comprising one or more pharmaceutically acceptable carriers.

9. The pharmaceutical composition according to claim 8, further comprising one or more second therapeutically active agents, wherein the second therapeutically active agents are antimetabolites, growth factor inhibitors, mitosis inhibitors, anti-tumor hormones, alkylating agents, platinum metal, topoisomerase inhibitors, hormonal drugs, immunomodulators, tumor suppressor genes, cancer vaccines, immune checkpoint inhibitors, antibodies associated with tumor immunotherapy, or small molecule drugs associated with tumor immunotherapy.

10. Use of the compound, or the pharmaceutically acceptable salt, the ester, the stereoisomer or the tautomer thereof according to any one of claims 1-7 and the pharmaceutical composition according to claim 8 in the manufacture of a medicament for the treatment and/or prevention of diseases associated with abnormal signaling pathways resulting from abnormal expression of TAM family receptors and/or ligands thereof, wherein the diseases associated with abnormal signaling pathways resulting from abnormal expression of TAM family receptors and/or ligands thereof include at least one of the following diseases: tumor, endometriosis, vascular disease/trauma, psoriasis, visual defect/lesion, kidney disease, rheumatoid arthritis and osteoporosis.

11. Use of the compound, or the pharmaceutically acceptable salt, the ester, the stereoisomer or the tautomer thereof according to any one of claims 1-7 and the pharmaceutical composition according to claim 8 in the manufacture of a medicament for the treatment and/or prevention of diseases mediated by abnormal expression of TAM family kinases/Ron kinase receptors and/or ligands thereof, wherein the diseases mediated by abnormal expression of TAM family kinases/Ron kinase receptors and/or ligands thereof include at least one of the following diseases: tumor, immuno-oncology, endometriosis, vascular disease/trauma, psoriasis, visual defect/lesion, kidney disease, rheumatoid arthritis and osteoporosis.
